# EUROPEAN PATENT SPECIFICATION

(11) **EP 2 414 337 B1**
(45) Date of publication and mention of the grant of the patent: **01.05.2013**
(21) Application number: 10715104.5
(22) Date of filing: 31.03.2010
(51) Int. Cl.: C07D 239/48, C12N 11/06, C12Q 1/48

(54) **Methods for the identification of kinase interacting molecules and for the purification of kinase proteins**
Verfahren zur Identifizierung von mit Kinase interagierenden Molekülen und zur Reinigung von Kinase-Proteinen
Procédé d'identification de molécules d'interaction de kinase et de purification de protéines de kinase

(30) Priority: 03.04.2009 EP 09004956
(43) Date of publication of application: 08.02.2012
(73) Proprietor: Cellzome GmbH, 69117 Heidelberg (DE)
(72) Inventor: RAMSDEN, Nigel, Herts SG8 7QP (GB); MAJOR, Jeremy, Brentford, Middlesex TW8 9GS (GB); HARRISON, John, Cambrige CB1 3SX (GB); LEGGATE, Daniel, CB22 4NL (GB); GRANDI, Paola, 69126 Heidelberg (DE)
(74) Representative: Lahrtz, Fritz
(86) International application number: PCT/EP2010/002050
(87) International publication number: WO 2010/112210

(56) References cited:
- WO-A-2007/053452
- WO-A-2008/009458
- BAMBOROUGH ET AL: "N-4-Pyrimidinyl-1H-indazol-4-amine inhibitors of Lck: Indazoles as phenol isosteres with improved pharmacokinetics" BIOORGANIC & MEDICINAL CHEMISTRY LETTERS, PERGAMON, ELSEVIER SCIENCE, GB, vol. 17, no. 15, 1 August 2007 (2007-08-01), pages 4363-4368, XP022144705 ISSN: 0960-894X

## Description

The present invention relates to immobilization compounds, immobilization products and preparations thereof as well as methods and uses for the identification of kinase interacting compounds or for the purification or identification of kinase proteins.

Kinases catalyze the phosphorylation of proteins, lipids, sugars, nucleosides and other cellular metabolites and play key roles in all aspects of eukaryotic cell physiology. Especially, protein kinases and lipid kinases participate in the signaling events which control the activation, growth, differentiation and survival of cells in response to extracellular mediators or stimuli such as growth factors, cytokines or chemokines. In general, protein kinases are classified in two groups, those that preferentially phosphorylate tyrosine residues and those that preferentially phosphorylate serine and/or threonine residues.

Inappropriately high protein kinase activity is involved in many diseases including cancer, metabolic diseases and autoimmune/inflammatory disorders. This can be caused either directly or indirectly by the failure of control mechanisms due to mutation, overexpression or inappropriate activation of the enzyme. In all of these instances, selective inhibition of the kinase is expected to have a beneficial therapeutic effect.

The complement of protein kinases encoded in the human genome comprises 518 family members (kinome) which can be grouped into several subfamilies according to sequence similarity (Review: Manning et al., 2002, The Protein Kinase Complement of the Human Genome, Science 298, 1912-1934). Small molecule kinase inhibitors are pursued as new anticancer therapeutics. Understanding the selectivity of a kinase inhibitor is crucial for the goal of developing selective inhibitors for therapeutically relevant kinases (Zhang et al., 2009. Nature Reviews Cancer 9, 28-39). The repertoire of kinases targeted by a given inhibitor can be determined by profiling its activity in binding and enzymatic assays against extensive panels of recombinant kinases, by profiling activity in cellular assays and by affinity approaches combined with detection by mass spectrometry.

Kinases are also important therapeutic targets for the development of anti-inflammatory drugs (Cohen, 2009. Current Opinion in Cell Biology 21, 1-8), for example kinases that are involved in the orchestration of adaptive and innate immune responses. Kinase targets of particular interest are members of the IRAK family and NIK.

The interleukin-1 receptor-associated kinases (IRAKs) are critically involved in the regulation of intracellular signaling networks controlling inflammation (Ringwood and Li, 2008. Cytokine 42, 1-7). IRAKs are expressed in many cell types and can mediate signals from various cell receptors including toll-like receptors (TLRs). IRAK4 is thought to be the initial protein kinase activated downstream of the interleukin-1 (IL-1) receptor and all toll-like-receptors (TLRs) except TLR3, and initiates signaling in the innate immune system via the rapid activation of IRAK1 and slower activation of IRAK2. IRAK1 was first identified through biochemical purification of the IL-1 dependent kinase activity that co-immunoprecipitates with the IL-1 type 1 receptor (Cao et al., 1996. Science 271(5252):1128-31). IRAK2 was identified by the search of the human expressed sequence tag (EST) database for sequences homologous to IRAK1 (Muzio et al., 1997. Science 278(5343):1612-5). IRAK3 (also called IRAKM) was identified using a murine EST sequence encoding a polypeptide with significant homology to IRAK1 to screen a human phytohemagglutinin-activated peripheral blood leukocyte (PBL) cDNA library (Wesche et al., 1999. J. Biol. Chem. 274(27):19403-10). IRAK4 was identified by database searching for IRAK-like sequences and PCR of a universal cDNA library (Li et al., 2002. Proc. Natl. Acad. Sci. USA 99(8):5567-5572).

Mice that express a catalytically inactive mutant of IRAK4 instead of the wild-type kinase are completely resistant to septic shock triggered by several TLR agonists and are impaired in their response to IL-1. Children who lack IRAK4 activity due to a genetic defect suffer from recurring infection by pyogenic bacteria. It appears that IRAK-dependent TLRs and IL-IRs are vital for childhood immunity against some pyogenic bacteria but play a redundant role in protective immunity to most infections in adults. Therefore IRAK4 inhibitors may be useful for the treatment of chronic inflammatory diseases in adults without making them too susceptible to bacterial and viral infections (Cohen, 2009. Current Opinion in Cell Biology 21, 1-8). Potent IRAK4 inhibitors have been developed (Buckley et al., 2008. Bioorg Med Chem Lett. 18(12):3656-60). IRAK1 is essential for the TLR7-mediated and TLR9-mediated activation of IRF7 and the production of interferon-alpha (IFN-α) suggesting that IRAKI inhibitors may be useful for the treatment of Systemic lupus erythematosus (SLE). IRAK2 is activated downstream of IRAK4 and plays a role in proinflammatory cytokine production. Therefore IRAK2 inhibitors may be useful for inflammatory diseases.

NF-kappa-B-inducing kinase (NIK) is a cytoplasmic serine/threonine kinase which activates NF-kappa-B mainly through the non-canonical NF-kappa-B pathway (Malinin et al., 1997. Nature 385(6616):540-544). The non-canonical or alternative NF-kappa-B pathway is switched on by several ligands of the TNF family leading to elevated expression and activity of NIK. Recent evidence suggests that constitutive activation of the non-canonical NF-kappa-B pathway is a frequent cause of multiple myeloma (MM) and results from inactivating mutations in TRAF3 and/or elevated expression of NIK. The enhanced expression of NIK can be caused by the loss of TRAF3 or mutations in the NIK gene itself (Annunziata et al., 2007. Cancer Cell 12(2):115-130; Keats et al., 2007. Cancer Cell 12(2):131-144). Therefore drugs that inhibit NIK may be useful for the treatment of this cancer of the immune system (Cohen, 2009. Current Opinion in Cell Biology 21, 1-8).

Another, although not in all instances necessary prerequisite for the identification of selective kinase inhibitors is a method that allows to determine the target selectivity of these molecules. For example, it can be intended to provide molecules that bind to and inhibit a particular drug target but do not interact with a closely related target, inhibition of which could lead to unwanted side effects. Conventionally large panels of individual enzyme assays are used to assess the inhibitory effect of a compound for kinases (Davies et al., 2000. Biochemical Journal 351(Pt 1):95-105; Bain et al., 2007. Biochemical Journal 408(3):297-315). More recently, kinases or kinase domains displayed on bacteriophages have been employed to assess the ability of a given compound to interact with a large set of kinases (Karaman et al., 2008. Nature Biotechnology 26, 127-132). In addition, chemical proteomics methods have been described which allow the profiling of kinase inhibitors against the proteome (WO 2006/134056; Bantscheff et al., 2007. Nature Biotechnology 25, 1035-1044; Patricelly et al., 2007. Biochemistry 46, 350-358; Gharbi et al., 2007. Biochem. J. 404, 15-21; WO2008/015013).

WO 2007/053452 A1 discloses biaryl meta-pyrimidine compounds capable of inhibiting kinases, such as members of the Jak kinase family, and various other specific receptor and non receptor kinases. However, this document is not directed to immobilization products which are useful for the identification of kinase interacting molecules including the purification of kinase proteins. The compounds of the present invention differ from the compounds disclosed in WO 2007/053452 A1 in that they (i) contain a sulfonylamino group rather than an aminosulfonyl group, and (ii) in that they contain a primary amino group rather than a N-substituted pyrrolidine ring.

In view of the above, there is a need for providing effective tools and methods for the identification and selectivity profiling of kinase interacting compounds as well as for the purification of kinases.

The present invention relates inter alia to an immobilization compound of formula (I) 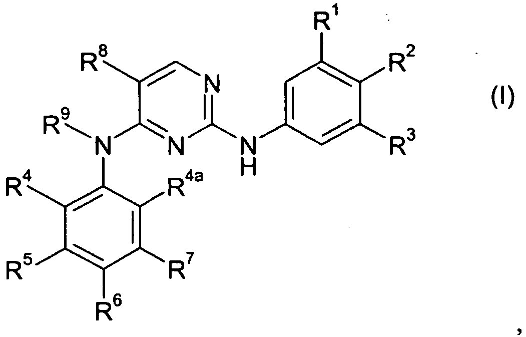 or a salt thereof, wherein
One of R¹, R², R³ is X-(CH₂)ₙ-NH₂ and the other two are independently selected from the group consisting of H; halogen; CN; C(O)OR¹⁰; OR¹⁰; C(O)R¹⁰; C(O)N(R¹⁰R^{10a}); S(O)₂N(R¹⁰R^{10a}); S(O)N(R¹⁰R^{10a}); S(O)₂R¹⁰; S(O)R¹⁰; N(R¹⁰)S(O)₂N(R^{10a}R^{10b}); SR¹⁰; N(R¹⁰R^{10a}); NO₂; OC(O)R¹⁰; N(R¹⁰)C(O)R^{10a}; N(R¹⁰)S(O)₂R^{10a}; N(R¹⁰)S(O)R^{10a}; N(R¹⁰)C(O)N(R^{10a}R^{10b}); N(R¹⁰)C(O)OR^{10a}; OC(O)N(R¹⁰R^{10a}); C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R¹¹, which are the same or different;
X is a single covalent chemical bond; O; S; NH; NHC(O); or C(O)NH;
n is 0; 1; 2; 3; 4; 5; or 6, when X is a single covalent chemical bond or NHC(O); and n is 2; 3; 4; 5; or 6, when X is O; S; or C(O)NH;
R¹⁰, R^{10a}, R^{10b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R¹², which are the same or different;
R¹¹, R¹² are independently selected from the group consisting of halogen; CN; C(O)OR¹³; OR¹³; C(O)R¹³; C(O)N(R¹³R^{13a}); S(O)₂N(R¹³R^{13a}); S(O)N(R¹³R^{13a}); S(O)₂R¹³; S(O)R¹³; N(R¹³)S(O)₂N(R^{13a}R^{13b}); N(R¹³)S(O)N(R^{13a}R^{13b}); SR¹³; N(R¹³R^{13a}); NO₂; OC(O)R¹³; N(R¹³)C(O)R^{13a}; N(R¹³)S(O)₂R^{13a}; N(R¹³)S(O)R^{13a}; N(R¹³)C(O)N(R^{13a}R^{13b}); N(R¹³)C(O)OR^{13a}; and OC(O)N(R¹³R^{13a}).
R¹³, R^{13a}, R^{13b} are independently selected from the group consisting of H; C₁₋₆ alkyl; C₂₋₆ alkenyl; and C₂₋₆alkynyl, wherein C₁₋₆ alkyl; C₂₋₆ alkenyl; and C₂₋₆ alkynyl are optionally substituted with one or more halogen, which are the same or different;
R⁴, R⁵, R⁶, R⁷, R^{4a} are independently selected from the group consisting of H; X¹; halogen; CN; C(O)OR¹⁴; OR¹⁴; C(O)R¹⁴; C(O)N(R¹⁴R^{14a}); S(O)₂N(R¹⁴R^{14a}); S(O)N(R¹⁴R^{14a}); S(O)₂R¹⁴; S(O)R¹⁴; SR¹⁴; N(R¹⁴R^{14a}); NO₂; OC(O)R¹⁴; N(R¹⁴)C(O)R^{14a}; N(R¹⁴)S(O)₂R^{14a}; N(R¹⁴)S(O)R^{14a}; N(R¹⁴)C(O)N(R^{14a}R^{14b}); N(R¹⁴)C(O)OR^{14a}; OC(O)N(R¹⁴R^{14a}); C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R¹⁵, which are the same or different,
   provided that one of R⁴, R⁵, R⁶, R⁷, R^{4a} is X¹;
R¹⁴, R^{14a}, R^{14b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R¹⁶, which are the same or different;
R¹⁵, R¹⁶ are independently selected from the group consisting of halogen; CN; C(O)OR¹⁷; OR¹⁷; C(O)R¹⁷; C(O)N(R¹⁷R^{17a}); S(O)₂N(R¹⁷R^{17a}); S(O)N(R¹⁷R^{17a}); S(O)₂R¹⁷; S(O)R¹⁷; N(R¹⁷)S(O)₂N(R^{17a}R^{17b}); N(R¹⁷)S(O)N(R^{17a}R^{17b}); SR¹⁷; N(R¹⁷R^{17a}); NO₂; OC(O)R¹⁷; N(R¹⁷)C(O)R^{17a}; N(R¹⁷)S(O)₂R^{17a}; N(R¹⁷)S(O)R^{17a}; N(R¹⁷)C(O)N(R^{17a}R^{17b}); N(R¹⁷)C(O)OR^{17a}; OC(O)N(R¹⁷R^{17a}); C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more halogen, which are the same or different;
R¹⁷, R^{17a}, R^{17b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more halogen, which are the same or different;
X¹ is N(R^{18a})S(O)₂R¹⁸; or N(R^{18a})S(O)₂N(R^{18b}R¹⁸);
R⁹, R^{18a}, R^{18b} are independently selected from the group consisting of H; C₁₋₆ alkyl; C₃₋₅ cycloalkyl; and C₃₋₅ cycloalkylmethyl, wherein C₁₋₄ alkyl; C₃₋₅ cycloalkyl and C₃₋₅ cycloalkylmethyl are optionally substituted with one or more halogen, which are the same or different;
R¹⁸ is T; C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more R¹⁹, which are the same or different;
R¹⁹ is T; halogen; CN; C(O)OR²⁰; OR²⁰; C(O)R²⁰; C(O)N(R²⁰R^{20a}); S(O)₂N(R²⁰R^{20a}); S(O)N(R²⁰R^{20a}); S(O)₂R²⁰; S(O)R²⁰; N(R²⁰)S(O)₂N(R^{20a}R^{20b}); N(R²⁰)S(O)N(R^{20a}R^{20b}); SR²⁰; N(R²⁰R^{20a}); NO₂; OC(O)R²⁰; N(R²⁰)C(O)R^{20a}; N(R²⁰)S(O)₂R^{20a}; N(R²⁰)S(O)R^{20a}; N(R²⁰)C(O)N(R^{20a}R^{20b}); N(R²⁰)C(O)OR^{20a}; OC(O)N(R²⁰R^{20a}); C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more halogen, which are the same or different;
R²⁰, R^{20a}, R^{20b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more halogen, which are the same or different;
T is phenyl; C₃₋₇ cycloalkyl; or 4 to 7 membered heterocyclyl, wherein T is optionally substituted with one or more R²¹, which are the same or different;
R²¹ is halogen; CN; C(O)OR²²; OR²²; oxo (=O), where the ring is at least partially saturated; C(O)R²²; C(O)N(R²²R^{22a}); S(O)₂N(R²²R^{22a}); S(O)N(R²²R^{22a}); S(O)₂R²²; S(O)R²²; N(R²²)S(O)₂N(R^{22a}R^{22b}); N(R²²)S(O)N(R^{22a}R^{22b}); SR²²; N(R²²R^{22a}); NO₂; OC(O)R²² ; N(R²²)C(O)R^{22a}; N(R²²)S(O)₂R^{22a}; N(R²²)S(O)R^{22a}; N(R²²)C(O)N(R^{22a}R^{22b}); N(R²²)C(O)OR^{22a}; OC(O)N(R²²R^{22a}); C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more halogen, which are the same or different;
R²², R^{22a}, R^{22b} are independently selected from the group consisting of H; C₁₋₆ alkyl; C₂₋₆ alkenyl; and C₂₋₆alkynyl, wherein C₁₋₆ alkyl; C₂₋₆ alkenyl; and C₂₋₆ alkynyl are optionally substituted with one or more halogen, which are the same or different;
R⁸ is H; F; Cl; Br; CN; C₁₋₄ alkyl; CH₂F; CHF₂; CF₃; OH; OCH₃; NO₂; NH₂; NHCH₃; N(CH₃)₂; or NO₂.

In case a variable or substituent can be selected from a group of different variants and such variable or substituent occurs more than once the respective variants can be the same or different.

Within the meaning of the present invention the terms are used as follows:
"Alkyl" means a straight-chain or branched saturated hydrocarbon chain. Each hydrogen of an alkyl carbon may be replaced by a substituent.
"Alkenyl" means a straight-chain or branched hydrocarbon chain, that contains at least one carbon-carbon double bond. Each hydrogen of an alkenyl carbon may be replaced by a substituent.
"Alkynyl" means a straight-chain or branched hydrocarbon chain, that contains at least one carbon-carbon triple bond. Each hydrogen of an alkynyl carbon may be replaced by a substituent.
"C₁₋₄ alkyl" means an alkyl chain having 1 - 4 carbon atoms, e.g. if present at the end of a molecule: methyl, ethyl, n-propyl, isopropyl, n-butyl, isobutyl, sec-butyl tert-butyl, or e.g. -CH₂-, -CH₂-CH₂-, -CH(CH₃)-, -C(CH₂)-, -CH₂-CH₂-CH₂-, -CH(C₂H₅)-, -C(CH₃)₂-, when two moieties of a molecule are linked by the alkyl group. Each hydrogen of a C₁₋₄ alkyl carbon may be replaced by a substituent.
"C₁₋₆ alkyl" means an alkyl chain having 1 - 6 carbon atoms, e.g. if present at the end of a molecule: C₁₋₄ alkyl, methyl, ethyl, n-propyl, isopropyl, n-butyl, isobutyl, sec-butyl, tert-butyl, n-pentyl, n-hexyl, or e.g. -CH₂-, -CH₂-CH₂-, -CH(CH₃)-, -CH₂-CH₂-CH₂-, -CH(C₂H₅)-, -C(CH₃)₂-, when two moieties of a molecule are linked by the alkyl group. Each hydrogen of a C₁₋₆ alkyl carbon may be replaced by a substituent.
"C₂₋₆ alkenyl" means an alkenyl chain having 2 to 6 carbon atoms, e.g. if present at the end of a molecule: -CH=CH₂, -CH=CH-CH₃, -CH₂-CH=CH₂, -CH=CH-CH₂-CH₃, -CH=CH-CH=CH₂, or e.g. -CH=CH-, when two moieties of a molecule are linked by the alkenyl group. Each hydrogen of a C₂₋₆ alkenyl carbon may be replaced by a substituent.
"C₂₋₆ alkynyl" means an alkynyl chain having 2 to 6 carbon atoms, e.g. if present at the end of a molecule: -C≡CH, -CH₂-C≡CH, CH₂-CH₂-C≡CH, CH₂-C≡C-CH₃, or e.g. -C≡C- when two moieties of a molecule are linked by the alkynyl group. Each hydrogen of a C₂₋₆ alkynyl carbon may be replaced by a substituent.
"C₃₋₇ cycloalkyl" or "C₃₋₇ cycloalkyl ring" means a cyclic alkyl chain having 3 - 7 carbon atoms, e.g. cyclopropyl, cyclobutyl, cyclopentyl, cyclohexyl, cyclohexenyl, cycloheptyl. Each hydrogen of a cycloalkyl carbon may be replaced by a substituent. Accordingly, "C₃₋₅ cycloalkyl" means a cycloalkyl having 3 to 5 carbon atoms.
"Halogen" means fluoro, chloro, bromo or iodo. It is generally preferred that halogen is fluoro or chloro.
"4 to 7 membered heterocyclyl" or "4 to 7 membered heterocycle" means a ring with 4, 5, 6 or 7 ring atoms that may contain up to the maximum number of double bonds (aromatic or non-aromatic ring which is fully, partially or un-saturated) wherein at least one ring atom up to 4 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a 4 to 7 membered heterocycles are azetidine, oxetane, thietane, furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, diazepane, azepine or homopiperazine.

The immobilization compounds claimed in the present invention have been named as "immobilization compounds" due to their preferred use in the preparation of immobilization products as described below. However, other possible uses, e.g. as a soluble competitor in assays or as a labelled probe, are also explicitly included within the present invention.

In case the immobilization compounds according to formula (I) contain one or more acidic or basic groups, the invention also comprises their corresponding salts. Thus, the immobilization compounds of the formula (I) which contain acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Immobilization compounds of the formula (I) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the immobilization compounds of the formula (I) simultaneously contain acidic and basic groups in the molecule, the invention also includes, in addition to the salt forms mentioned, inner salts or betaines (zwitterions). The respective salts according to the formula (I) can be obtained by customary methods which are known to the person skilled in the art like, for example by contacting these with an organic or inorganic acid or base in a solvent or dispersant, or by anion exchange or cation exchange with other salts.

The present invention furthermore includes all solvates of the immobilization compounds according to the invention.

As it can be taken from the Examples, immobilization compounds falling under formula (I) have been shown to bind to kinases, which makes them useful tools in the context of assays for the identification of kinase interacting compounds.

Preferred immobilization compounds of formula (I) are those immobilization compounds in which one or more of the residues contained therein have the meanings given below, with all combinations of preferred substituent definitions being a subject of the present invention. With respect to all preferred immobilization compounds of the formulae (I) the present invention also includes all tautomeric and stereoisomeric forms and mixtures thereof in all ratios.

In preferred embodiments of the present invention, the substituents mentioned below independently have the following meaning. Hence, one or more of these substituents can have the preferred or more preferred meanings given below.

Preferably, one of R¹, R², R³ is X-(CH₂)ₙ-NH₂ and the other two are H.

Preferably, X is O.

Preferably, n is 2.

Preferably, one of R⁴, R⁵, R⁶, R⁷, R^{4a} is X¹ and the other are H.

Preferably, R^{4a} is X¹.

Preferably, X¹ is N(R^{18a})S(O)₂R¹⁸.

Preferably, R^{18a}, R^{18b}, R⁹ are H.

Preferably, R¹⁸ is C₁₋₆ alkyl; more preferably, methyl.

Preferably, R⁸ is H; or halogen; more preferably, H; F; or Cl; even more preferably F; or Cl.

Preferred immobilization compounds of formula (I) of the present invention are selected from the group consisting of 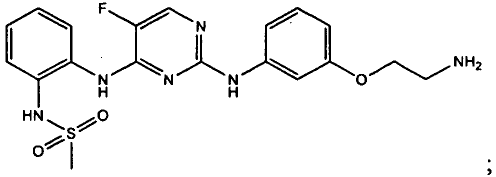 and 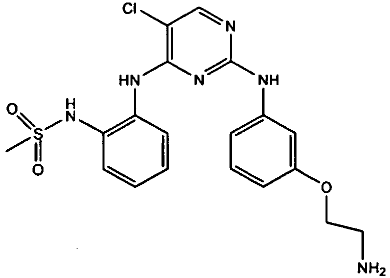 or a mixture of both.

The immobilization compounds of the present invention can be prepared by methods well known in the art. Exemplary analogous routes for the synthesis are described in, e.g., WO-A 2005/016894.

A general route for the synthesis of immobilization compounds of the present invention is shown in Scheme 1.

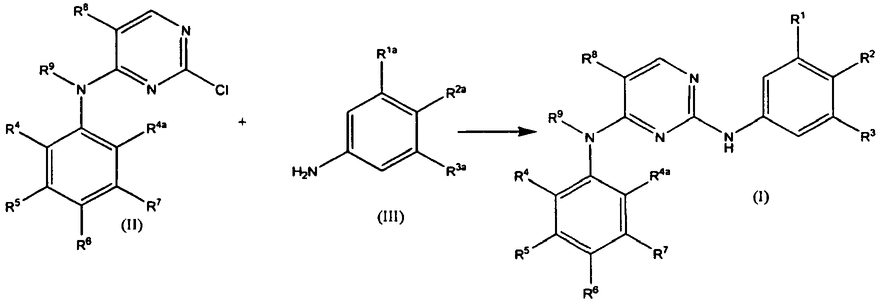

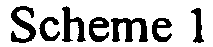

Accordingly, compounds of formula (II) and (III) may be reacted under elevated temperature and afterwards treated with an appropriate acid to de-protect the primary amino function of the residue X(CH₂)ₙNH₂ to yield compounds of formula (I), wherein R¹, R², R³, R⁴, R^{4a}, R⁵, R⁶, R⁷, R⁸, R⁹ have the meaning as indicated above and one of R^{1a}, R^{2a}, R^{3a} is X(CH₂)ₙNHC(O)O^{t}Bu and the others are defined as corresponding residues of R¹, R², R³.

By way of example a compound of formula (I), wherein R¹, R², R⁴, R⁵, R⁶, R¹, R⁹ are H; R⁸ is F; R^{3a} is X(CH₂)ₙNHC(O)O^{t}Bu, wherein n is 2 and X is O; and R^{4a} is NHS(O)₂CH₃ is shown in Scheme 2 (DMF = N,N-dimethyl formamide, Me = methyl, Bu = butyl, BOC = tert-butyloxycarbonyl).

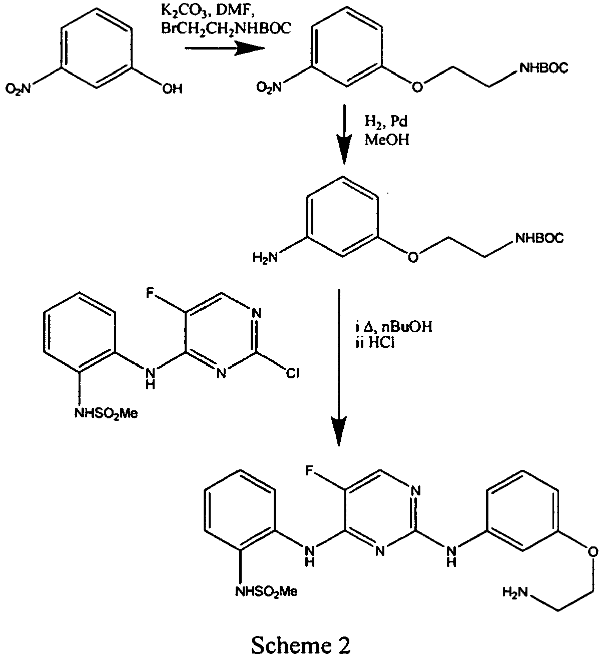

The invention further relates to a method for the preparation of an immobilization product, wherein at least one immobilization compound according to the invention is immobilized on a solid support. Such immobilization products obtainable according to the method of the invention are e.g. useful in the methods of the invention for the identification of kinase interacting compounds or in diagnostic methods for the diagnosis of myeloproliferative diseases.

According to the method of the invention, at least one immobilization compound of the invention is immobilized on a solid support. Throughout the invention, the term "solid support" relates to every undissolved support being able to immobilize a small molecule ligand on its surface.

According to the invention, the term "at least one immobilization compound" means either that at least one immobilization compound of the same type is immobilized on the solid support or that one or more different immobilization compounds (each of them either in singular or plural) may be immobilized on the solid support. Preferably, one or two different immobilization compounds are immobilized on the solid support, more preferably the preferred immobilization compounds of formula (I) of the present invention selected from the group consisting of 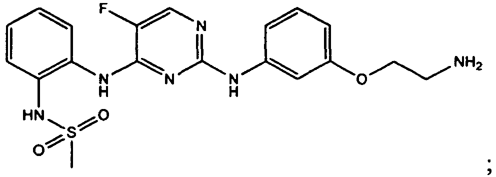 and 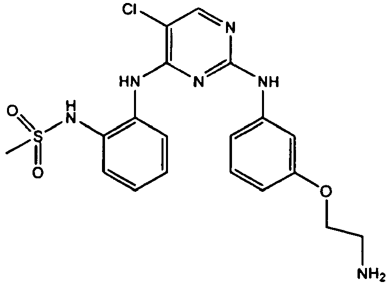 are immobilized.

The solid support may be selected from the group consisting of agarose, modified agarose, sepharose beads (e.g. NHS-activated sepharose), latex, cellulose, and ferro- or ferrimagnetic particles.

In case that the solid support is a material comprising various entities, e.g. in case that the solid support comprises several beads or particles, it is envisaged within the present invention that, if different immobilization compounds are immobilized, on each single entity, e.g. each bead or particle, one or more different immobilization compounds are immobilized. Therefore, in case that two immobilization compounds are used, it is envisaged within the present invention that on each single entity one or two different immobilization compounds are immobilized. If no measures are taken that on one entity only one different immobilization compound is immobilized, it is very likely that on each entity all different immobilization compounds will be present.

The immobilization compound or compounds of the invention may be coupled to the solid support either covalently or non-covalently. Non-covalent binding includes binding via biotin affinity ligands binding to steptavidin matrices.

Preferably, the immobilization compound or compounds are covalently coupled to the solid support.

Methods for immobilizing compounds on solid supports are known in the art and further exemplified in Example 1.

In general, before the coupling, the matrixes can contain active groups such as NHS, Carbodimide etc. to enable the coupling reaction with the immobilization compound. The immobilization compound can be coupled to the solid support by direct coupling (e.g. using functional groups such as amino-, sulfhydryl-, carboxyl-, hydroxyl-, aldehyde-, and ketone groups) and by indirect coupling (e.g. via biotin, biotin being covalently attached to the immobilization product of the invention and non-covalent binding of biotin to streptavidin which is bound directly to the solid support).

The linkage to the solid support material may involve cleavable and non-cleavable linkers. The cleavage may be achieved by enzymatic cleavage or treatment with suitable chemical methods.

Therefore, according to a preferred embodiment of the invention, the immobilization product results from a covalent direct or linker mediated attachment of the at least one immobilization compound of the invention to the solid support.

The linker may be a C₁₋₁₀ alkylene group, which is optionally interrupted or terminated by one or more atoms or functional groups selected from the group consisting of S, O, NH, C(O)O, C(O), and C(O)NH and wherein the linker is optionally substituted with one or more substituents independently selected from the group consisting of halogen, OH, NH₂, C(O)H, C(O)NH₂, SO₃H, NO₂, and CN.

The term "C₁₋₁₀ alkylene" means an alkylene chain having 1 - 10 carbon atoms, e.g. methylene, ethylene, -CH=CH-, -C≡C-, n-propylene and the like, wherein each hydrogen of a carbon atom may be replaced by a substituent.

The term "interrupted" means that the one or more atoms or functional groups are inserted between two carbon atoms of the alkylene chain or -when "terminated"- at the end of said chain.

Preferably, said immobilization occurs via the primary amino group in the residue X-(CH₂)ₙNH₂ for one of R¹, R², R³ in formula (I) above. More preferred, said amino group is part of an amid functional group, so that the immobilization occurs via amid bond forming of an immobilization compound of the present invention or a mixture thereof and optionally activated carboxylic acid functional groups of the solid support. Perhaps well known protective group techniques may be required during the immobilization step.

The invention further relates to an immobilization product, obtainable by the method of the invention.

Furthermore, the present invention relates to an immobilization product, comprising the immobilization compound of the invention immobilized on a solid support, in particular wherein the solid support is selected from the group consisting of agarose, modified agarose, sepharose beads (e.g. NHS-activated sepharose), latex, cellulose, and ferro- or ferrimagnetic particles.

Therefore, an immobilization product which is obtainable by the method of the invention is or comprises an immobilization compound immobilized on a solid support. This immobilization product will be referred to in the following as the immobilization product of the invention and is used in the methods of the present invention.

In a preferred embodiment, the immobilization compound or immobilization product of the invention may further be labelled.

By "labeled" is meant that the respective substance is either directly or indirectly labeled with a molecule which provides a detection signal, e.g. radioisotope, fluorescent tag, chemiluminescent tag, a peptide or specific binding molecules. Specific binding molecules include pairs, such as biotin and streptavidin, digoxin and antidigoxin. The label can directly or indirectly provide a detectable signal. The tag can also be a peptide which can be used, for example, in an enzyme fragment complementation assay (e.g. beta-galactosidase enzyme fragment complementation; Zaman et al., 2006. Assay Drug Dev. Technol. 4(4):411-420). The labeled compounds would be useful not only in imaging techniques but also in assays, both in vitro and in vivo, for identifying kinase interacting compounds by inhibition of binding of the labeled compound, for example in kinase assays that contain such labeled compounds.

Radioisotopes are commonly used in biological applications for the detection of a variety of biomolecules and have proven to be useful in binding assays. Several examples of probes have been designed to incorporate ³H (also written as T for tritium) because it can replace hydrogen in a probe without altering its structure (Fenteany et al., 1995. Science 268:726-731). An "isotopically" or "radio-labeled" compound is a compound of the invention where one or more atoms are replaced or substituted by an atom having an atomic mass or mass number different from the atomic mass or mass number typically found in nature (i.e., naturally occurring). Suitable radionuclides that may be incorporated in compounds of the present invention include but are not limited to ²H (also written D for Deuterium), ¹¹C, ¹³C, ¹⁴C, ¹³N, ¹⁵N, ¹⁵O, ¹⁷O, ¹⁸O, ¹⁸F, ³⁵S, ³⁶Cl, ⁸²Br, ⁷⁵Br, ⁷⁶Br, ⁷⁷Br, ¹²³I, ¹²⁴I, ¹²⁵I and ¹³¹I.

Guidance for the selection and methods for the attachment of fluorescent tags (e.g. fluorescein, rhodamine, dansyl, NBD (nitrobenz-2-oxa-1,3-diazole), BODIPY (dipyrromethene boron difluoride), and cyanine (Cy)-dyes) to small molecule ligands are generally known in the art (Vedvik et al., 2004. Assay Drug Dev. Technol. 2(2): 193-203; Zhang et al., 2005. Analytical Biochemistry 343(1):76-83). The application of fluorescent probes (fluorophores) in assays for high throughput screening (HTS) of protein kinases was described (Zaman et al., 2003. Comb. Chem. High Throughput Screen 6(4): 313-320). The change of the fluorescent properties after binding of the fluorescent probe to the target kinase can be determined by measuring for example fluorescence polarization (Kashem et al., 2007. J. Biomol. Screening 12(1):70-83), fluorescence resonance energy transfer (FRET; Zhang et al., 2005. Analytical Biochemistry 343(1):76-83) or fluorescence lifetime (Moger et al., 2006. J. Biomol. Screening 11(7): 765-772). In addition, the ALPHAScreen technology can be used where the excitation of a donor bead at 680 nm produces singlet oxygen which can diffuse to an acceptor bead undergoing a chemiluminescent reaction (Glickman et al., 2002. J. Biomol. Screen. 7(1):3-10).

One possible use of the immobilization products of the invention is in the context of the identification of compounds interacting with a variety of kinases, e.g. with kinases of the IRAK family or with NIK. Therefore, the present invention also relates to such methods and uses.

In a first aspect of the methods of the invention, the invention therefore relates to a method for the identification of a kinase interacting compound, comprising the steps of
a) providing a protein preparation containing a variety of kinases,
b) contacting the protein preparation with the immobilization product of the invention under conditions allowing the formation of one or more different complexes between one of the kinases and the immobilization product,
c) incubating the one or more different complexes with a given compound, and
d) determining whether the compound is able to separate the kinase from the immobilization product.

In a preferred embodiment, the variety of kinases includes one or more members of the IRAK family.

Consequently, in the context of this first aspect of the invention, the invention also relates to a method for the identification of a compound interacting with one or more members of the IRAK family, comprising the steps of
a) providing a protein preparation containing one or more members of the IRAK family,
b) contacting the protein preparation with the immobilization product of the invention under conditions allowing the formation of one or more different complexes between one of the members of the IRAK family and the immobilization product,
c) incubating the one or more different complexes with a given compound, and
d) determining whether the compound is able to separate the member of the IRAK family from the immobilization product.

In a further preferred embodiment, the variety of kinases includes NIK.

Consequently, in the context of this first aspect of the invention, the invention also relates to a method for the identification of a NIK interacting compound, comprising the steps of
a) providing a protein preparation containing NIK,
b) contacting the protein preparation with the immobilization product of the invention under conditions allowing the formation of a complex between NIK and the immobilization product,
c) incubating the complex with a given compound, and
d) determining whether the compound is able to separate NIK from the immobilization product.

In a second aspect, the present invention relates into a method for the identification of a kinase interacting compound, comprising the steps of
a) providing a protein preparation containing a variety of kinases,
b) contacting the protein preparation with the immobilization product of the invention and with a given compound under conditions allowing the formation of one or more different complexes between one of the kinases and the immobilization product, and
c) detecting the complex or the complexes formed in step b).

In a preferred embodiment, the variety of kinases includes one or more members of the IRAK family.

Consequently, in the context of this second aspect of the invention, the invention also relates to a method for the identification of a compound interacting with one or more members of the IRAK family, comprising the steps of
a) providing a protein preparation containing one or more members of the IRAK family,
b) contacting the protein preparation with the immobilization product of the invention and with a given compound under conditions allowing the formation of one or more different complexes between one of the members of the IRAK family and the immobilization product, and
c) detecting the complex or the complexes formed in step b).

In a further preferred embodiment, the variety of kinases includes NIK.

Consequently, in the context of this second aspect of the invention, the invention also relates to a method for the identification of a NIK interacting compound, comprising the steps of
a) providing a protein preparation containing NIK,
b) contacting the protein preparation with the immobilization product of the invention and with a given compound under conditions allowing the formation of a complex between NIK and the immobilization product, and
c) detecting the complex formed in step b).

In a third aspect, the present invention relates to a method for the identification of a kinase interacting compound, comprising the steps of:
a) providing two aliquots of a protein preparation containing a variety of kinases,
b) contacting one aliquot with the immobilization product of the invention under conditions allowing the formation of one or more different complexes between one of the kinases and the immobilization product,
c) contacting the other aliquot with the immobilization product of the invention and with a given compound under conditions allowing the formation of one or more different complexes between one of the kinases and the immobilization product, and
d) determining the amount of the complex or the complexes formed in steps b) and c).

In a preferred embodiment, the variety of kinases includes one or more members of the IRAK family.

Consequently, in the context of this third aspect of the invention, the invention also relates to a method for the identification of a compound interacting with one or more members of the IRAK family, comprising the steps of:
a) providing two aliquots of a protein preparation containing one or more members of the IRAK familly,
b) contacting one aliquot with the immobilization product of the invention under conditions allowing the formation of one or more different complexes between one member of the IRAK family and the immobilization product,
c) contacting the other aliquot with the immobilization product of the invention and with a given compound under conditions allowing the formation of one or more different complexes between one member of the IRAK family and the immobilization product, and
d) determining the amount of the complex or the complexes formed in steps b) and c).

In a further preferred embodiment, the variety of kinases includes NIK.

Consequently, in the context of this third aspect of the invention, the invention also relates to a method for the identification of a NIK interacting compound, comprising the steps of:
a) providing two aliquots of a protein preparation containing NIK,
b) contacting one aliquot with the immobilization product of the invention under conditions allowing the formation of a complex between NIK and the immobilization product,
c) contacting the other aliquot with the immobilization product of the invention and with a given compound under conditions allowing the formation of a complex between NIK and the immobilization product, and
d) determining the amount of the complex formed in steps b) and c).

In a fourth aspect, the invention relates to a method for the identification of a kinase interacting compound, comprising the steps of:
a) providing two aliquots comprising each at least one cell containing a variety of kinases,
b) incubating one aliquot with a given compound,
c) harvesting the cells of each aliquot,
d) lysing the cells in order to obtain protein preparations,
e) contacting the protein preparations with the immobilization product of the invention under conditions allowing the formation of one or more different complexes between one of the kinases and the immobilization product, and
f) determining the amount of the complex or the complexes formed in each aliquot in step e).

In a preferred embodiment, the variety of kinases includes one or more members of the IRAK family.

Consequently, in the context of this fourth aspect of the invention, the invention also relates to a method for the identification of a compound interacting with one or more members of the IRAK family, comprising the steps of:
a) providing two aliquots comprising each at least one cell containing one or more members of the IRAK family,
b) incubating one aliquot with a given compound,
c) harvesting the cells of each aliquot,
d) lysing the cells in order to obtain protein preparations,
e) contacting the protein preparations with the immobilization product of the invention under conditions allowing the formation of one or more different complexes between one member of the IRAK family and the immobilization product, and
determining the amount of the complex or the complexes formed in each aliquot in step e).

In a further preferred embodiment, the variety of kinases includes NIK.

Consequently, in the context of this fourth aspect of the invention, the invention also relates to a method for the identification of a NIK interacting compound, comprising the steps of:
a) providing two aliquots comprising each at least one cell containing NIK,
b) incubating one aliquot with a given compound,
c) harvesting the cells of each aliquot,
d) lysing the cells in order to obtain protein preparations,
e) contacting the protein preparations with the immobilization product of the invention under conditions allowing the formation of a complexes between NIK and the immobilization product, and
f) determining the amount of the complex formed in each aliquot in step e).

In the context of the present invention, it has been found that the immobilization products of the present invention are suitable for the identification of compounds interacting with kinases in several ways:
a) The immobilization products of the present invention bind to a variety of kinases. Especially, they bind to kinases listed in tables 4, 5, 6 and 7 shown in the examples.

For example, the following kinases were identified in example 2 (Table 4):
AAK1, ACK, ALK4, AMPKa1, AurA, AurB, BIKE, BTK, CaMK2d, CaMK2g, CaMKK2, CDC2, CDK10, CDK2, CDK5, CDK9, CHK1, CK2a1, CK2a2, CLK1, CRK7, CSK, DNAPK, DYRK1A, EphB6, Erk1, Erk2, FER, FES, GAK, GCK, GSK3A, GSK3B, HIP1, HIPK2, HPK1, IKKe, IRAK1, IRAK3, IRAK4, IRE1, JAK1, JAK2, JNK1, JNK2, KHS1, LCK, LOK, LRRK2, MAP2K1, MAP2K3, MAP2K5, MAP3K1, MAP3K2, MAP3K3, MAP3K4, MAP3K5, MARK2, MARK3, MLK3, MLK4, MPSK1, MSK1, MSK2, MST1, MST4, MYT1, NEK3, NEK9, PAK4, PHKg2, PIP5K2A, PIP5K2C, PITSLRE, PKCa, PKCb, PKD2, PKD3, PKN1, PKN2, PLK1, PLK4, PYK2, QIK, RET, RSK2, RSK3, SLK, STK33, SYK, TAO3, TBK1, TEC, TGFbR1, TYK2, ULK3, Weel, YES.

In addition, for example, the following kinases were identified in example 3 (Table 5): ADCK1, ALK2, ARG, BCR, DRAK2, HRI, INSR, JAK3, LYN, MAP2K6, MST2, NEK6, NEK7, NIK, PIK4Ca, RIPK2, RSK4, ULK1.

In addition, for example, the following kinases were identified in example 4 (Table 6): AMPKa2, CaMKK1, FAK, FLT4, KDR, MRCKa, MRCKb, PKG1, SRC, TIE2, TNK1.

In addition, for example, the following kinases were identified in example 5 (Table 7): BMPR1A, DDR1, FYN, HIPK3, MAP3K7, MER, MET, PIP5K2B, RON, ULK4.

Consequently, in the methods of the present invention, these immobilization products can be used to identify compounds binding to at least one kinase out of said variety of kinases.
b) The immobilization products of the present invention bind to the IRAK family and are therefore suitable for the identification of compounds interacting with said kinase family.
c) The immobilization compounds of the present invention bind to NIK and are therefore suitable for the identification of compounds interacting with NIK.

According to the present invention, a member of the IRAK family can either be IRAK 1, IRAK 2, IRAK 3 and IRAK 4.

According to a preferred embodiment of the invention, the member of the IRAK family is IRAK 1 or IRAK 4.

According to the invention, the term IRAK refers to "interleukin-1 receptor-associated kinase", comprising IRAK1, IRAK2, LRAK3 and IRAK4 (see above).

According to the present invention, the term NIK. refers to "NF-kappa-B-inducing kinase" (see above).

According to the present invention, the expression "NIK" or "IRAK"relates to both human and other proteins of this family. The expression especially includes functionally active derivatives thereof, or functionally active fragments thereof, or a homologues thereof, or variants encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1 % SDS for 1.5 hours at 60°C.

Moreover, according to the present invention, the expression "NIK" or "IRAK" includes mutant forms said kinases.

In some aspects of the invention, first a protein preparation containing said kinases or kinase is provided. The methods of the present invention can be performed with any protein preparation as a starting material, as long as the respective kinase is solubilized in the preparation. Examples include a liquid mixture of several proteins, a cell lysate, a partial cell lysate which contains not all proteins present in the original cell or a combination of several cell lysates. The term "protein preparation" also includes dissolved purified protein.

The presence of the kinases in a protein preparation of interest can be detected on Western blots probed with antibodies that are specifically directed against said kinase. Alternatively, also mass spectrometry (MS) could be used to detect the kinases (see below).

Cell lysates or partial cell lysates can be obtained by isolating cell organelles (e.g. nucleus, mitochondria, ribosomes, golgi etc.) first and then preparing protein preparations derived from these organelles. Methods for the isolation of cell organelles are known in the art (Chapter 4.2 Purification of Organelles from Mammalian Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

In addition, protein preparations can be prepared by fractionation of cell extracts thereby enriching specific types of proteins such as cytoplasmic or membrane proteins (Chapter 4.3 Subcellular Fractionation of Tissue Culture Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

Furthermore protein preparations from body fluids can be used (e.g. blood, cerebrospinal fluid, peritoneal fluid and urine).

For example whole embryo lysates derived from defined development stages or adult stages of model organisms such as C. elegans can be used. In addition, whole organs such as heart dissected from mice can be the source of protein preparations. These organs can also be perfused in vitro in order to obtain a protein preparation.

Furthermore, the protein preparation may be a preparation containing the kinase or the kinases which has been recombinantely produced. Methods for the production of recombinant proteins in prokaryotic and eukaryotic cells are widely established (Chapter 5 Production of Recombinant Proteins in "Current Protocols in Protein Science", Editors: John. E. Coligan, Ben M. Dunn, Hidde L. Ploegh, David W. Speicher, Paul T. Wingfield; Wiley, 1995, ISBN: 0-471-14098-8).

In a preferred embodiment of the methods of the invention, the provision of a protein preparation includes the steps of harvesting at least one cell containing the kinase or the kinases and lysing the cell.

Suitable cells for this purpose are e.g. those cells or tissues where the kinases are expressed. In any given cell or tissue only a subset of the kinome may be expressed. Therefore it may be necessary to generate multiple protein preparations from a variety of cell types and tissues to cover the kinome, especially for selectivity profiling of kinase inhibitors. As established cell lines may not reflect the physiological expression pattern of kinases, primary animal or human cells may be used, for example cells isolated from blood samples.

Therefore, in a preferred embodiment, cells isolated from peripheral blood represent a suitable biological material. Procedures for the preparation and culture of human lymphocytes and lymphocyte subpopulations obtained from peripheral blood (PBLs) are widely known (W.E Biddison, Chapter 2.2 "Preparation and culture of human lymphocytes" in Current Protocols in Cell Biology, 1998, John Wiley & Sons, Inc.). For example, density gradient centrifugation is a method for the separation of lymphocytes from other blood cell populations (e.g. erythrocytes and granulocytes). Human lymphocyte subpopulations can be isolated via their specific cell surface receptors which can be recognized by monoclonal antibodies. The physical separation method involves coupling of these antibody reagents to magnetic beads which allow the enrichment of cells that are bound by these antibodies (positive selection).

As an alternative to primary human cells cultured cell lines (e.g. MOLT-4 cells, Jurkat, Ramos or HeLa cells) can be used.

In a preferred embodiment, the cell is part of a cell culture system and methods for the harvest of a cell out of a cell culture system are known in the art (literature supra).

The choice of the cell will mainly depend on the expression of the kinases, since it has to be ensured that the protein is principally present in the cell of choice. In order to determine whether a given cell is a suitable starting system for the methods of the invention, methods like Westemblot, PCR-based nucleic acids detection methods, Northernblots and DNA-microarray methods ("DNA chips") might be suitable in order to determine whether a given protein of interest is present in the cell.

The choice of the cell may also be influenced by the purpose of the study. If the in vivo efficacy for a given drug needs to be analyzed then cells or tissues may be selected in which the desired therapeutic effect occurs (e.g. B-cells). By contrast, for the elucidation of protein targets mediating unwanted side effects the cell or tissue may be analysed in which the side effect is observed (e.g. cardiomyocytes, vascular smooth muscle or epithelium cells).

Furthermore, it is envisaged within the present invention that the cell containing the kinases or the kinase may be obtained from an organism, e.g. by biopsy. Corresponding methods are known in the art. For example, a biopsy is a diagnostic procedure used to obtain a small amount of tissue, which can then be examined microscopically or with biochemical methods. Biopsies are important to diagnose, classify and stage a disease, but also to evaluate and monitor drug treatment.

It is encompassed within the present invention that by the harvest of the at least one cell, the lysis is performed simultaneously. However, it is equally preferred that the cell is first harvested and then separately lysed.

Methods for the lysis of cells are known in the art (Karwa and Mitra: Sample preparation for the extraction, isolation, and purification of Nuclei Acids; chapter 8 in "Sample Preparation Techniques in Analytical Chemistry", Wiley 2003, Editor: Somenath Mitra, print ISBN: 0471328456; online ISBN: 0471457817). Lysis of different cell types and tissues can be achieved by homogenizers (e.g. Potter-homogenizer), ultrasonic desintegrators, enzymatic lysis, detergents (e.g. NP-40, Triton X-100, CHAPS, SDS), osmotic shock, repeated freezing and thawing, or a combination of these methods.

According to the methods of the invention, the protein preparation containing one or more kinases is contacted with the immobilization product under conditions allowing the formation of a complex between the said kinase and the immobilization product of the invention.

In the present invention, the term "a complex between a kinase and the immobilization product" denotes a complex where the immobilization product interacts with a kinase , e.g. by covalent or, most preferred, by non-covalent binding.
In the context of the present invention, compounds are identified which interfere with the formation of a complex between the immobilization product and a kinase present in a cell or protein preparation. In case that only one kinase is to be detected or present, the formation of one complex is observed and tested. In case that several kinases are to be detected or present, the formation of several, different complexes is observed and tested.

The skilled person will know which conditions can be applied in order to enable the formation of said complex.

In the context of the present invention, the term "under conditions allowing the formation of the complex" includes all conditions under which such formation, preferably such binding is possible. This includes the possibility of having the solid support on an immobilized phase and pouring the lysate onto it. In another preferred embodiment, it is also included that the solid support is in a particulate form and mixed with the cell lysate. Such conditions are known to the person skilled in the art.

In the context of non-covalent binding, the binding between the immobilization product and the kinase is, e.g., via salt bridges, hydrogen bonds, hydrophobic interactions or a combination thereof.

In a preferred embodiment, the steps of the formation of said complex are performed under essentially physiological conditions. The physical state of proteins within cells is described in Petty, 1998 (Howard R. Petty1, Chapter 1, Unit 1.5 in: Juan S. Bonifacino, Mary Dasso, Joe B. Harford, Jennifer Lippincott-Schwartz, and Kenneth M. Yamada (eds.) Current Protocols in Cell Biology Copyright © 2003 John Wiley & Sons, Inc. All rights reserved. DOI: 10.1002/0471143030.cb0101s00Online Posting Date: May, 2001Print Publication Date: October, 1998).

The contacting under essentially physiological conditions has the advantage that the interactions between the ligand, the cell preparation (i. e. the kinase to be characterized) and optionally the compound reflect as much as possible the natural conditions. "Essentially physiological conditions" are *inter alia* those conditions which are present in the original, unprocessed sample material. They include the physiological protein concentration, pH, salt concentration, buffer capacity and post-translational modifications of the proteins involved. The term "essentially physiological conditions" does not require conditions identical to those in the original living organism, wherefrom the sample is derived, but essentially cell-like conditions or conditions close to cellular conditions. The person skilled in the art will, of course, realize that certain constraints may arise due to the experimental set-up which will eventually lead to less cell-like conditions. For example, the eventually necessary disruption of cell walls or cell membranes when taking and processing a sample from a living organism may require conditions which are not identical to the physiological conditions found in the organism. Suitable variations of physiological conditions for practicing the methods of the invention will be apparent to those skilled in the art and are encompassed by the term "essentially physiological conditions" as used herein. In summary, it is to be understood that the term "essentially physiological conditions" relates to conditions close to physiological conditions, as e. g. found in natural cells, but does not necessarily require that these conditions are identical.

For example, "essentially physiological conditions" may comprise 50-200 mM NaCl or KCI, pH 6.5-8.5, 20-37°C, and 0.001-10 mM divalent cation (e.g. Mg++, Ca++,); more preferably about 150 m NaCl or KCl, pH7.2 to 7.6, 5 mM divalent cation and often include 0.01-1.0 percent non-specific protein (e.g. BSA). A non-ionic detergent (Tween, NP-40, Triton-X100) can often be present, usually at about 0.001 to 2%, typically 0.05-0.2% (volume/volume). For general guidance, the following buffered aequous conditions may be applicable: 10-250 mM NaCl, 5-50 mM Tris HCl, pH5-8, with optional addition of divalent cation(s) and/or metal chelators and/or non-ionic detergents.

Preferably, "essentially physiological conditions" mean a pH of from 6.5 to 7.5, preferably from 7.0 to 7.5, and / or a buffer concentration of from 10 to 50 mM, preferably from 25 to 50 mM, and / or a concentration of monovalent salts (e.g. Na or K) of from 120 to 170 mM, preferably 150 mM. Divalent salts (e.g. Mg or Ca) may further be present at a concentration of from 1 to 5 mM, preferably 1 to 2 mM, wherein more preferably the buffer is selected from the group consisting of Tris-HCl or HEPES.

The skilled person will appreciate that between the individual steps of the methods of the invention, washing steps may be necessary. Such washing is part of the knowledge of the person skilled in the art. The washing serves to remove non-bound components of the cell lysate from the solid support. Nonspecific (e.g. simple ionic) binding interactions can be minimized by adding low levels of detergent or by moderate adjustments to salt concentrations in the wash buffer.

According to the identification methods of the invention, the read-out system is either the detection or determination of a kinase (first aspect of the invention), the detection of the complex between a kinase and the immobilization product (second aspect of the invention), or the determination of the amount of the complex between a kinase and the immobilization product (second, third and fourth aspect of the invention).

In the method according to the first aspect of the invention, the detection or determination of the amount of separated kinase is preferably indicative for the fact that the compound is able to separate the kinase from the immobilization product. This capacity indicates that the respective compound interacts, preferably binds to the kinase, which is indicative for its therapeutic potential.

In one embodiment of the method according to the second aspect of the invention, the complex formed during the method of the invention is detected. The fact that such complex is formed preferably indicates that the compound does not completely inhibit the formation of the complex. On the other hand, if no complex is formed, the compound is presumably a strong interactor with the kinase, which is indicative for its therapeutic potential.

According to the methods of the second, third and fourth aspect of the invention the amount of the complex formed during the method is determined. In general, the less complex in the presence of the respective compound is formed, the stronger the respective compound interacts with the kinase, which is indicative for its therapeutic potential.

The detection of the complex formed according to the second aspect of the invention can be performed by using labeled antibodies directed against the kinase and a suitable readout system.

According to a preferred embodiment of the second aspect of the invention, the complex between one kinase and the immobilization product is detected by determining its amount.

In the course of the second, third and fourth aspect of the invention, it is preferred that the kinase are separated from the immobilization product in order to determine the amount of said complex.

According to invention, separating means every action which destroys the interactions between the immobilization compound and the kinase. This includes in a preferred embodiment the elution of the kinase from the immobilization compound.

The elution can be achieved by using non-specific reagents as described in detail below (ionic strength, pH value, detergents). In addition, it can be tested whether a compound of interest can specifically elute the the kinase from the immobilization compound. Such kinase interacting compounds are described further in the following sections.

Such non-specific methods for destroying the interaction are principally known in the art and depend on the nature of the ligand enzyme interaction. Principally, change of ionic strength, the pH value, the temperature or incubation with detergents are suitable methods to dissociate the target enzymes from the immobilized compound. The application of an elution buffer can dissociate binding partners by extremes of pH value (high or low pH; e.g. lowering pH by using 0.1 M citrate, pH2-3), change of ionic strength (e.g. high salt concentration using NaI, KI, MgCl₂, or KCl), polarity reducing agents which disrupt hydrophobic interactions (e.g. dioxane or ethylene glycol), or denaturing agents (chaotropic salts or detergents such as Sodium-docedyl-sulfate, SDS; Review: Subramanian A., 2002, Immunoaffinty chromatography).

In some cases, the solid support has preferably to be separated from the released material. The individual methods for this depend on the nature of the solid support and are known in the art. If the support material is contained within a column the released material can be collected as column flowthrough. In case the support material is mixed with the lysate components (so called batch procedure) an additional separation step such as gentle centrifugation may be necessary and the released material is collected as supernatant. Alternatively magnetic beads can be used as solid support so that the beads can be eliminated from the sample by using a magnetic device.

In step d) of the method according to the first aspect of the invention, it is determined if the kinase has been separated from the immobilization product of the invention. This may include the detection of the kinase or the determination of the amount of the kinase.

Consequently, at least in preferred embodiments of all identification methods of the invention, methods for the detection of a separated kinase or for the determination of their amount are used. Such methods are known in the art and include physico-chemical methods such as protein sequencing (e.g. Edmann degradation), analysis by mass spectrometry methods or immunodetection methods employing antibodies directed against the kinase.

Throughout the invention, if an antibody is used in order to detect a kinase or in order to determine its amount (e.g. via ELISA), the skilled person will understand that, if a specific isoform of a kinase is to be detected or if the amount of a specific isoform of a kinase is to be determined, an isoform-specific antibody may be used. As indicated above, such antibodies are known in the art. Furthermore, the skilled person is aware of methods for producing the same.

Preferably, a kinase is detected or the amount of a kinase is determined by mass spectrometry or immunodetection methods.

The identification of proteins with mass spectrometric analysis (mass spectrometry) is known in the art (Shevchenko et al., 1996, Analytical Chemistry 68: 850-858; Mann et al., 2001, Analysis of proteins and proteomes by mass spectrometry, Annual Review of Biochemistry 70, 437-473) and is further illustrated in the example section.

Preferably, the mass spectrometry analysis is performed in a quantitative manner, for example by using iTRAQ technology (isobaric tags for relative and absolute quatification) or cICAT (cleavable isotope-coded affinity tags) (Wu et al., 2006. J. Proteome Res. 5,651-658).

According to a further preferred embodiment of the present invention, the characterization by mass spectrometry (MS) is performed by the identification of proteotypic peptides of the kinase. The idea is that the kinase is digested with proteases and the resulting peptides are determined by MS. As a result, peptide frequencies for peptides from the same source protein differ by a great degree, the most frequently observed peptides that "typically" contribute to the identification of this protein being termed "proteotypic peptide". Therefore, a proteotypic peptide as used in the present invention is an experimentally well observable peptide that uniquely identifies a specific protein or protein isoform.

According to a preferred embodiment, the characterization is performed by comparing the proteotypic peptides obtained in the course of practicing the methods of the invention with known proteotypic peptides. Since, when using fragments prepared by protease digestion for the identification of a protein in MS, usually the same proteotypic peptides are observed for a given enzyme, it is possible to compare the proteotypic peptides obtained for a given sample with the proteotypic peptides already known for enzymes of a given class of enzymes and thereby identifying the enzyme being present in the sample.

As an alternative to mass spectrometry analysis, the eluted kinase (including coeluted binding partners or scaffold proteins), can be detected or its amount can be determined by using a specific antibody directed against the kinase (or against an isoform of a kinase, see above).

Furthermore, in another preferred embodiment, once the identity of the coeluted binding partner has been established by mass spectrometry analysis, each binding partner can be detected with specific antibodies directed against this protein.

Suitable antibody-based assays include but are not limited to Western blots, ELISA assays, sandwich ELISA assays and antibody arrays or a combination thereof. The establishment of such assays is known in the art (Chapter 11, Immunology, pages 11-1 to 11-30 in: Short Protocols in Molecular Biology. Fourth Edition, Edited by F.M. Ausubel et al., Wiley, New York, 1999).

These assays can not only be configured in a way to detect and quantify a kinase interacting protein of interest (e.g. a catalytic or regulatory subunit of a kinase complex), but also to analyse posttranslational modification patterns such as phosphorylation or ubiquitin modification.

Furthermore, the identification methods of the invention involve the use of compounds which are tested for their ability to be a kinase interacting compound.

Principally, according to the present invention, such a compound can be every molecule which is able to interact with the kinase, eg. by inhibiting its binding to the immobilization product of the invention. Preferably, the compound has an effect on the kinase, e.g. a stimulatory or inhibitory effect.

Preferably, said compound is selected from the group consisting of synthetic or naturally occurring chemical compounds or organic synthetic drugs, more preferably small molecule organic drugs or natural small molecule compounds. Preferably, said compound is identified starting from a library containing such compounds. Then, in the course of the present invention, such a library is screened.

Such small molecules are preferably not proteins or nucleic acids. Preferably, small molecules exhibit a molecular weight of less than 1000 Da, more preferred less than 750 Da, most preferred less than 500 Da.

A "library" according to the present invention relates to a (mostly large) collection of (numerous) different chemical entities that are provided in a sorted manner that enables both a fast functional analysis (screening) of the different individual entities, and at the same time provide for a rapid identification of the individual entities that form the library. Examples are collections of tubes or wells or spots on surfaces that contain chemical compounds that can be added into reactions with one or more defined potentially interacting partners in a high-throughput fashion. After the identification of a desired "positive" interaction of both partners, the respective compound can be rapidly identified due to the library construction. Libraries of synthetic and natural origins can either be purchased or designed by the skilled artisan.

Examples of the construction of libraries are provided in, for example, Breinbauer R, Manger M, Scheck M, Waldmann H. Natural product guided compound library development. Curr. Med. Chem. 2002 Dec;9(23):2129-45, wherein natural products are described that are biologically validated starting points for the design of combinatorial libraries, as they have a proven record of biological relevance. This special role of natural products in medicinal chemistry and chemical biology can be interpreted in the light of new insights about the domain architecture of proteins gained by structural biology and bioinformatics. In order to fulfill the specific requirements of the individual binding pocket within a domain family it may be necessary to optimise the natural product structure by chemical variation. Solid-phase chemistry is said to become an efficient tool for this optimisation process, and recent advances in this field are highlighted in this review article. Other related references include Edwards PJ, Morrell AI. Solid-phase compound library synthesis in drug design and development. Curr Opin Drug Discov Devel. 2002 Jul;5(4):594-605.; Merlot C, Domine D, Church DJ. Fragment analysis in small molecule discovery. Curr Opin Drug Discov Devel. 2002 May;5(3):391-9. Review; Goodnow RA Jr. Current practices in generation of small molecule new leads. J Cell Biochem Suppl. 2001;Suppl 37:13-21; which describes that the current drug discovery processes in many pharmaceutical companies require large and growing collections of high quality lead structures for use in high throughput screening assays. Collections of small molecules with diverse structures and "drug-like" properties have, in the past, been acquired by several means: by archive of previous internal lead optimisation efforts, by purchase from compound vendors, and by union of separate collections following company mergers. Although high throughput/combinatorial chemistry is described as being an important component in the process of new lead generation, the selection of library designs for synthesis and the subsequent design of library members has evolved to a new level of challenge and importance. The potential benefits of screening multiple small molecule compound library designs against multiple biological targets offers substantial opportunity to discover new lead structures.

In a preferred embodiment of the second and third aspect of the invention, the kinase containing protein preparation is first incubated with the compound and then with the immobilization product. However, the simultaneous incubation of the compound and the immobilization product of the invention (coincubation) with the kinase containing protein preparation is equally preferred (competitive binding assay).

In case that the incubation with the compound is first, the kinase is preferably first incubated with the compound for 10 to 60 minutes, more preferred 30 to 45 minutes at a temperature of 4°C to 37°C, more preferred 4°C to 25°C, most preferred 4°C. Preferably compounds are used at concentrations ranging from 1 nM to 100 µM, preferably from 10 nM to 10 µM. The second step, contacting with the immobilized ligand, is preferably performed for 10 to 60 minutes at 4°C.

In case of simultaneous incubation, the kinase is preferably simultaneously incubated with the compound and the immobilization product of the invention for 30 to 120 minutes, more preferred 60 to 120 minutes at a temperature of 4°C to 37°C, more preferred 4°C to 25°C, most preferred 4°C. Preferably compounds are used at concentrations ranging from I nM to 100 µM, preferably from 10 nM to 10 µM.

Furthermore, steps a) to c) of the second aspect of the invention may be performed with several protein preparations in order to test different compounds. This embodiment is especially interesting in the context of medium or high throughput screenings (see below).

In a preferred embodiment of the method of the invention according to the third or fourth aspect, the amount of the complex formed in step c) is compared to the amount formed in step b)

In a preferred embodiment of the method of the invention according to the third or fourth aspect, a reduced amount of the complex formed in step c) in comparison to step b) indicates that a kinase is a target of the compound. This results from the fact that in step c) of this method of the invention, the compound competes with the immobilized compound for the binding of the kinase. If less kinase is present in the aliquot incubated with the compound, this means preferably that the compound has competed with the inhibitor for the interaction with the enzyme and is, therefore, a direct target of the protein and vice versa.

Preferably, the identification methods of the invention are performed as a medium or high throughput screening.

The interaction compound identified according to the present invention may be further characterized by determining whether it has an effect on the kinase, for example on its kinase activity (Davies et al., 2000. Biochemical Journal 351(Pt 1):95-105; Bain et al., 2007. Biochemical Journal 408(3):297-315).

The compounds identified according to the present invention may further be optimized (lead optimisation). This subsequent optimisation of such compounds is often accelerated because of the structure-activity relationship (SAR) information encoded in these lead generation libraries. Lead optimisation is often facilitated due to the ready applicability of high-throughput chemistry (HTC) methods for follow-up synthesis.

An example for lead optimization of IRAK kinase inhibitors was reported (Buckley et al., 2008. Bioorg Med Chem Lett. 18(12):3656-60).

The invention further relates to a method for the preparation of a pharmaceutical composition comprising the steps of
a) identifying a kinase interacting compound as described above, and
b) formulating the interacting compound to a pharmaceutical composition.

Methods for the formulation of identified compounds are known in the art. Furthermore, it is known in the art how to administer such pharmaceutical compositions.

The obtained pharmaceutical composition can be used for the prevention or treatment of diseases where the respective kinase plays a role, e.g. for the prevention or treatment of cancer (Zhang et al., 2009. Nature Reviews Cancer 9, 28-39) or inflammatory diseases (Cohen, 2009. Current Opinion in Cell Biology 21, 1-8). For example, IRAK4 inhibitors may be useful for the treatment of chronic inflammatory diseases in adults without making them too susceptible to bacterial and viral infections. IRAK1 is essential for the TLR7-mediated and TLR9-mediated activation of IRF7 and the production of interferon-alpha (IFN-α) suggesting that IRAK1 inhibitors may be useful for the treatment of Systemic lupus erythematosus (SLE). IRAK2 is activated downstream of IRAK4 and plays a role in proinflammatory cytokine production. Therefore IRAK2 inhibitors may be useful for inflammatory diseases. Drugs that inhibit NIK may be useful for the treatment multiple myeloma, a cancer of the immune system (Annunziata et al., 2007. Cancer Cell 12(2):115-130; Keats et al., 2007. Cancer Cell 12(2):131-144).

The invention further relates to a method for the purification of a kinase, comprising the steps of
a) providing a protein preparation containing said kinase,
b) contacting the protein preparation with the immobilization product of the invention under conditions allowing the formation of a complex between the kinase and the immobilization product, and
c) separating the kinase from the immobilization product.

As mentioned above, it has been surprisingly found that the compound of the invention and therefore also the immobilization product of the invention is a ligand which recognizes the kinases mentioned above. This enables efficient purification methods for said kinases.

Preferred kinases to be purified include IRAK1, IRAK4 or NIK.

With respect to the kinases, the protein preparation containing the kinases, the conditions for contacting with the immobilization product of the invention, the immobilization product of the invention, the complex between the kinases and the immobilization product of the invention, the separation of the kinases from the immobilization product of the invention, and the detection of the kinases or the determination of its amount, the embodiments as defined above for the identification methods of the invention also apply to the purification method of the invention.

In a preferred embodiment, the method of purification further comprises the step of purifying a specific isoform or specific isoforms of said kinases, preferably the step of purifying IRAK1, or IRAK4.

Preferably, said purification is performed using an isoform specific antibody as explained above, more preferably an IRAK1 or IRAK4 specific antibody.

In a preferred embodiment, the purification method of the invention further comprises after step c) the identification of proteins being capable of binding to said kinases. This is especially interesting when the formation of the complex is performed under essentially physiological conditions, because it is then possible to preserve the natural condition of the enzyme which includes the existence of binding partners, enzyme subunits or post-translational modifications, which can then be identified with the help of mass spectrometry (MS).

Consequently, in a preferred embodiment, the purification method of the invention further comprises after step c) the determination whether the kinase is further posttranslationally modified, e.g. by ubiquitin modification.

The binding proteins or the posttranslational modifications can be determined as explained above for the detection of kinases or the determination of the amount of kinases. Preferably, said methods include mass spectrometry of imunodetection methods as described above.

The invention further relates to a method for determining the presence of one or more kinases in a sample, comprising the steps of:
a) providing a protein preparation expected to contain said one or more kinases,
b) contacting the protein preparation with the immobilization product of the invention under conditions allowing the formation of a complex between one of the kinases and the immobilization product, and
c) detecting whether one or more kinases have formed a complex with the immobilization product.

In a preferred embodiment of the invention, said detecting in step c) is performed by separating said one or more kinases from the immobilization product and further identification of said one or more kinases.

Said identification may be performed by mass spectrometry or immunodetection methods as described above.

Preferably, also in the context of this method of the invention the kinase is IRAK1, IRAK4 or NIK.

According to an especially preferred embodiment of this method of the invention, the kinase contains at least one mutation.

With respect to said one or more kinases, the protein preparation containing said kinases, the conditions for contacting with the immobilization product of the invention, the immobilization product of the invention, the complex between said kinase and the immobilization product of the invention, the separation of kinases from the immobilization product of the invention, and the detection of kinases or the determination of its amount, the embodiments as defined above for the identification methods of the invention also apply to the purification method of the invention.

The invention further relates to the use of compound or the immobilization product of the invention for the identification of a kinase interacting compounds and for the purification of a kinase. The embodiments as defined above also apply to the uses of the invention.

The invention further relates to a kit comprising the compound or the immobilization product of the invention. Such a kit is especially useful for performing the methods of the invention. Further components of the kit may be antibodies for the detection of kinase proteins, for example antibodies specific for IRAK1 and/or IRAK4 or NIK. Such antibodies and their use are known in the art and they are commercially available (Davidson et al., 2006. J. Immunology 177, 8202-8211; Saitoh et al., 2008. Blood 111, 5118-5129). Furthermore, the kit may contain further auxiliary components like buffers, means for the detection of antibodies, and positive controls. Such components are known in the art.

The invention is further illustrated by the following figures and examples, which are not considered as being limiting for the scope of protection conferred by the claims of the present application. In case where in the following examples the term "affinity matrix" is used, this term refers to an immobilization product as defined in the present application.

### Short description of the figures

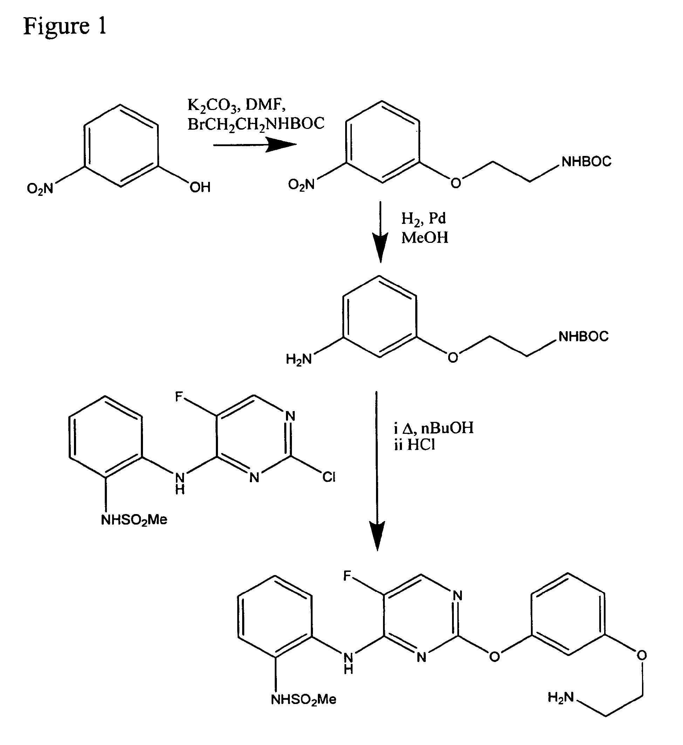

**Figure 1****:** Synthesis of N-(2-(2-(3-(2-aminoethoxy)phenylamino)-5-fluoropyrimidin-4-ylamino)phenyl)methanesulfonamide. The compound was synthesized as described in example 1.
**Figure 2****:** Structure of N-(2-(2-(3-(2-aminoethoxy)phenylamino)-5-fluoropyrimidin-4-ylamino)phenyl)methanesulfonamide (CZC25342).
**Figure 3****:** Structure of N-(2-(2-(3-(2-aminoethoxy)phenylamino)-5-chloropyrimidin-4 ylamino)phenyl)methanesulfonamide (CZC31326).
**Figure 4****:** Kinobeads experiment with the immobilized compound (Figure 3, CZC31326) for mass spectrometry analysis of captured proteins.
   A protein gel after staining with Coomassie brilliant blue is shown. The experiment was performed as described in example 2 with HL60 cell lysate. Proteins bound to the affinity matrix were eluted with SDS sample buffer and separated by SDS-polyacrylamide gel electrophoresis. The indicated gel areas were cut out as gel slices, proteins were treated with trypsin and ITRAQ-labeled peptides were analysed by mass spectrometry.
   Left lane (P29577B): cell lysate treated with 10 µM free compound CZC31326; middle lane: protein molecular weight marker; right lane (P29578B): DMSO control.

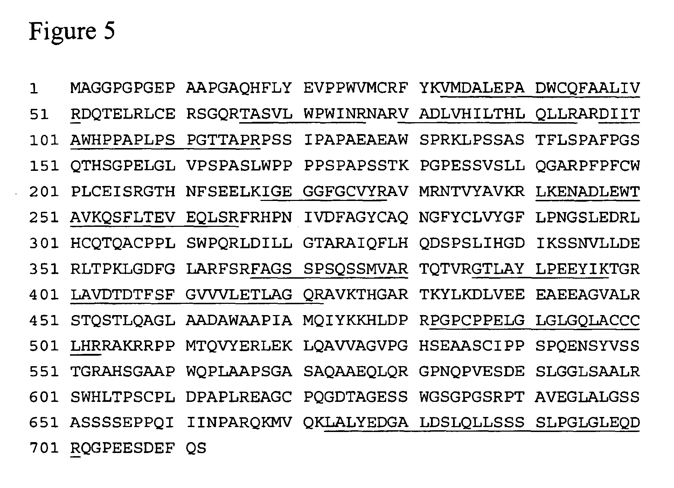

**Figure 5****:** Amino acid sequence of human IRAK1 (IPI00293652.1). Peptides identified by mass spectrometry are underlined.

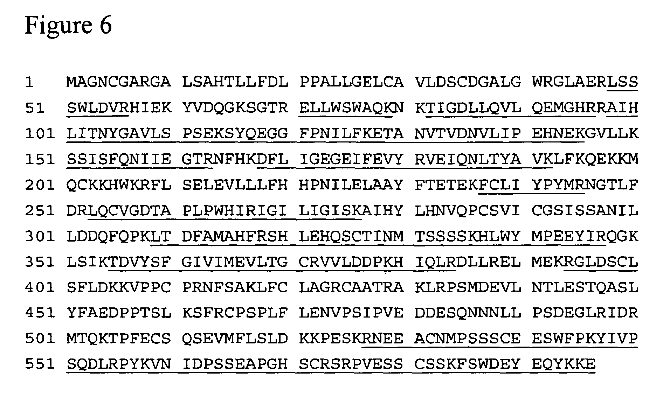

**Figure 6****:** Amino acid sequence of human IRAK3 (IPI00026984.1). Peptides identified by mass spectrometry are underlined.

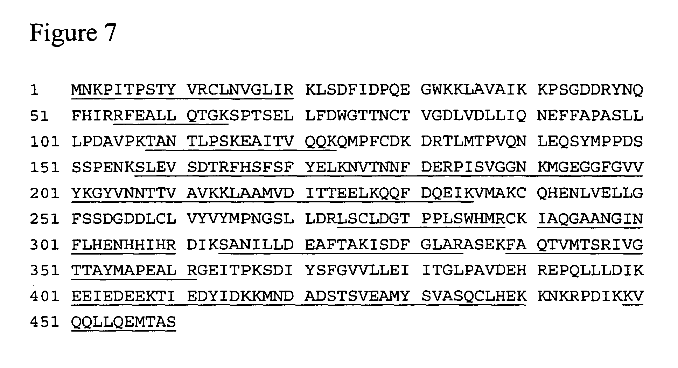

**Figure 7****:** Amino acid sequence of human IRAK4 (IPI00007641.2). Peptides identified by mass spectrometry are underlined.

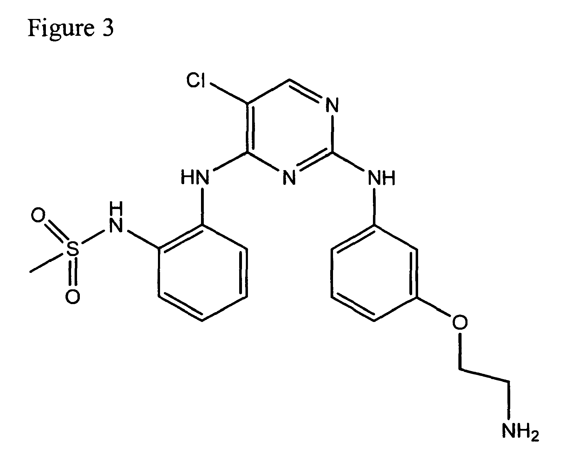

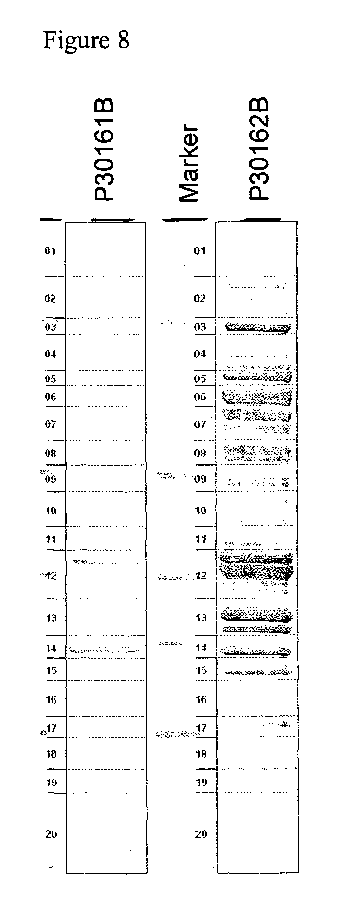

**Figure 8****:** Kinobeads experiment with the immobilized compound (Figure 3, CZC31326) for mass spectrometry analysis of captured proteins from L-363 cell lysate.
   A protein gel after staining with Coomassie brilliant blue is shown. The experiment was performed as described in example 3 with L-363 cell lysate. Proteins bound to the affinity matrix were eluted with SDS sample buffer and separated by SDS-polyacrylamide gel electrophoresis. The indicated gel areas were cut out as gel slices, proteins were treated with trypsin and ITRAQ-labeled peptides were analysed by mass spectrometry. Left lane (P30161B): cell lysate treated with 10 µM free compound CZC31326; middle lane: protein molecular weight marker; right lane (P30162B): cell lysate treated with 0.4% DMSO.

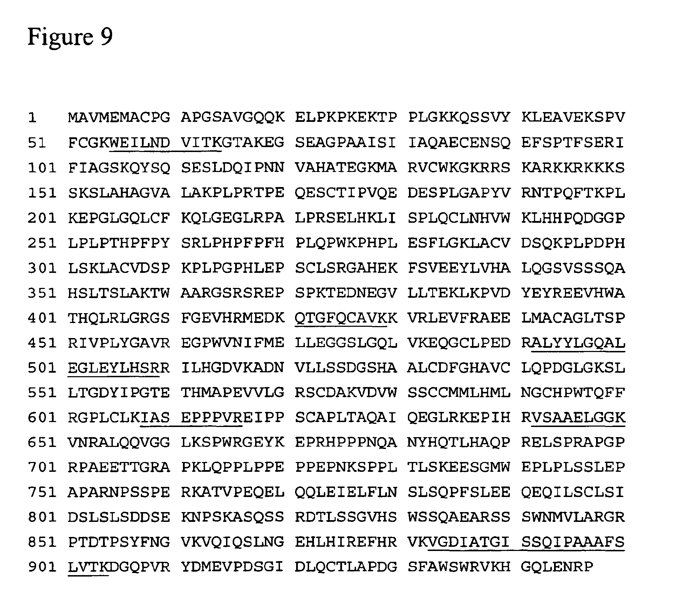

**Figure 9****:** Amino acid sequence of human NIK (IPI00016099.1). Peptides identified by mass spectrometry are underlined.

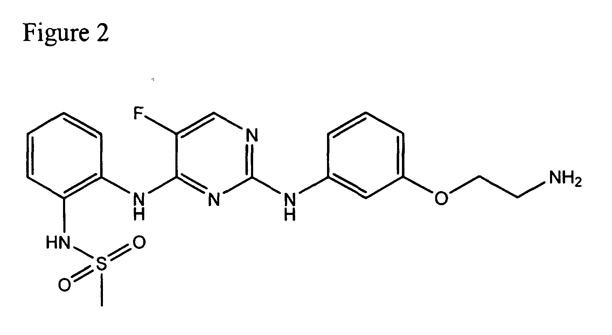

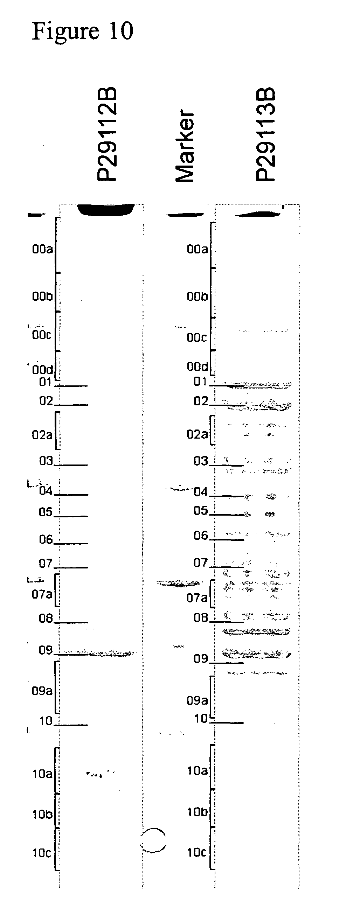

**Figure 10****:** Kinobeads experiment with the immobilized compound (Figure 2, CZC31326) and HeLa Placenta lysate mix.
   A protein gel after staining with Coomassie brilliant blue is shown. The experiment was performed as described in example 4 with a mix of HeLa and placenta cell lysates. Left lane (P29112B): cell lysate treated with 10 µM free compound CZC31326; middle lane: protein molecular weight marker; right lane (P29113B): cell lysate treated with 0.4% DMSO.

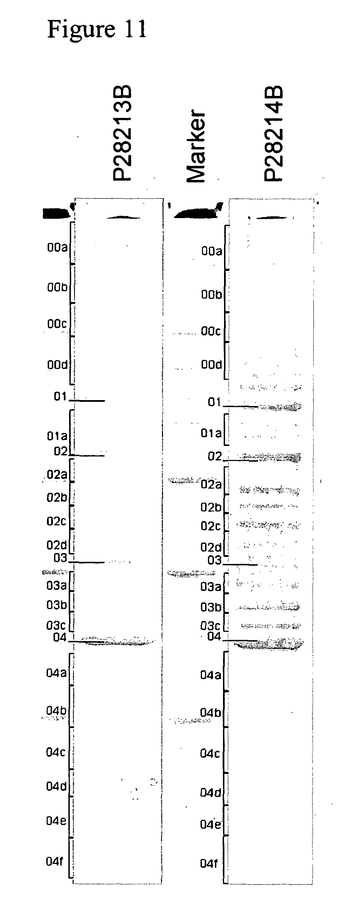

**Figure 11****:** Kinobeads experiment with the immobilized compound (Figure 2, CZC25342) and HeLa placenta lysate mix.
   A protein gel after staining with Coomassie brilliant blue is shown. The experiment was performed as described in example 5 with a mix of HeLa and placenta cell lysates. Left lane (P28213B): cell lysate treated with 10 µM free compound CZC25342; middle lane: protein molecular weight marker; right lane P28214B): cell lysate treated with 0.4% DMSO.

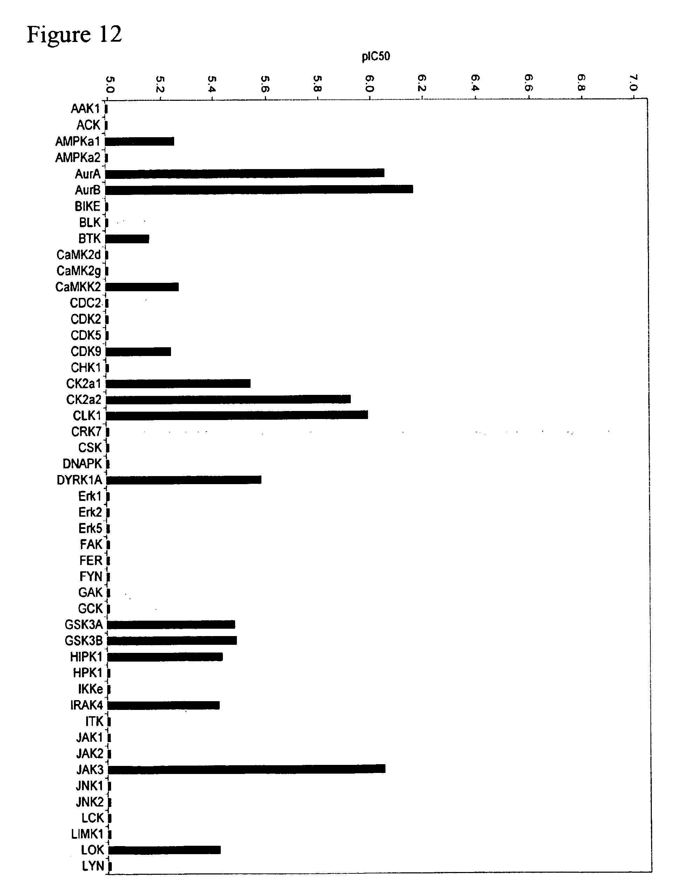

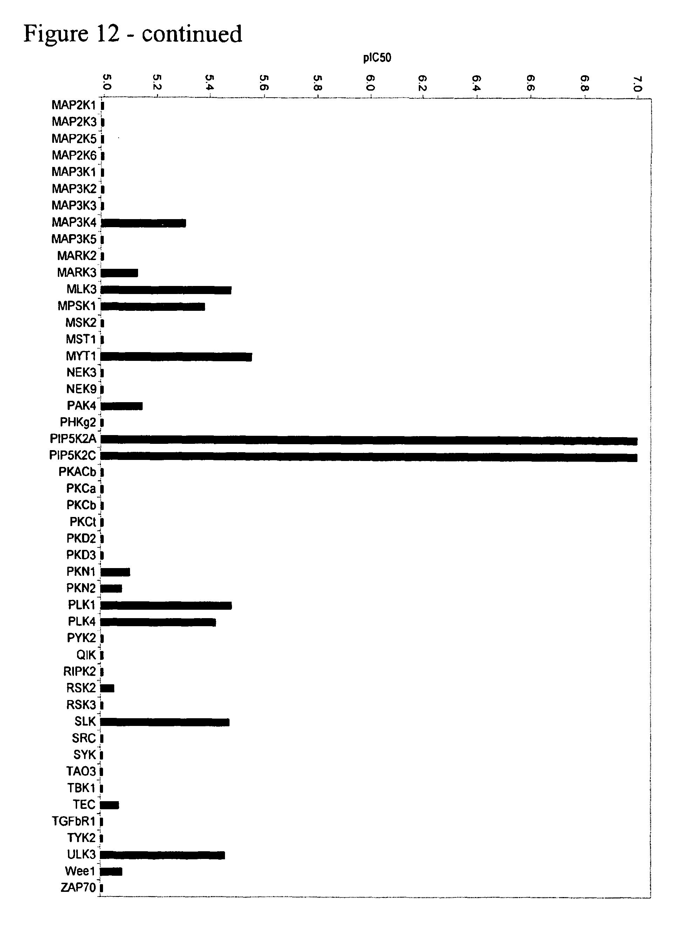

**Figure 12****:** Selectivity profiling experiment for test compound CZC00019943 using N-(2-(2-(3-(2-aminoethoxy)phenylamino)-5-chloropyrimidin-4 ylamino)phenyl)methanesulfonamide as a capture compound. The experiment was performed as described in example 6.

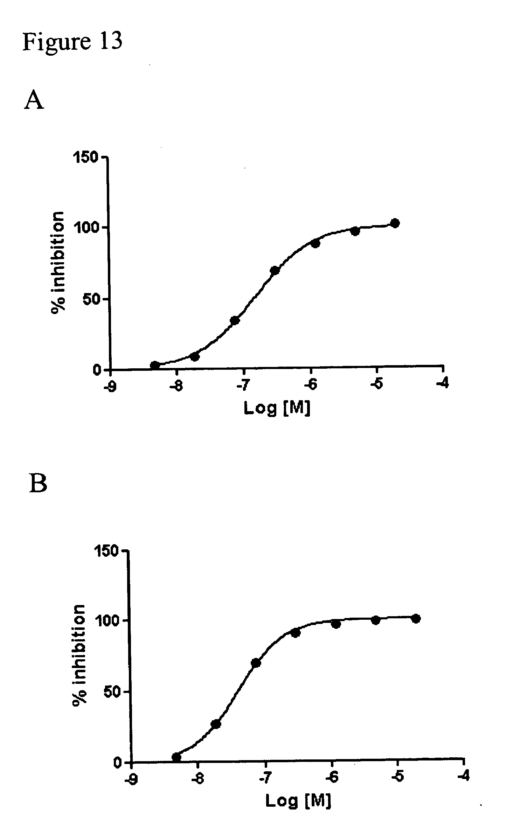

**Figure 13****:** Competition binding assay with reference compound 1 (CZC31326) in HL60 cell lysate and detection of IRAK1 and IRAK4 with specific antibodies.

The experiment was performed as described in example 7. The test compound (or DMSO as solvent control) and diluted cell lysate were added to the affinity matrix and incubated for two hours at 4°C. After washing, captured proteins were eluted with SDS-containing sample buffer. After spotting of eluate samples onto Nitrocellulose membranes IRAK1 was detected with an anti-IRAK1 antibody and IRAK4 with anti-IRAK4 antibody followed by incubation with a secondary fluorescently labeled detection antibody.
A: Dose response curve for IRAK1 and CZC31326; IC₅₀ = 0.15 µM
B: Dose response curve for IRAK4 and CZC31326; IC₅₀ = 0.04 µM

### Examples

### Example 1: Preparation of the affinity matrix

### Analytical Methods

NMR spectra were obtained on a Bruker dpx400. LCMS was carried out on an Agilent 1100 using a ZORBAX^{®} SB-C18, 4.6 x 150 mm, 5 microns or ZORBAX^{®} SB-C18, 4.6 x 75 mm, 3.5 micron column. Column flow was 1mL/min and solvents used were water and acetonitrile (0.1% formic acid) with an injection volume of 10uL. Wavelengths were 254 and 210 nm. Methods are described below.

### Method A

Column: Gemini C18, 3 x 30 mm, 3 microns Flow: 1.2 mL/min. Gradient: Table 1

| **Table 1** | | |
|---|---|---|
| Time (min) | Water | Acetonitrile |
| 0 | 95 | 5 |
| 3 | 5 | 95 |
| 4.5 | 5 | 95 |
| 4.6 | 95 | 5 |
| 5.00 | STOP | |

This example describes the synthesis of compounds and methods for their immobilization on a solid support yielding the affinity matrix used in the following examples for the capturing of kinases from cell lysates.

### Synthesis of compounds

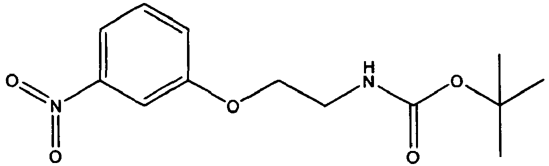

### tert-Butyl 2-(3-nitrophenoxy)ethylcarbamate

A mixture of 3-nitrophenol (272 mg), tert-butyl 2-bromoethylcarbamate (439 mg) and potassium carbonate (405 mg) was stirred overnight at 50°C in DMF (5ml). The reaction was cooled, diluted with ethyl acetate (10 ml) and washed with water (10 ml) and brine (10 ml). The organic layer was dried (MgSO4), filtered and solvents removed under reduced pressure. The residue was purified by flash column chromatography (10% ether in hexane to 60% ether in hexane) ¹H NMR (CDCL₃) δ 7.08 (t, 1H), 6.34 (m, 2H), 6.25 (m, 1H), 5.02 (br, 1H), 3.99 (m, 2H), 3.54 (m, 2H), 1.47 (s, 9H); LCMS method A, RT = 2.84 min.

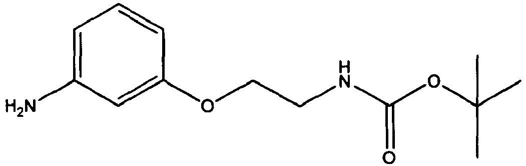

### tert-Butyl 2-(3-aminophenoxy)ethylcarbamate

tert-Butyl 2-(3-nitrophenoxy)ethylcarbamate (225mg) and palladium (10% on C, 10mg) were stirred in methanol (5ml) under an atmosphere of hydrogen for 2 hours. The reaction was filtered through Cellite and solvents removed under reduced pressure. ¹H NMR (CDCl₃) δ 6.76 (m, 4H), 4.90 (brs, 1H), 3.99 (m, 2H), 3.50 (m, 2H), 1.38 (s, 9H); LCMS method A, RT = 1.84 min M+H+ = 253

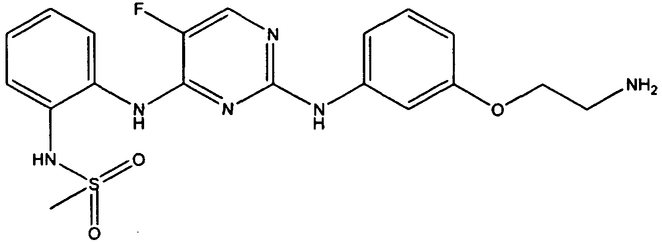

### N-(2-(2-(3-(2-aminoethoxy)phenylamino)-5-fluoropyrimidin-4-ylamino)phenyl)methanesulfonamide

N-(2-(2-chloro-5-fluoropyrimidin-4-ylamino)phenyl)methanesulfonamide (73mg) and tert-Butyl 2-(3-aminophenoxy)ethylcarbamate (58 mgs) in n-butanol (1ml) were heated at 120oC in a microwave for 3 hrs. Solvents were removed under reduced pressure and the residue was redissolved in methanol (1ml) and 4M HCl in dioxan (1ml). The reaction was allowed to stand at room temperature for 1hr. Solvents were removed under reduced pressure and the residue was purified by prep hplc. ¹H NMR (d6-DMSO) δ 9.22 (s, 1H), 8.84 (brs, 1H), 8.23 (s, 2H), 8.13 (d, 1H), 7.97 (m, 1H), 7.38 (m, 2H), 7.22 (dd, 1H), 7.17 (m, 1H), 7.08 (t, 1H), 6.50 (m, 1H) 3.99 (t, 2H), 3.15 (t, 2H), 2.87 (s, 3H); LCMS method A, RT = 1.54 min M+H+ = 532

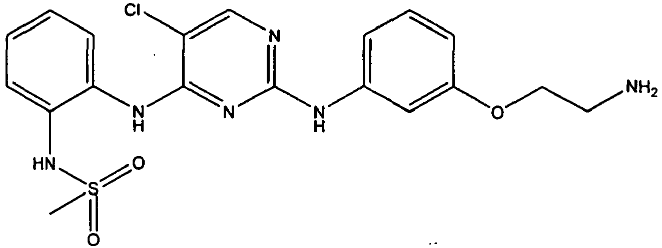

### N (2-(2-(3-(2-aminoethoxy)phenylamino)-5-chloropyrimidin-4-ylamino)phenyl)methanesulfonamide

N-(2-(2,5-dichloropyrimidin-4-ylamino)phenyl)methanesulfonamide (106mg, 1eq, 0.32mmol) and tert-butyl 2-(3-aminophenoxy)ethylcarbamate (80mg, 0.32mmol, 1eq) were combined with isopropyl alcohol (2.0mL) and hydrochloric acid SG1.18 (0.008mL, 0.25mmol, 0.8eq). The reaction mixture was heated and stirred to 120'C for 1hour by microwave. After this time the solvent was taken off under vacuum to give a solid which was taken up in a solution of hydrochloric acid in ethanol (2.0M, 3.0mL). The mixture was stirred at room temperature for 18 hours. The mixture was concentrated to yield a beige solid which was purified by prep chromatography. ¹H NMR: (d6-DMSO) δ = 9.37 (s, 1H); 9.13 (br s, 1H); 8.25 (d, 1H); 8.15 (s, 1H); 7.39 (s, 1H); 7.24-7.28 (t m, 2H); 7.13 (t, 1H); 6.98 (t m, 1H); 6.88 (t, 1H); 6.54 (dd, 1H); 3.97 (t, 1H); 3.17 (s, 2H); 3.08 (t, 1H); 2.77 (s, 3H) LCMS method A (MH+) = 449/451, RT = 1.66 min

**Table 2: Abbreviations used**

| | |
|---|---|
| br | broad |
| CDC13 | deuterochloroform |
| d | doublet |
| dd | doublet of doublets |
| DMSO | dimethyl sulphoxide |
| MH4OH | Ammonium hydroxyde |
| g | gram |
| HCl | Hydrochloric acid |
| HOBT | N-Hydroxybenzotriazole |
| m | multiplet |
| mg | milligram |
| ml | millilitre |
| mmol | millimole |
| M | molar |
| MHz | megahertz |
| DMF | Dimethylformamide |
| Hz | Hertz |
| equiv | Equivalent |
| DCM | Dichloromethane |
| THF | Tetrahydrofuran |
| NMR | nuclear magnetic resonance |
| q | quartet |
| s | singlet |
| t | triplet |

### Immobilization of compounds on beads (affinity matrix)

NHS-activated Sepharose 4 Fast Flow (Amersham Biosciences, 17-0906-01) was equilibrated with anhydrous DMSO (Dimethylsulfoxid, Fluka, 41648, H20 <= 0.005%). 1 ml of settled beads was placed in a 15 ml Falcon tube, compound stock solution (usually 100 mM in DMF or DMSO) was added (final concentration 0.2-2 µmol/ml beads) as well as 15 µl of triethylamine (Sigma, T-0886, 99% pure). Beads were incubated at room temperature in darkness on an end-over-end shaker (Roto Shake Genie, Scientific Industries Inc.) for 16 - 20 hours. Coupling efficiency is determined by HPLC. Non-reacted NHS-groups were blocked by incubation with aminoethanol at room temperature on the end-over-end shaker over night. Beads were washed with 10 ml of DMSO and were stored in isopropanol at -20°C. These beads were used as the affinity matrix in the following examples. Control beads (no compound immobilized) were generated by blocking the NHS-groups by incubation with aminoethanol as described above.

### Example 2: Kinobeads experiment using immobilized compound and HL60 cell lysate

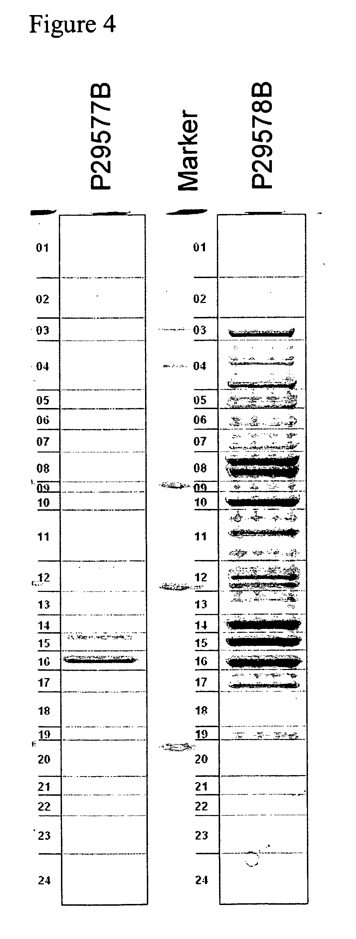

This example demonstrates the use of an immobilized compound (structure shown in Figure 3, CZC31326) for the capturing and identification of kinases from cell lysate in a competition binding assay. To one aliquot of cell lysate 10 µM of the free compound (Figure 3, CZC31326) was added and allowed to bind to proteins in the lysate. Then the affinity matrix with the immobilized compound (Example 1) was added to capture proteins that were not interacting with the previously added free compound. Beads were separated from the lysate and bead bound proteins were eluted in SDS sample buffer and subsequently separated by SDS-Polyacrylamide gel electrophoresis (Figure 4). Suitable gel bands were cut out and subjected to in-gel proteolytic digestion with trypsin. The second lysate aliquot was processed identically, however no free compound was added (DMSO solvent control). Peptides originating from samples 1 and 2 were labeled with iTRAQ reagents (iTRAQ 114 and iTRAQ 117) and the combined samples were analyzed with a nano-flow liquid chromatography system coupled online to a tandem mass spectrometer (LC-MS/MS) experiment followed by iTRAQ reporter ion quantification in the MS/MS spectra (Ross et al., 2004. Mol. Cell. Proteomics 3(12):1154-1169). Further experimental protocols can be found in WO2006/134056 and a previous publication (Bantscheff et al., 2007. Nature Biotechnology 25, 1035-1044).

The identified kinases are shown in Table 4 including the percent competition values for the sample to which 10 µM free compound had been added. In total 98 different kinases were identified and competed by different degrees. For illustration, the identified peptides for IRAK1, IRAK3 and IRAK4 are shown in Figures 5, 6 and 7.

### 1. Cell culture

HL60 cells (DSMZ #ACC3) were either obtained from an external supplier (CIL SA, Mons, Belgium) or grown in one litre Spinner flasks (Integra Biosciences, #182101) in suspension in RPMI 1640 medium (Invitrogen, #21875-034) supplemented with 10% Fetal Bovine Serum (Invitrogen, #10270-106). Cells were harvested by centrifugation, washed once with 1 x PBS buffer (Invitrogen, #14190-094) and cell pellets were frozen in liquid nitrogen and subsequently stored at -80°C.

### 2. Preparation of cell lysates

Cells were homogenized in a Potter S homogenizer in lysis buffer: 50 mM Tris-HCl, 0.8% NP40, 5% glycerol, 150 mM NaCl, 1.5 mM MgCl₂, 25 mM NaF, 1 mM sodium vanadate, 1 mM DTT, pH 7.5. One complete EDTA-free tablet (protease inhibitor cocktail, Roche Diagnostics, 1 873 580) per 25 ml buffer was added. The material was dounced 20 times using a mechanized POTTER S, transferred to 50 ml falcon tubes, incubated for 30 minutes rotating at 4° C and spun down for 10 minutes at 20,000 x g at 4°C (10,000 rpm in Sorvall SLA600, precooled). The supernatant was transferred to an ultracentrifuge (UZ)-polycarbonate tube (Beckmann, 355654) and spun for 1 hour at 145.000 x g at 4°C (40.000 rpm in Ti50.2, precooled). The supernatant was transferred again to a fresh 50 ml falcon tube, the protein concentration was determined by a Bradford assay (BioRad) and samples containing 50 mg of protein per aliquot were prepared. The samples were immediately used for experiments or frozen in liquid nitrogen and stored frozen at -80°C.

### 3. Capturing of kinases from cell lysate

Sepharose-beads with the immobilized compound (100 µl beads per pull-down experiment) were equilibrated in lysis buffer and incubated with a cell lysate sample containing 50 mg of protein on an end-over-end shaker (Roto Shake Genie, Scientific Industries Inc.) for 2 hours at 4°C. Beads were collected, transferred to Mobicol-columns (MoBiTech 10055) and washed with 10 ml lysis buffer containing 0.4% NP40 detergent, followed by 5 ml lysis buffer containing 0.2 % detergent. To elute bound proteins, 60 µl 2x SDS sample buffer was added to the column. The column was incubated for 30 minutes at 50°C and the eluate was transferred to a siliconized microfuge tube by centrifugation. Proteins were then alkylated with 108 mM iodoacetamid. Proteins were then separated by SDS-Polyacrylamide electrophoresis (SDS-PAGE).

### 4. Protein Identification by Mass Spectrometry

### 4.1 Protein digestion prior to mass spectrometric analysis

Gel-separated proteins were digested in-gel essentially following a previously described procedure (Shevchenko et al., 1996, Anal. Chem. 68:850-858). Briefly, gel-separated proteins were excised from the gel using a clean scalpel, destained twice using 100 µl 5mM triethylammonium bicarbonate buffer (TEAB; Sigma T7408) and 40% ethanol in water and dehydrated with absolute ethanol. Proteins were subsequently digested in-gel with porcine trypsin (Promega) at a protease concentration of 10 ng/µl in 5mM TEAB. Digestion was allowed to proceed for 4 hours at 37°C and the reaction was subsequently stopped using 5 µl 5% formic acid.

### 4.2 Sample preparation prior to analysis by mass spectrometry

Gel plugs were extracted twice with 20 µl 1% formic acid and three times with increasing concentrations of acetonitrile. Extracts were subsequently pooled with acidified digest supernatants and dried in a vacuum centrifuge.

### 4.3 iTRAQ labeling of peptide extracts

The peptide extracts of samples treated with 10 µM of free compound (CZC31326) and the solvent control (0.5% DMSO) were treated with different variants of the isobaric tagging reagent (iTRAQ Reagents Multiplex Kit, part number 4352135, Applied Biosystems, Foster City, CA, USA). The iTRAQ reagents are a set of multiplexed, amine-specific, stable isotope reagents that can label peptides on amino groups in up to four different biological samples enabling simultaneous identification and quantitation of peptides. The iTRAQ reagents were used according to instructions provided by the manufacturer. The samples were resuspended in 10 µl 50 mM TEAB solution, pH 8.5 and 10 µl ethanol were added. The iTRAQ reagent was dissolved in 120 µl ethanol and 10 µl of reagent solution were added to the sample. The labeling reaction was performed at room temperature for one hour on a horizontal shaker and stopped by adding 5 µl of 100 mM TEAB and 100 mM glycine in water. The two labeled sampled were then combined, dried in a vacuum centrifuge and resuspended in 10 µl of 0.1 % formic acid in water.

### 4.4 Mass spectrometric data acquisition

Peptide samples were injected into a nano LC system (CapLC, Waters or nano-LC 1D+, Eksigent) which was directly coupled either to a quadrupole TOF (QTOF Ultima, QTOF Micro, Waters), ion trap (LTQ) or Orbitrap mass spectrometer. Peptides were separated on the LC system using a gradient of aqueous and organic solvents (see below). Solvent A was 0.1 % formic acid and solvent B was 70% acetonitrile in 0.1 % formic acid.

**Table 3: Peptides elution off the LC system**

| Time (min) | % solvent A | % solvent B |
|---|---|---|
| 0 | 95 | 5 |
| 8.0 | 95 | 5 |
| 15 | 85 | 15 |
| 64.5 | 60 | 40 |
| 84.5 | 38 | 62 |
| 87 | 5 | 95 |
| 91 | 250 | 95 |
| 91.5 | 2095 | 5 |

### 4.5 Protein identification

The peptide mass and fragmentation data generated in the LC-MS/MS experiments were used to query a protein data base consisting of an in-house curated version of the International Protein Index (IPI) protein sequence database combined with a decoy version of this database (Elias and Gygi, 2007, Target-decoy search strategy for increased confidence in large-scale protein identifications by mass spectrometry. Nature Methods 4, 207-214). Proteins were identified by correlating the measured peptide mass and fragmentation data with data computed from the entries in the database using the software tool Mascot (Matrix Science; Perkins et al., 1999. Probability-based protein identification by searching sequence databases using mass spectrometry data. Electrophoresis 20, 3551-3567). Search criteria varied depending on which mass spectrometer was used for the analysis. Protein acceptance thresholds were adjusted to achieve a false discovery rate of below 1% as suggested by hit rates on the decoy data base (Elias and Gygi, 2007, Target-decoy search strategy for increased confidence in large-scale protein identifications by mass spectrometry. Nature Methods 4, 207-214).

### 4.6 Protein quantitation

Relative protein quantitation was performed using peak areas of iTRAQ reporter ion signals essentially as described in an earlier publication (Bantscheff et al., 2007. Nature Biotechnology 25, 1035-1044).

**Table 4: Identified kinases with CZC31326 from HL60 lysate**

| **Representative Sequence** | **Kinase Name** | **Kinase Group** | **Quantified Spectra** | **Redundant Peptides** | **Competition %** |
|---|---|---|---|---|---|
| IPI00479760.6 | AAK1 | Other | 200 | 839 | 97.8 |
| IPI00552750.2 | ACK | TK | 12 | 14 | 93.6 |
| IPI00005732.1 | ALK4 | TKL | 4 | 7 | 94.5 |
| IPI00410287.3 | AMPKa1 | CAMK | 146 | 230 | 97.7 |
| IPI00298940.3 | AurA | Other | 142 | 173 | 96.6 |
| IPI00796914.1 | AurB | Other | 21 | 27 | 95 |
| IPI00337426.1 | BIKE | Other | 110 | 338 | 97.6 |
| IPI00029132.3 | BTK | TK | 167 | 199 | 97.1 |
| IPI00828081.1 | CaMK2d | CAMK | 15 | 38 | 90.4 |
| IPI00169392.5 | CaMK2g | CAMK | 117 | 413 | 90.4 |
| IPI00290239.2 | CaMKK2 | Other | 72 | 410 | 98.1 |
| IPI00026689.4 | CDC2 | CMGC | 60 | 86 | 93.1 |
| IPI00014873.2 | CDK10 | CMGC | 9 | 33 | 96.7 |
| IPI00031681.1 | CDK2 | CMGC | 76 | 114 | 95.1 |
| IPI00023530.6 | CDK5 | CMGC | 143 | 214 | 95.2 |
| IPI00552413.2 | CDK9 | CMGC | 51 | 144 | 98.2 |
| IPI00023664.3 | CHK1 | CAMK | 14 | 18 | 95.7 |
| IPI00016613.2 | CK2a1 | Other | 141 | 408 | 97.9 |
| IPI00020602.1 | CK2a2 | Other | 79 | 138 | 97.7 |
| IPI00848015.1 | CLK1 | CMGC | 6 | 18 | 97.4 |
| IPI00021175.1 | CRK7 | CMGC | 37 | 49 | 92.1 |
| IPI00013212.1 | CSK | TK | 8 | 18 | 80.3 |
| IPI00296337.2 | DNAPK | Atypical | 42 | 200 | 75.5 |
| IPI00014344.1 | DYRK1A | CMGC | 45 | 168 | 96.1 |
| IPI00005222.2 | EphB6 | TK | 18 | 18 | 93.6 |
| IPI00018195.3 | Erk1 | CMGC | 12 | 24 | 84.8 |
| IPI00003479.3 | Erk2 | CMGC | 197 | 260 | 75.8 |
| IPI00029263.2 | FER | TK | 151 | 378 | 97.7 |
| IPI00294344.2 | FES | TK | 218 | 390 | 96.3 |
| IPI00787531.1 | GAK | Other | 169 | 209 | 97.9 |
| IPI00149094.4 | GCK | STE | 21 | 52 | 96.5 |
| IPI00292228.1 | GSK3A | CMGC | 101 | 204 | 96.2 |
| IPI00216190.1 | GSK3B | CMGC | 80 | 185 | 96.1 |
| IPI00747261.1 | HIPK1 | CMGC | 10 | 65 | 97.1 |
| IPI00289892.1 | HIPK2 | CMGC | 4 | 24 | 94.4 |
| IPI00020258.3 | HPK1 | STE | 88 | 127 | 97.7 |
| IPI00029045.1 | IKKe | Other | 17 | 25 | 91.4 |
| IPI00293652.1 | IRAK1 | TKL | 25 | 131 | 94.9 |
| IPI00026984.1 | IRAK3 | TKL | 109 | 131 | 97.2 |
| IPI00007641.2 | IRAK4 | TKL | 79 | 85 | 97.5 |
| IPI00015974.1 | IRE1 | Other | 10 | 10 | 95.1 |
| IPI00011633.3 | JAK1 | TK | 159 | 208 | 97.8 |
| IPI00031016.1 | JAK2 | TK | 53 | 60 | 96.8 |
| IPI00024672.1 | JNK1 | CMGC | 80 | 335 | 88.8 |
| IPI00303550.2 | JNK2 | CMGC | 85 | 522 | 87.8 |
| IPI00294842.3 | KHS1 | STE | 6 | 7 | 93.2 |
| IPI00515097.3 | LCK | TK | 4 | 12 | 86.8 |
| IPI00304742.4 | LOK | STE | 9 | 26 | 95.3 |
| IPI00794835.1 | LRRK2 | TKL | 10 | 56 | 71.7 |
| IPI00219604.3 | MAP2K1 | STE | 6 | 9 | 85.2 |
| IPI00218858.1 | MAP2K3 | STE | 20 | 53 | 90.9 |
| IPI00185860.4 | MAP2K5 | STE | 32 | 38 | 93.1 |
| IPI00787127.1 | MAP3K1 | STE | 52 | 183 | 87 |
| IPI00513803.3 | MAP3K2 | STE | 34 | 50 | 96.3 |
| IPI00181703.1 | MAP3K3 | STE | 14 | 58 | 97.6 |
| IPI00386260.3 | MAP3K4 | STE | 63 | 222 | 97.2 |
| IPI00412433.1 | MAP3K5 | STE | 92 | 160 | 88.4 |
| IPI00555838.1 | MARK2 | CAMK | 64 | 513 | 97.1 |
| IPI00183118.4 | MARK3 | CAMK | 32 | 242 | 97.2 |
| IPI00000977.1 | MLK3 | TKL | 44 | 51 | 96.5 |
| IPI00142487.2 | MLK4 | TKL | 25 | 50 | 95 |
| IPI00306833.3 | MPSK1 | Other | 9 | 10 | 93.4 |
| IPI00335101.1 | MSK1 | AGC | 11 | 15 | 94.7 |
| IPI00022536.1 | MSK2 | AGC | 30 | 72 | 96.8 |
| IPI00011488.4 | MST1 | STE | 11 | 13 | 97.7 |
| IPI00292827.3 | MST4 | STE | 6 | 12 | 96.1 |
| IPI00384765.2 | MYT1 | Other | 25 | 55 | 90.5 |
| IPI00465101.5 | NEK3 | Other | 37 | 96 | 97 |
| IPI00301609.8 | NEK9 | Other | 133 | 302 | 93 |
| IPI00014068.1 | PAK4 | STE | 88 | 168 | 97.5 |
| IPI00012891.1 | PHKg2 | CAMK | 26 | 31 | 96.6 |
| IPI00009688.1 | PIP5K2A | Lipid Kinase | 17 | 26 | 97.6 |
| IPI00152303.7 | PIP5K2C | Lipid Kinase | 35 | 62 | 98.3 |
| IPI00843864.1 | PITSLRE | CMGC | 8 | 140 | 94.8 |
| IPI00385449.4 | PKCa | AGC | 6 | 16 | 96.2 |
| IPI00219628.3 | PKCb | AGC | 15 | 50 | 97.3 |
| IPI00009334.4 | PKD2 | CAMK | 87 | 138 | 97.9 |
| IPI00015538.1 | PKD3 | CAMK | 23 | 53 | 96.9 |
| IPI00412672.1 | PKN1 | AGC | 35 | 47 | 97 |
| IPI00844111.1 | PKN2 | AGC | 5 | 28 | 93.9 |
| IPI00021248.1 | PLK1 | Other | 25 | 31 | 97.8 |
| IPI00410344.2 | PLK4 | Other | 6 | 9 | 91.7 |
| IPI00029702.1 | PYK2 | TK | 199 | 394 | 95.7 |
| IPI00465291.3 | QIK | CAMK | 6 | 8 | 94.2 |
| IPI00013983.1 | RET | TK | 71 | 96 | 97.1 |
| IPI00020898.1 | RSK2 | AGC | 80 | 171 | 96.7 |
| IPI00477982.1 | RSK3 | AGC | 160 | 268 | 96.9 |
| IPI00022827.1 | SLK | STE | 72 | 184 | 97.5 |
| IPI00302351.2 | STK33 | CAMK | 34 | 48 | 96.9 |
| IPI00018597.1 | SYK | TK | 302 | 372 | 95.6 |
| IPI00410485.2 | TAO3 | STE | 53 | 68 | 98 |
| IPI00293613.2 | TBK1 | Other | 192 | 243 | 94.8 |
| IPI00000878.3 | TEC | TK | 34 | 80 | 97 |
| IPI00005733.1 | TGFbR1 | TKL | 8 | 22 | 95.6 |
| IPI00022353.4 | TYK2 | TK | 53 | 61 | 97.1 |
| IPI00411818.4 | ULK3 | Other | 40 | 47 | 97.7 |
| IPI00025830.1 | Wee1 | Other | 75 | 85 | 98.2 |
| IPI00013981.4 | YES | TK | 18 | 20 | 91.1 |

### Example 3: Kinobeads experiment using immobilized compound and L-363 cell lysate

This example demonstrates the use of an immobilized compound (structure shown in Figure 3, CZC31326) for the capturing and identification of kinases from L-363 cell lysate in a competition binding assay. To one aliquot of cell lysate 10 µM of the free compound (Figure 3, CZC31326) was added and allowed to bind to proteins in the lysate. Then the affinity matrix with the immobilized compound was added to capture proteins that were not interacting with the previously added free compound. Beads were separated from the lysate and bead bound proteins were eluted in SDS sample buffer and subsequently separated by SDS-Polyacrylamide gel electrophoresis (Figure 8). Suitable gel bands were cut out and subjected to in-gel proteolytic digestion with trypsin. The second lysate aliquot was processed identically, however no free compound was added (DMSO solvent control). Peptides originating from samples 1 and 2 were labeled with iTRAQ reagents (iTRAQ 114 and iTRAQ 117) and the combined samples were analyzed with a nano-flow liquid chromatography system coupled online to a tandem mass spectrometer (LC-MS/MS) experiment followed by iTRAQ reporter ion quantification in the MS/MS spectra as described in Example 2.

For this experiment the human plasma cell leukaemia line L-363 was used (German Collection of Microorganisms and Cell Cultures, DSMZ number ACC49, Braunschweig, Germany). Cells were treated for three hours with 20 µM of the proteasome inhibitor MG132 (Z-Leu-Leu-Leu-al, Sigma C2211) prior to cell harvesting.

The identified kinases are shown in Table 5 including the percent competition values for the sample to which 10 µM free compound had been added. In total of 93 different kinases were identified and competed by different degrees. For illustration, the identified peptides for NIK are shown in Figure 9.

**Table 5: Identified kinases with CZC31326 from L-363 cell lysate**

| **Representative Sequence** | **Kinase Name** | **Kinase Group** | **Quantified Spectra** | **Redundant Peptides** | **Competition %** |
|---|---|---|---|---|---|
| IPI00479760.6 | AAK1 | Other | 144 | 435 | 97.4 |
| IPI00787836.1 | ADCK1 | Atypical | 10 | 25 | 93.8 |
| IPI00029219.1 | ALK2 | TKL | 10 | 11 | 95.7 |
| IPI00410287.3 | AMPKa1 | CAMK | 93 | 172 | 97.7 |
| IPI00329488.4 | ARG | TK | 7 | 48 | 95.8 |
| IPI00298940.3 | AurA | Other | 39 | 47 | 97 |
| IPI00004497.2 | BCR | Atypical | 10 | 13 | 93.6 |
| IPI00337426.1 | BIKE | Other | 54 | 162 | 97 |
| IPI00029132.3 | BTK | TK | 32 | 35 | 95.7 |
| IPI00828081.1 | CaMK2d | CAMK | 98 | 775 | 78 |
| IPI00169392.5 | CaMK2q | CAMK | 77 | 386 | 75.9 |
| IPI00290239.2 | CaMKK2 | Other | 38 | 218 | 97 |
| IPI00026689.4 | CDC2 | CMGC | 44 | 54 | 95.1 |
| IPI00031681.1 | CDK2 | CMGC | 58 | 76 | 95.2 |
| IPI00023530.6 | CDK5 | CMGC | 128 | 190 | 96.4 |
| IPI00552413.2 | CDK9 | CMGC | 16 | 44 | 97.7 |
| IPI00023664.3 | CHK1 | CAMK | 6 | 8 | 94.8 |
| IPI00016613.2 | CK2a1 | Other | 61 | 199 | 97.9 |
| IPI00020602.1 | CK2a2 | Other | 64 | 87 | 97.5 |
| IPI00296337.2 | DNAPK | Atypical | 137 | 296 | 84 |
| IPI00014934.1 | DRAK2 | CAMK | 4 | 5 | 93.3 |
| IPI00014344.1 | DYRK1A | CMGC | 14 | 90 | 96.5 |
| IPI00018195.3 | Erk1 | CMGC | 8 | 37 | 83.2 |
| IPI00003479.3 | Erk2 | CMGC | 52 | 90 | 74.4 |
| IPI00029263.2 | FER | TK | 125 | 290 | 97.8 |
| IPI00787531.1 | GAK | Other | 109 | 143 | 98.2 |
| IPI00149094.4 | GCK | STE | 4 | 8 | 95.1 |
| IPI00292228.1 | GSK3A | CMGC | 23 | 46 | 96.9 |
| IPI00216190.1 | GSK3B | CMGC | 35 | 112 | 94.6 |
| IPI00289892.1 | HIPK2 | CMGC | 12 | 45 | 95.6 |
| IPI00020258.3 | HPK1 | STE | 75 | 100 | 98 |
| IPI00328149.3 | HRI | Other | 5 | 12 | 95.8 |
| IPI00029045.1 | IKKe | Other | 44 | 54 | 89.4 |
| IPI00025803.3 | INSR | TK | 6 | 36 | 90.1 |
| IPI00293652.1 | IRAK1 | TKL | 28 | 107 | 95.1 |
| IPI00007641.2 | IRAK4 | TKL | 32 | 39 | 97.4 |
| IPI00015974.1 | IRE1 | Other | 25 | 30 | 97.9 |
| IPI00011633.3 | JAK1 | TK | 340 | 400 | 97.2 |
| IPI00031016.1 | JAK2 | TK | 44 | 51 | 96.1 |
| IPI00219418.2 | JAK3 | TK | 84 | 109 | 98.1 |
| IPI00024672.1 | JNK1 | CMGC | 31 | 85 | 87 |
| IPI00303550.2 | JNK2 | CMGC | 29 | 174 | 88 |
| IPI00304742.4 | LOK | STE | 4 | 8 | 86.9 |
| IPI00298625.2 | LYN | TK | 26 | 35 | 94.5 |
| IPI00218858.1 | MAP2K3 | STE | 5 | 15 | 93.9 |
| IPI00185860.4 | MAP2K5 | STE | 12 | 13 | 97.5 |
| IPI00003814.1 | MAP2K6 | STE | 32 | 36 | 94.2 |
| IPI00787127.1 | MAP3K1 | STE | 50 | 60 | 96.2 |
| IPI00513803.3 | MAP3K2 | STE | 30 | 50 | 96 |
| IPI00181703.1 | MAP3K3 | STE | 5 | 30 | 97.4 |
| IPI00386260.3 | MAP3K4 | STE | 12 | 39 | 95.8 |
| IPI00412433.1 | MAP3K5 | STE | 9 | 40 | 91.3 |
| IPI00555838.1 | MARK2 | CAMK | 32 | 392 | 97 |
| IPI00183118.4 | MARK3 | CAMK | 19 | 108 | 97 |
| IPI00000977.1 | MLK3 | TKL | 8 | 8 | 97.5 |
| IPI00306833.3 | MPSK1 | Other | 7 | 8 | 93.3 |
| IPI00022536.1 | MSK2 | AGC | 18 | 42 | 94.2 |
| IPI00011488.4 | MST1 | STE | 73 | 125 | 98.3 |
| IPI00411984.4 | MST2 | STE | 35 | 68 | 98.2 |
| IPI00384765.2 | MYT1 | Other | 20 | 46 | 93.1 |
| IPI00747017.1 | NEK1 | Other | 30 | 99 | 95.7 |
| IPI00465101.5 | NEK3 | Other | 31 | 41 | 96.8 |
| IPI00396662.2 | NEK6 | Other | 8 | 29 | 83.7 |
| IPI00152658.1 | NEK7 | Other | 8 | 16 | 93.9 |
| IPI00301609.8 | NEK9 | Other | 139 | 312 | 92.4 |
| IPI00016099.1 | NIK | STE | 17 | 20 | 98 |
| IPI00008883.3 | NuaK2 | CAMK | 6 | 9 | 96.7 |
| IPI00014068.1 | PAK4 | STE | 60 | 89 | 97.3 |
| IPI00012891.1 | PHKg2 | CAMK | 9 | 10 | 94.1 |
| IPI00070943.3 | PIK4Ca | Lipid Kinase | 5 | 6 | 86 |
| IPI00009688.1 | PIP5K2A | Lipid Kinase | 4 | 5 | 96.4 |
| IPI00152303.7 | PIP5K2C | Lipid Kinase | 11 | 13 | 92 |
| IPI00009334.4 | PKD2 | CAMK | 49 | 101 | 98 |
| IPI00015538.1 | PKD3 | CAMK | 80 | 131 | 98.1 |
| IPI00412672.1 | PKN1 | AGC | 12 | 14 | 95.9 |
| IPI00021248.1 | PLK1 | Other | 27 | 33 | 96.8 |
| IPI00410344.2 | PLK4 | Other | 7 | 9 | 91.2 |
| IPI00029702.1 | PYK2 | TK | 315 | 866 | 96.1 |
| IPI00465291.3 | QIK | CAMK | 11 | 12 | 96.5 |
| IPI00021917.1 | RIPK2 | TKL | 24 | 24 | 95.5 |
| IPI00020898.1 | RSK2 | AGC | 40 | 86 | 97.7 |
| IPI00477982.1 | RSK3 | AGC | 86 | 165 | 96.9 |
| IPI00007123.1 | RSK4 | AGC | 24 | 48 | 96.7 |
| IP100022827.1 | SLK | STE | 17 | 50 | 95 |
| IPI00302351.2 | STK33 | CAMK | 51 | 67 | 95.7 |
| IPI00410485.2 | TAO3 | STE | 11 | 12 | 94.7 |
| IPI00293613.2 | TBK1 | Other | 151 | 190 | 95.8 |
| IPI00000878.3 | TEC | TK | 54 | 134 | 92.8 |
| IPI00022353.4 | TYK2 | TK | 111 | 127 | 96.9 |
| IPI00289357.4 | ULK1 | Other | 8 | 12 | 96.5 |
| IPI00411818.4 | ULK3 | Other | 18 | 20 | 98.5 |
| IPI00025830.1 | Wee1 | Other | 51 | 63 | 96.4 |
| IPI00013981.4 | YES | TK | 29 | 51 | 89.6 |

### Example 4: Kinobeads experiment using immobilized compound CZC31326 and mixed HeLa placenta cell lysates

This example demonstrates the use of an immobilized compound (structure shown in Figure 3, CZC31326) for the capturing and identification of kinases from mixed HeLa placenta lysates (Bantscheff et al., 2007. Nature Biotechnology 25, 1035-1044) in a competition binding assay. To one aliquot of cell lysate 10 µM of the free compound (Figure 3, CZC31326) was added and allowed to bind to proteins in the lysate. Then the affinity matrix with the immobilized compound was added to capture proteins that were not interacting with the previously added free compound. Beads were separated from the lysate and bead bound proteins were eluted in SDS sample buffer and subsequently separated by SDS-Polyacrylamide gel electrophoresis (Figure 10). Suitable gel bands were cut out and subjected to in-gel proteolytic digestion with trypsin. The second lysate aliquot was processed identically, however no free compound was added (DMSO solvent control). Peptides originating from samples 1 and 2 were labeled with iTRAQ reagents (iTRAQ 114 and iTRAQ 117) and the combined samples were analyzed with a nano-flow liquid chromatography system coupled online to a tandem mass spectrometer (LC-MS/MS) experiment followed by iTRAQ reporter ion quantification in the MS/MS spectra as described in Example 2.

The identified kinases are shown in Table 6 including the percent competition values for the sample to which 10 µM free compound had been added. In total of 88 different kinases were identified and competed by different degrees.

**Table 6: Identified kinases from HeLa Placenta lysate mix with CZC31326**

| **Representative Sequence** | **Kinase Name** | **Kinase Group** | **Quantified Spectra** | **Redundant Peptides** | **Competition %** |
|---|---|---|---|---|---|
| IPI00479760.6 | AAK1 | Other | 323 | 868 | 94.5 |
| IPI00029219.1 | ALK2 | TKL | 7 | 7 | 94.3 |
| IPI00410287.3 | AMPKa1 | CAMK | 192 | 379 | 94.9 |
| IPI00307755.3 | AMPKa2 | CAMK | 26 | 64 | 95.2 |
| IPI00329488.4 | ARG | TK | 9 | 39 | 91.2 |
| IPI00298940.3 | AurA | Other | 113 | 153 | 94 |
| IPI00796914.1 | AurB | Other | 10 | 18 | 90.8 |
| IPI00004497.2 | BCR | Atypical | 4 | 8 | 90.2 |
| IPI00337426.1 | BIKE | Other | 20 | 80 | 92.9 |
| IPI00828081.1 | CaMK2d | CAMK | 109 | 939 | 72.1 |
| IPI00169392.5 | CaMK2g | CAMK | 232 | 1027 | 84.8 |
| IPI00657696.1 | CaMKK1 | Other | 8 | 40 | 94.2 |
| IPI00290239.2 | CaMKK2 | Other | 52 | 173 | 94.3 |
| IPI00026689.4 | CDC2 | CMGC | 30 | 42 | 93.4 |
| IPI00031681.1 | CDK2 | CMGC | 72 | 117 | 93 |
| IPI00023530.6 | CDK5 | CMGC | 104 | 153 | 92.6 |
| IPI00552413.2 | CDK9 | CMGC | 23 | 68 | 95.3 |
| IPI00016613.2 | CK2a1 | Other | 70 | 182 | 94.6 |
| IPI00020602.1 | CK2a2 | Other | 54 | 72 | 95.4 |
| IPI00296337.2 | DNAPK | Atypical | 77 | 278 | 69.2 |
| IPI00018195.3 | Erk1 | CMGC | 18 | 49 | 77.8 |
| IPI00003479.3 | Erk2 | CMGC | 66 | 117 | 68.5 |
| IPI00413961.3 | FAK | TK | 271 | 796 | 93.8 |
| IPI00029263.2 | FER | TK | 90 | 224 | 95.3 |
| IPI00294344.2 | FES | TK | 52 | 71 | 95.1 |
| IPI00293565.4 | FLT4 | TK | 63 | 90 | 89.3 |
| IPI00787531.1 | GAK | Other | 144 | 177 | 93.6 |
| IPI00149094.4 | GCK | STE | 22 | 56 | 93.8 |
| IPI00292228.1 | GSK3A | CMGC | 72 | 154 | 93.7 |
| IPI00216190.1 | GSK3B | CMGC | 74 | 178 | 93.3 |
| IPI00029045.1 | IKKe | Other | 24 | 35 | 89.7 |
| IPI00026984.1 | IRAK3 | TKL | 8 | 8 | 92 |
| IPI00007641.2 | IRAK4 | TKL | 74 | 82 | 93.8 |
| IPI00015974.1 | IRE1 | Other | 6 | 8 | 91.8 |
| IPI00011633.3 | JAK1 | TK | 206 | 260 | 95 |
| IPI00024672.1 | JNK1 | CMGC | 42 | 157 | 85 |
| IPI00303550.2 | JNK2 | CMGC | 93 | 402 | 83.1 |
| IPI00021396.1 | KDR | TK | 5 | 8 | 90 |
| IPI00794835.1 | LRRK2 | TKL | 9 | 43 | 85.2 |
| IPI00298625.2 | LYN | TK | 10 | 21 | 76.1 |
| IPI00219604.3 | MAP2K1 | STE | 4 | 8 | 83.7 |
| IPI00185860.4 | MAP2K5 | STE | 29 | 98 | 90.4 |
| IPI00003814.1 | MAP2K6 | STE | 16 | 19 | 89.2 |
| IPI00513803.3 | MAP3K2 | STE | 37 | 46 | 93.9 |
| IPI00386260.3 | MAP3K4 | STE | 21 | 72 | 92.2 |
| IPI00412433.1 | MAP3K5 | STE | 28 | 114 | 86 |
| IPI00418221.3 | MAP3K6 | STE | 14 | 27 | 84.3 |
| IPI00412740.5 | MAP3K7 | STE | 9 | 15 | 82.1 |
| IPI00555838.1 | MARK2 | CAMK | 64 | 734 | 93.7 |
| IPI00183118.4 | MARK3 | CAMK | 26 | 222 | 93.1 |
| IPI00000977.1 | MLK3 | TKL | 56 | 59 | 93.8 |
| IPI00142487.2 | MLK4 | TKL | 4 | 14 | 88.9 |
| IPI00306833.3 | MPSK1 | Other | 7 | 8 | 91.5 |
| IPI00640957.2 | MRCKa | AGC | 28 | 216 | 92.1 |
| IPI00477763.3 | MRCKb | AGC | 117 | 160 | 94 |
| IPI00022536.1 | MSK2 | AGC | 33 | 80 | 95.3 |
| IPI00011488.4 | MST1 | STE | 17 | 26 | 94.6 |
| IPI00411984.4 | MST2 | STE | 8 | 16 | 93.9 |
| IPI00465101.5 | NEK3 | Other | 19 | 39 | 94.8 |
| IPI00301609.8 | NEK9 | Other | 234 | 546 | 89 |
| IPI00014068.1 | PAK4 | STE | 221 | 318 | 94.2 |
| IPI00012891.1 | PHKg2 | CAMK | 4 | 4 | 89.9 |
| IPI00009688.1 | PIP5K2A | Lipid Kinase | 6 | 10 | 95 |
| IPI00152303.7 | PIP5K2C | Lipid Kinase | 58 | 91 | 95.4 |
| IPI00385449.4 | PKCa | AGC | 10 | 16 | 94.9 |
| IPI00009334.4 | PKD2 | CAMK | 28 | 44 | 95.4 |
| IPI00015538.1 | PKD3 | CAMK | 15 | 26 | 95.7 |
| IPI00436355.1 | PKG1 | AGC | 37 | 92 | 90.4 |
| IP100412672.1 | PKN1 | AGC | 10 | 11 | 93.7 |
| IPI00844111.1 | PKN2 | AGC | 16 | 43 | 94 |
| IPI00021248.1 | PLK1 | Other | 40 | 46 | 94.7 |
| IPI00029702.1 | PYK2 | TK | 46 | 121 | 92.7 |
| IPI00021917.1 | RIPK2 | TKL | 13 | 17 | 92.2 |
| IPI00020898.1 | RSK2 | AGC | 50 | 112 | 96 |
| IPI00477982.1 | RSK3 | AGC | 106 | 210 | 94.8 |
| IPI00022827.1 | SLK | STE | 53 | 130 | 93.2 |
| IPI00328867.6 | SRC | TK | 17 | 88 | 92.2 |
| IPI00018597.1 | SYK | TK | 39 | 58 | 93.4 |
| IPI00410485.2 | TAO3 | STE | 28 | 37 | 94.4 |
| IPI00293613.2 | TBK1 | Other | 224 | 281 | 93.8 |
| IPI00000878.3 | TEC | TK | 35 | 80 | 89.2 |
| IPI00005733.1 | TGFbR1 | TKL | 5 | 16 | 90.8 |
| IPI00412829.1 | TIE2 | TK | 20 | 23 | 91.6 |
| IPI00022633.3 | TNK1 | TK | 76 | 123 | 94.3 |
| IPI00022353.4 | TYK2 | TK | 74 | 96 | 93.8 |
| IPI00411818.4 | ULK3 | Other | 52 | 74 | 94.6 |
| IPI00025830.1 | Wee1 | Other | 45 | 56 | 95.2 |
| IPI00013981.4 | YES | TK | 65 | 109 | 90.3 |

### Example 5: Kinobeads experiment using immobilized compound CZC25342 and mixed HeLa placenta cell lysates

This example demonstrates the use of an immobilized compound (Figure 2, CZC25342) for the capturing and identification of kinases from mixed HeLa placenta lysates (Bantscheff et al., 2007. Nature Biotechnology 25, 1035-1044) in a competition binding assay. To one aliquot of cell lysate 10 µM of the free compound (Figure 2, CZC25342) was added and allowed to bind to proteins in the lysate. Then the affinity matrix with the immobilized compound was added to capture proteins that were not interacting with the previously added free compound. Beads were separated from the lysate and bead bound proteins were eluted in SDS sample buffer and subsequently separated by SDS-Polyacrylamide gel electrophoresis (Figure 11). Suitable gel bands were cut out and subjected to in-gel proteolytic digestion with trypsin. The second lysate aliquot was processed identically, however no free compound was added (DMSO solvent control). Peptides originating from samples 1 and 2 were labeled with iTRAQ reagents (iTRAQ 114 and iTRAQ 117) and the combined samples were analyzed with a nano-flow liquid chromatography system coupled online to a tandem mass spectrometer (LC-MS/MS) experiment followed by iTRAQ reporter ion quantification in the MS/MS spectra as described in Example 2.

The identified kinases are shown in Table 7 including the percent competition values for the sample to which 10 µM free compound had been added. In total of 83 different kinases were identified and competed by different degrees.

**Table 7: Identified kinases from HeLa Placenta lysate mix with CZC25342**

| **Representative Sequence** | **Kinase Name** | **Kinase Group** | **Quantified Spectra** | **Redundant Peptides** | **Competition** % |
|---|---|---|---|---|---|
| IPI00479760.6 | AAK1 | Other | 190 | 620 | 95.6 |
| IPI00029219.1 | ALK2 | TKL | 15 | 23 | 96.4 |
| IPI00410287.3 | AMPKa1 | CAMK | 171 | 660 | 95.2 |
| IPI00307755.3 | AMPKa2 | CAMK | 18 | 53 | 97.1 |
| IPI00298940.3 | AurA | Other | 139 | 179 | 93.6 |
| IPI00796914.1 | AurB | Other | 23 | 31 | 93.1 |
| IPI00337426.1 | BIKE | Other | 15 | 46 | 94.8 |
| IPI00005731.2 | BMPR1A | TKL | 5 | 7 | 84.1 |
| IPI00828081.1 | CaMK2d | CAMK | 108 | 1486 | 40.6 |
| IPI00169392.5 | CaMK2g | CAMK | 274 | 1352 | 75.2 |
| IPI00290239.2 | CaMKK2 | Other | 50 | 240 | 96.3 |
| IPI00026689.4 | CDC2 | CMGC | 18 | 22 | 94.3 |
| IPI00023530.6 | CDK5 | CMGC | 15 | 20 | 92.6 |
| IPI00552413.2 | CDK9 | CMGC | 21 | 34 | 97.6 |
| IPI00016613.2 | CK2a1 | Other | 96 | 310 | 95.2 |
| IPI00020602.1 | CK2a2 | Other | 82 | 153 | 95.4 |
| IPI00657861.1 | DDR1 | TK | 8 | 24 | 94.1 |
| IPI00296337.2 | DNAPK | Atypical | 14 | 48 | 89.2 |
| IPI00003479.3 | Erk2 | CMGC | 16 | 28 | 48.5 |
| IPI00413961.3 | FAK | TK | 133 | 447 | 96 |
| IPI00029263.2 | FER | TK | 89 | 284 | 96.7 |
| IPI00294344.2 | FES | TK | 44 | 72 | 96.9 |
| IPI00293565.4 | FLT4 | TK | 99 | 226 | 88.2 |
| IPI00219012.4 | FYN | TK | 8 | 35 | 93.2 |
| IPI00787531.1 | GAK | Other | 42 | 78 | 95.9 |
| IPI00292228.1 | GSK3A | CMGC | 50 | 145 | 94.6 |
| IPI00216190.1 | GSK3B | CMGC | 56 | 215 | 92.5 |
| IPI00099522.2 | HIPK3 | CMGC | 5 | 10 | 95.4 |
| IPI00029045.1 | IKKe | Other | 31 | 42 | 69 |
| IPI00026984.1 | IRAK3 | TKL | 8 | 9 | 86.2 |
| IPI00007641.2 | IRAK4 | TKL | 28 | 49 | 95.6 |
| IPI00015974.1 | IRE1 | Other | 4 | 7 | 83.4 |
| IPI00011633.3 | JAK1 | TK | 189 | 277 | 96.7 |
| IPI00024672.1 | JNK1 | CMGC | 50 | 397 | 84.3 |
| IPI00303550.2 | JNK2 | CMGC | 85 | 384 | 83.8 |
| IPI00021396.1 | KDR | TK | 12 | 20 | 92.5 |
| IPI00304742.4 | LOK | STE | 4 | 22 | 97.7 |
| IPI00794835.1 | LRRK2 | TKL | 19 | 25 | 87.8 |
| IPI00298625.2 | LYN | TK | 6 | 37 | 83.1 |
| IPI00185860.4 | MAP2K5 | STE | 18 | 21 | 87.4 |
| IPI00787127.1 | MAP3K1 | STE | 19 | 117 | 64.6 |
| IPI00513803.3 | MAP3K2 | STE | 7 | 10 | 94.7 |
| IPI00386260.3 | MAP3K4 | STE | 13 | 24 | 92.2 |
| IPI00412433.1 | MAP3K5 | STE | 12 | 67 | 94.1 |
| IPI00418221.3 | MAP3K6 | STE | 9 | 30 | 91.6 |
| IPI00412740.5 | MAP3K7 | STE | 14 | 26 | 85.8 |
| IPI00555838.1 | MARK2 | CAMK | 46 | 822 | 93.4 |
| IPI00183118.4 | MARK3 | CAMK | 4 | 105 | 96.2 |
| IPI00029756.1 | MER | TK | 6 | 7 | 96.5 |
| IPI00294528.1 | MET | TK | 16 | 84 | 94.2 |
| IPI00000977.1 | MLK3 | TKL | 40 | 59 | 94.9 |
| IPI00142487.2 | MLK4 | TKL | 10 | 24 | 85.8 |
| IPI00306833.3 | MPSK1 | Other | 7 | 9 | 92.1 |
| IPI00022536.1 | MSK2 | AGC | 26 | 70 | 93.2 |
| IPI00465101.5 | NEK3 | Other | 13 | 31 | 94.8 |
| IPI00301609.8 | NEK9 | Other | 256 | 698 | 83 |
| IPI00014068.1 | PAK4 | STE | 153 | 267 | 94.8 |
| IPI00012891.1 | PHKg2 | CAMK | 7 | 8 | 93.4 |
| IPI00009688.1 | PIP5K2A | Lipid Kinase | 14 | 46 | 98.7 |
| IPI00216470.1 | PIP5K2B | Lipid Kinase | 27 | 51 | 97.2 |
| IPI00152303.7 | PIP5K2C | Lipid Kinase | 89 | 193 | 97.3 |
| IPI00385449.4 | PKCa | AGC | 6 | 16 | 96.4 |
| IPI00436355.1 | PKG1 | AGC | 4 | 15 | 79.3 |
| IPI00844111.1 | PKN2 | AGC | 7 | 30 | 98 |
| IPI00021248.1 | PLK1 | Other | 12 | 30 | 94.6 |
| IPI00029702.1 | PYK2 | TK | 23 | 83 | 96.3 |
| IPI00021917.1 | RIPK2 | TKL | 33 | 43 | 94.4 |
| IPI00030273.1 | RON | TK | 28 | 74 | 96 |
| IPI00020898.1 | RSK2 | AGC | 31 | 59 | 95.6 |
| IPI00477982.1 | RSK3 | AGC | 40 | 122 | 95.5 |
| IPI00022827.1 | SLK | STE | 39 | 116 | 95.2 |
| IPI00328867.6 | SRC | TK | 35 | 166 | 89.5 |
| IPI00018597.1 | SYK | TK | 12 | 31 | 94.8 |
| IPI00293613.2 | TBK1 | Other | 260 | 363 | 88.8 |
| IPI00000878.3 | TEC | TK | 49 | 175 | 89.3 |
| IPI00005733.1 | TGFbR1 | TKL | 39 | 63 | 95.6 |
| IPI00412829.1 | TIE2 | TK | 27 | 38 | 91.4 |
| IPI00022633.3 | TNK1 | TK | 64 | 128 | 94.1 |
| IPI00022353.4 | TYK2 | TK | 107 | 154 | 96.6 |
| IPI00411818.4 | ULK3 | Other | 47 | 60 | 94.8 |
| IPI00807602.1 | ULK4 | Other | 9 | 33 | 84.7 |
| IPI00025830.1 | Wee1 | Other | 39 | 54 | 97.5 |
| IPI00013981.4 | YES | TK | 61 | 142 | 91.7 |

### Example 6: Kinobeads selectivity profiling of test compound CZC00019943

This example illustrates the use of a competition binding assay in cell lysate to establish the kinase selectivity profile of the test compound CZC00019943. This compound was added at defined concentrations (10 µM, 1 µM and 0.1 µM CZC00019943) to HL60 cell lysate thereby allowing the test compound to bind to the target proteins in the lysate. Then the lysate was contacted with the immobilized compound (CZC31326) to capture remaining free target proteins. The proteins bound to the immobilized compound were eluted with detergent-containing buffer, separated on a SDS-polyacryamide gel and analyzed by mass spectrometry as described in example 2.

The peptide extracts corresponding to samples treated with different concentrations of the test compound (10 µM, 1 µM and 0.1 µM CZC00019943) and the solvent control (0.5% DMSO) were treated with different variants of the isobaric tagging reagent (iTRAQ Reagents Multiplex Kit, part number 4352135, Applied Biosystems, Foster City, CA, USA). The iTRAQ reagents are a set of multiplexed, amine-specific, stable isotope reagents that can label peptides in up to four different biological samples enabling simultaneous identification and quantitation of peptides. The iTRAQ reagents were used according to instructions provided by the manufacturer.

The test compound CZC00019943 was used at three different concentrations in the cell lysate and the IC₅₀ values were normalized to the DMSO control. For selected kinases the IC₅₀ values were plotted against the concentration of CZC00019943 and curve fitting was performed using the Xlfit program (ID Busiess Solutions Ltd.) as peviously described. (Bantscheff et al., 2007. Nature Biotechnology 25, 1035-1044). The IC₅₀ value corresponds to the test compound concentration at which the relative intensity of the MS signal for a kinase is 50% compared to the solvent (DMSO) control. The logarythmic values (pIC₅₀) for kinases detected in this experiment are displayed as bar charts (Figure 12).

### Example 7: Assay for the identification of IRAK1 and IRAK4 interacting compounds

This example demonstrates a competitive binding assay in which test compounds are added directly into a cell lysate. Various concentrations of test compounds were added to HL60 lysate samples and allowed to bind to the proteins contained in the lysate sample. At the same time the affinity matrix with the immobilized compound (Figure 3, CZC31326) was added as well in order to capture proteins not bound to the test compound. After the incubation time the beads with captured proteins were separated from the lysate. Bound proteins were then eluted and the presence of IRAK1 and IRAK4 was detected and quantitavely determined using specific antibodies and the Odyssey Infrared Detection system. Further experimental protocols can be found in WO2006/134056. Dose response curves for free compound CZC31326 were generated (Figure 13).

### Washing of the affinity matrix

The affinity matrix as described in example 1 was washed three times in 15 ml tubes with 15 ml of 1xDP buffer containing 0.2% NP40. Beads were collected by gentle centrifugation (2 minutes at 1200 rpm in Herareus centrifuge) and finally resuspended in 1xDP buffer containing 0.2% NP40 to prepare a 5% beads slurry.

### Preparation of test compounds

Stock solutions of test compounds were prepared in DMSO corresponding to a 50-fold higher concentration compared to the final desired test concentration (e.g. a 0.5 mM stock solution was prepared for a final test concentration of 10 µM). This dilution scheme resulted in a final DMSO concentration of 2%. For control experiments (no test compound) a buffer containing 2% DMSO was used.

### Dilution of cell lysate

Cell lysates were prepared as described in example 2. For a typical experiment one lysate aliquot containing 50 mg of protein was thawed in a 21 °C water bath and then kept at 4°C. To the lysate one volume of 1xDP buffer containing protease inhibitor (1 tablet of protease inhibitor dissolved in 25 ml of 1x DP buffer containing 0.8% NP40; EDTA-free tablet protease inhibitor cocktail from Roche Applied Sciences, catalogue number 41647) was added so that a final NP40 concentration of 0.8% was obtained. The lysate was further diluted by adding 1x DP buffer containing 0.8% NP40 and proteinase inhibitors so that a final protein concentration of 10 mg/ml was achieved.

### Incubation of lysate with test compound and affinity matrix

To a 96 well filter plate (Multiscreen HTS, HV Filter Plates, Millipore #MSHVN4550) the following components were added per well: 100 µl affinity matrix (5% beads slurry), 3 µl of compound solution, and 50 µl of diluted cell lysate. Plates were sealed and incubated for two hours in a cold room on a Thermoxer with shaking (650 rpm). Afterwards the plate was washed twice with 220 µl washing buffer (1xDP 0.4% NP40). The filter plate was placed on top of a collection plate (Greiner bio-one, PP-microplate 96 well V-shape, 651201) and the beads were then eluted with 40 µl of 2x concentrated sample buffer (100 mM Tris, pH 7.4, 4% SDS, 0.01% Bromophenol blue, 20% glycerol, 50 mM DTT). The eluate was stored at -20°C.

### Detection and quantification of eluted kinases

The kinases in the eluates were detected and quantified by spotting on Nitrocellulose membranes and using a first antibody directed against the kinase of interest and a fluorescently labeled secondary antibody (anti-mouse or anti-rabbit IRDye™ antibodies from Rockland). The Odyssey Infrared Imaging system from LI-COR Biosciences (Lincoln, Nebraska, USA) was operated according to instructions provided by the manufacturer (Schutz-Geschwendener et al., 2004. Quantitative, two-color Western blot detection with infrared fluorescence. Published May 2004 by LI-COR Biosciences, www.licor.com).

After spotting of the eluates the nitrocellulose membrane (BioTrace NT; PALL, BTNT30R) was first blocked by incubation with Odyssey blocking buffer (LICOR, 927-40000) for one hour at room temperature. Blocked membranes were then incubated for 16 hours at 25°C with the first antibody diluted in Odyssey blocking buffer (LICOR #927-40000). Afterwards the membrane was washed two times for 5 minutes with 15ml PBS buffer containing 0.1% Tween 20 (Sigma, T2700) at room temperature. Then the membrane was incubated for 60 minutes at room temperature with the detection antibody (IRDye™ labelled antibody from Rockland) diluted in Odyssey blocking buffer (LICOR #927-40000) containing 0.2% Tween-20 and 0.02% SDS. Afterwards the membrane was washed four times for 6 minutes each with 1 x PBS buffer containing 0.1% Tween 20 at room temperature. Then the membrane was rinsed twice with PBS buffer to remove residual Tween-20. The membrane was kept in 1 x PBS buffer at 4°C and then scanned with the Odyssey instrument. Fluorescence signals were recorded and analysed according to the instructions of the manufacturer. Dose response curves were computed with the XL fit program (XLfit4 Excel Add-In Version 4.2.0 Build 13; IDBS, Guilford, UK).

### Source of antibodies:

Anti- IRAK1: R&D Systems, AF4048, goat, diluted 1:2000; anti-IRAK4: R&D Systems, AF3919, goat, diluted 1:2000; fluorescently labeled secondary antibody IRDeye800 donkey anti-goat, Rockland 605-732-125, diluted 1:5000.

**Table 8: Preparation of 5x-DP buffer**

| **Substance:** | **Stock solution** | **Final conc. in 1 x lysis buffer** | **Add for 115 x lysis buffer** |
|---|---|---|---|
| **Tris/HCl pH 7.5** | 1 M | 50 mM | 250 ml |
| **Glycerol** | 87% | 5% | 288 ml |
| **MgCl₂** | 1M | 1.5 mM | 7.5 ml |
| **NaCl** | 5M | 150 mM | 150 ml |
| **Na₃VO₄** | 100 mM | 1 mM | 50 ml |

The 5x-DP buffer was filtered through a 0.22 µm filter and stored in 40 ml-aliquots at -80°C. Stock solutions were obtained from the following suppliers: 1.0 M Tris/HCl pH 7.5 (Sigma, T-2663), 87% Glycerol (Merck, catalogue number 04091.2500); 1.0 M MgCl₂ (Sigma, M-1028); 5.0 M NaCl (Sigma, S-5150).

## Claims

1. An immobilization compound of formula (I) 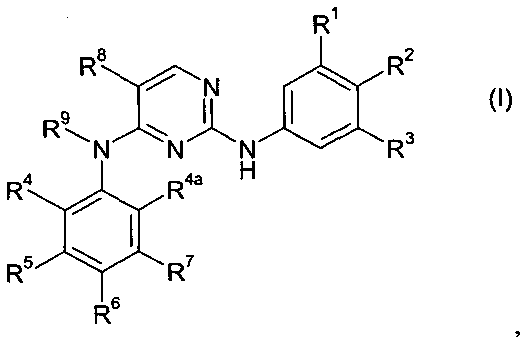 or a salt thereof, wherein
one of R¹, R², R³ is X-(CH₂)ₙ-NH₂ and the other two are independently selected from the group consisting of H; halogen; CN; C(O)OR¹⁰; OR¹⁰; C(O)R¹⁰; C(O)N(R¹⁰R^{10a}); S(O)₂N(R¹⁰R^{10a}); S(O)N(R¹⁰R^{10a}); S(O)₂R¹⁰; S(O)R¹⁰; N(R¹⁰)S(O)₂N(R^{10a}R^{10b}); SR¹⁰; N(R¹⁰R^{10a}); NO₂; OC(O)R¹⁰; N(R¹⁰)C(O)R^{10a}; N(R¹⁰)S(O)₂R^{10a}; N(R¹⁰)S(O)R^{10a}; N(R¹⁰)C(O)N(R^{10a}R^{10b}); N(R¹⁰)C(O)OR^{10a}; OC(O)N(R¹⁰R^{10a}); C₁₋₆alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R¹¹, which are the same or different;
X is a single covalent chemical bond; O; S; NH; NHC(O); or C(O)NH;
n is 0; 1; 2; 3; 4; 5; or 6, when X is a single covalent chemical bond or NHC(O); and n is 2; 3; 4; 5; or 6, when X is O; S; or C(O)NH;
R¹⁰, R^{10a}, R^{10b} are independently selected from the group consisting of H; C₁₋₆ alkyl; C₂₋₆ alkenyl; and C₂₋₆alkynyl, wherein C₁₋₆ alkyl; C₂₋₆ alkenyl; and C₂₋₆ alkynyl are optionally substituted with one or more R¹², which are the same or different;
R¹¹, R¹² are independently selected from the group consisting of halogen; CN; C(O)OR¹³; OR¹³; C(O)R¹³; C(O)N(R¹³R^{13a}); S(O)₂N(R¹³R^{13a}); S(O)N(R¹³R^{13a}); S(O)₂R¹³; S(O)R¹³; N(R¹³)S(O)₂N(R^{13a}R^{13b}); N(R¹³)S(O)N(R^{13a}R^{13b}); SR¹³; N(R¹³R^{13a}); NO₂; OC(O)R¹³; N(R¹³)C(O)R^{13a}; N(R¹³)S(O)₂R^{13a}; N(R¹³)S(O)R^{13a}; N(R¹³)C(O)N(R^{13a}R^{13b}); N(R¹³)C(O)OR^{13a}; and OC(O)N(R¹³R^{13a});
R¹³, R^{13a}, R^{13b} are independently selected from the group consisting of H; C₁₋₆ alkyl; C₂₋₆alkenyl; and C₂₋₆alkynyl, wherein C₁₋₆ alkyl; C₂₋₆ alkenyl; and C₂₋₆ alkynyl are optionally substituted with one or more halogen, which are the same or different;
R⁴, R⁵, R⁶, R⁷, R^{4a} are independently selected from the group consisting of H; X¹; halogen; CN; C(O)OR¹⁴; OR¹⁴; C(O)R¹⁴; C(O)N(R¹⁴R^{14a}); S(O)₂N(R¹⁴R^{14a}); S(O)N(R¹⁴R^{14a}); S(O)₂R¹⁴; S(O)R¹⁴; SR¹⁴; N(R¹⁴R^{14a}); NO₂; OC(O)R¹⁴; N(R¹⁴)C(O)R^{14a}; N(R¹⁴)S(O)₂R^{14a}; N(R¹⁴)S(O)R^{14a}; N(R¹⁴)C(O)N(R^{14a}R^{14b}); N(R¹⁴)C(O)OR^{14a}; OC(O)N(R¹⁴R^{14a}); C₁₋₆ alkyl; C₂₋₆ alkenyl; and C₂₋₆alkynyl, wherein C₁₋₆ alkyl; C₂₋₆ alkenyl; and C₂₋₆alkynyl are optionally substituted with one or more R¹⁵, which are the same or different,
provided that one of R⁴, R⁵, R⁶, R⁷, R^{4a} is X¹;
R¹⁴, R^{14a}, R^{14b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R¹⁶, which are the same or different;
R¹⁵, R¹⁶ are independently selected from the group consisting of halogen; CN; C(O)OR¹⁷; OR¹⁷; C(O)R¹⁷; C(O)N(R¹⁷R^{17a}); S(O)₂N(R¹⁷R^{17a}); S(O)N(R¹⁷R^{17a}); S(O)₂R¹⁷; S(O)R¹⁷; N(R¹⁷)S(O)₂N(R^{17a}R^{17b}); N(R¹⁷)S(O)N(R^{17a}R^{17b}); SR¹⁷; N(R¹⁷R^{17a}); NO₂; OC(O)R¹⁷; N(R¹⁷)C(O)R^{17a}; N(R¹⁷)S(O)₂R^{17a}; N(R¹⁷)S(O)R^{17a};
N(R¹⁷)C(O)N(R^{17a}R^{17b}); N(R¹⁷)C(O)OR^{17a}; OC(O)N(R¹⁷R^{17a}); C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more halogen, which are the same or different;
R¹⁷, R^{17a}, R^{17b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more halogen, which are the same or different;
X¹ is N(R¹S^{18a})S(O)₂R¹⁸; or N(R^{18a})S(O)₂N(R^{18b}R¹⁸);
R⁹, R^{18a}, R^{18b} are independently selected from the group consisting of H; C₁₋₄ alkyl; C₃₋₅ cycloalkyl; and C₃₋₅ cycloalkylmethyl, wherein C₁₋₄ alkyl; C₃₋₅ cycloalkyl and C₃₋₅ cycloalkylmethyl are optionally substituted with one or more halogen, which are the same or different;
R¹⁸ is T; C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more R¹⁹, which are the same or different;
R¹⁹ is T; halogen; CN; C(O)OR²⁰; OR²⁰; C(O)R²⁰; C(O)N(R²⁰R^{20a}); S(O)₂N(R²⁰R^{20a}); S(O)N(R²⁰R^{20a}); S(O)₂R²⁰; S(O)R²⁰; N(R²⁰)S(O)₂N(R)^{20a}R^{20b}); N(R²⁰)S(O)N(R^{20a}R^{20b}); , SR²⁰; , N(R²⁰R^{20a}); NO₂; OC(O)R²⁰; , N(R²⁰)C(O)R^{20a}; N(R²⁰)S(O)₂R^{20a}; N(R²⁰)S(O)R^{20a}; N(R²⁰)C(O)N(R^{20a}R^{20b}); N(R²⁰)C(O)OR^{20a}; , OC(O)N(R²⁰R^{20a}); C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more halogen, which are the same or different;
R²⁰, R^{20a}, R^{20b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more halogen, which are the same or different;
T is phenyl; C₃₋₇ cycloalkyl; or 4 to 7 membered heterocyclyl, wherein T is optionally substituted with one or more R²¹, which are the same or different;
R²¹, is halogen; CN; C(O)OR²²; OR²²; oxo (=O), where the ring is at least partially saturated; C(O)R²²; C(O)N(R²²R^{22a}); S(O)₂N(R²²R^{22a}); S(O)N(R²²R^{22a}); S(O)₂R²²; S(O)R²²; N(R²²)S(O)₂N(R^{22a}R^{22b}); N(R²²)S(O)N(R^{22a}R^{22b}); SR²²; N(R²²R^{22a})_{;} NO₂; OC(O)R²²; , N(R²²)C(O)R^{22a}; N(R²²)S(O)₂R^{22a}; N(R²²)S(O)R^{22a}; N(R²²)C(O)N(R^{22a}R^{22b}); N(R²²)C(O)OR^{22a}; OC(O)N(R²²R^{22a}); C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more halogen, which are the same or different;
R²², R^{22a}, R^{22b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more halogen, which are the same or different;
R⁸ is H; F; Cl; Br; CN; C₁₋₄ alkyl; CH₂F; CHF₂; CF₃; OH; OCH₃; NO₂; NH₂; NHCH₃; N(CH₃)₂; or NO₂.

2. The immobilization compound of claim 1, selected from the group consisting of 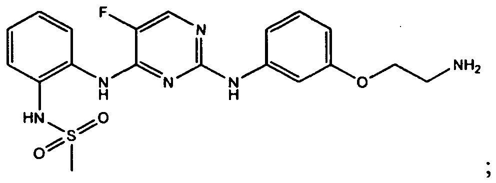 and

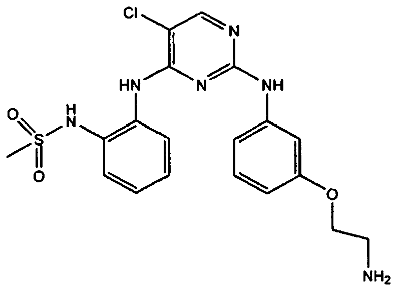

3. A method for the preparation of an immobilization product, wherein at least one immobilization compound according to claim 1 or 2 is immobilized on a solid support, in particular wherein the solid support is selected from the group consisting of agarose, modified agarose, sepharose beads (e.g. NHS-activated sepharose), latex, cellulose, and ferro- or ferrimagnetic particles.

4. The method of claim 3, wherein the immobilization product results from a covalent direct or linker mediated attachment of the immobilization compound to the solid support, in particular wherein the linker is a C₁₋₁₀ alkylene group, which is optionally interrupted or terminated by one or more atoms or functional groups selected from the group consisting of S, O, NH, C(O)O, C(O), and C(O)NH and wherein the linker is optionally substituted with one or more substituents independently selected from the group consisting of halogen, OH, NH₂, C(O)H, C(O)NH₂, SO₃H, NO₂, and CN, in particular wherein said immobilization occurs via the primary amino group in the residue X-(CH₂)ₙ-NH₂ for one of R¹, R², R³ in formula (I) of claim 1.

5. An immobilization product, obtainable by the method of any of claims 3 or 4.

6. An immobilization product, comprising the immobilization compound of any of claims 1 or 2 immobilized on a solid support, in particular wherein the solid support is selected from the group consisting of agarose, modified agarose, sepharose beads (e.g. NHS-activated sepharose), latex, cellulose, and ferro- or ferrimagnetic particles.

7. A method for the identification of a kinase interacting compound, comprising the steps of
a) providing a protein preparation containing a variety of kinases,
b) contacting the protein preparation with the immobilization product of any of claims 5 or 6 and with a given compound under conditions allowing the formation of one or more different complexes between one of the kinases and the immobilization product, and
c) detecting the complex or the complexes formed in step b).

8. A method for the identification of a kinase interacting compound, comprising the steps of:
a) providing two aliquots of a protein preparation containing a variety of kinases,
b) contacting one aliquot with the immobilization product of any of claims 5 or 6 under conditions allowing the formation of one or more different complexes between one of the kinases and the immobilization product,
c) contacting the other aliquot with the immobilization product of any of claims 5 or 6 and with a given compound under conditions allowing the formation of one or more different complexes between one of the kinases and the immobilization product, and
d) determining the amount of the complex or the complexes formed in steps b) and c).

9. A method for the identification of a kinase interacting compound, comprising the steps of:
a) providing two aliquots comprising each at least one cell containing a variety of kinases,
b) incubating one aliquot with a given compound,
c) harvesting the cells of each aliquot,
d) lysing the cells in order to obtain protein preparations,
e) contacting the protein preparations with the immobilization product of any of claims 5 or 6 under conditions allowing the formation of one or more different complexes between one of the kinases and the immobilization product, and
f) determining the amount of the complex or the complexes formed in each aliquot in step e).

10. The method of any of claims 7 to 9, wherein variety of kinases includes one or more members of the IRAK family, preferably IRAK 1 or IRAK 4.

11. The method of any of claims 7 to 9, wherein the variety of kinases includes NIK.

12. The method of any of claims 8 to 11, wherein a reduced amount of the complex formed in the aliquot incubated with the compound in comparison to the aliquot not incubated with the compound indicates that said kinase is a target of the compound.

13. The method of any of claims 8 to 11, wherein the amount of the complex is determined by separating the kinase from the immobilization product and subsequent detection of the separated kinase or subsequent determination of the amount of the separated kinase, in particular wherein the kinase is detected or the amount of the kinase is determined by mass spectrometry or immunodetection methods, preferably with an antibody directed against the kinase.

14. The method of any of claims 7 to 13, wherein said given compound is selected from the group consisting of synthetic compounds, or organic synthetic drugs, more preferably small molecule organic drugs, and natural small molecule compounds.

15. The method of any of claims 7 to 14, wherein the given compound is a kinase inhibitor.

16. The method of any of claims 7 to 15, wherein the provision of a protein preparation includes the steps of harvesting at least one cell containing kinases and lysing the cell.

17. The method of any of claims 7 to 16, wherein the steps of the formation of the complex are performed under essentially physiological conditions.

18. A method for determining the presence of one or more kinases in a sample, comprising the steps of:
a) providing a protein preparation expected to contain said one or more kinases,
b) contacting the protein preparation with the immobilization product of any of claims 5 or 6 under conditions allowing the formation of a complex between one of the kinases and the immobilization product, and
c) detecting whether one or more kinases have formed a complex with the immobilization product.

19. Use of the immobilization compound according to any of claims 1 or 2 or of the immobilization product of any of claims 5 or 6 for the identification of kinase interacting compounds or for the purification of kinases.

## Patentansprüche

1. Immobilisierungsverbindung der Formel (I) 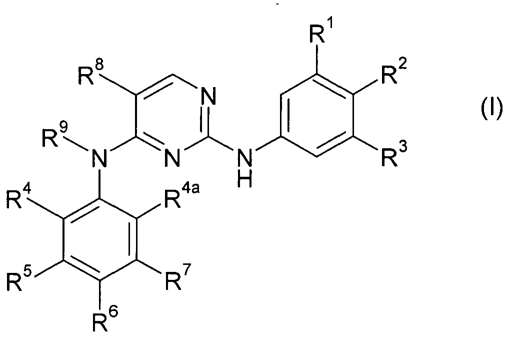 oder ein Salz davon, wobei
einer der Reste R¹, R², R³ X-(CH₂)ₙ-NH₂ ist und die anderen zwei unabhängig voneinander ausgewählt sind aus der Gruppe bestehend aus H; Halogen; CN; C(O)OR¹⁰; OR¹⁰; C(O)R¹⁰; C(O)N(R¹⁰R^{10a}); S(O)₂N(R¹⁰R^{10a}); S(O)N(R¹⁰R^{10a}); S(O)₂R¹⁰; S(O)R¹⁰; N(R¹⁰)S(O)₂N(R^{10a}R^{10b}); SR¹⁰; N(R¹⁰R^{10a}); NO₂; OC(O)R¹⁰; N(R¹⁰)C(O)R^{10a}; N(R¹⁰)S(O)₂R^{10a}; N(R¹⁰)S(O)R^{10a}; N(R¹⁰)C(O)N(R^{10a}R^{10b})_{;} N(R¹⁰)C(O)OR^{10a}; OC(O)N(R¹⁰R^{10a}); C₁₋₆ Alkyl; C₂₋₆ Alkenyl; und C₂₋₆ Alkynyl, wobei C₁₋₆ Alkyl; C₂₋₆ Alkenyl; und C₂₋₆ Alkynyl wahlweise substituiert sind mit einem oder mehreren R¹¹, die identisch oder unterschiedlich sind;
X ist eine einzelne kovalente chemische Bindung; O; S; NH; NHC(O); oder C(O)NH;
n ist 0; 1; 2; 3; 4; 5; oder 6, wenn X eine einzelne kovalente chemische Bindung ist oder NHC(O); und n ist 2; 3; 4; 5; oder 6, wenn X O; S; oder C(O)NH ist;
R¹⁰, R^{10a}, R^{10b} sind unabhängig ausgewählt aus der Gruppe bestehend aus H; C₁₋₆ Alkyl; C₂₋₆ Alkenyl; und C₂₋₆ Alkynyl, wobei C₁₋₆ Alkyl; C₂₋₆ Alkenyl; und C₂₋₆ Alkynyl wahlweise substituiert sind mit einem oder mehreren R¹², die identisch oder unterschiedlich sind;
R¹¹, R¹² sind unabhängig ausgewählt aus der Gruppe bestehend aus Halogen; CN; C(O)OR¹³; OR¹³; C(O)R¹³; C(O)N(R¹³R^{13a}); S(O)₂N(R¹³R^{13a}); S(O)N(R¹³R^{13a}); , S(O)₂R¹³; , S(O)R¹³; , N(R¹³)S(O)₂N(R^{13a}R^{13b}); , N(R¹³)S(O)N(R^{13a}R^{13b}); , SR¹³; , N(R¹³R^{13a}); NO₂; OC(O)R¹³; N(R¹³)C(O)R^{13a}; N(R¹³)S(O)₂R^{13a}; N(R¹³)S(O)R^{13a}; N(R¹³)C(O)N(R^{13a}R^{13b}); N(R¹³)C(O)OR^{13a}; und OC(O)N(R¹³R^{13a});
R¹³, R^{13a}, R^{13b} sind unabhängig ausgewählt aus der Gruppe bestehend aus H; C₁₋₆ Alkyl; C₂₋₆ Alkenyl; und C₂₋₆ Alkynyl, wobei C₁₋₆ Alkyl; C₂₋₆ Alkenyl; und C₂₋₆ Alkynyl wahlweise substituiert sind mit einem oder mehreren Halogen(en), die identisch oder unterschiedlich sind;
R⁴, R⁵, R⁶, R⁷, R^{4a} sind unabhängig ausgewählt aus der Gruppe bestehend aus H; X¹; Halogen; CN; C(O)OR¹⁴; OR¹⁴; C(O)R¹⁴; C(O)N(R¹⁴R^{14a}); S(O)₂N(R¹⁴R^{14a}); S(O)N(R¹⁴R^{14a}); S(O)₂R¹⁴; S(O)R¹⁴; SR¹⁴; N(R¹⁴R^{14a}); NO₂; OC(O)R¹⁴; N(R¹⁴)C(O)R^{14a}; N(R¹⁴)S(O)₂R^{14a}; N(R¹⁴)S(O)R^{14a}; N(R¹⁴)C(O)N(R^{14a}R^{14b}); N(R¹⁴)C(O)OR^{14a}; OC(O)N(R¹⁴R^{14a}); C₁₋₆ Alkyl; C₂₋₆ Alkenyl; und C₂₋₆ Alkynyl, wobei C₁₋₆ Alkyl; C₂₋₆ Alkenyl; und C₂₋₆ Alkynyl wahlweise substituiert sind mit einem oder mehreren R¹⁵, die identisch oder unterschiedlich sind, vorausgesetzt dass eines von R⁴, R⁵, R⁶, R⁷, R^{4a} ist X¹;
R¹⁴, R^{14a}, R^{14b} sind unabhängig ausgewählt aus der Gruppe bestehend aus H; C₁₋₆ Alkyl; C₂₋₆ Alkenyl; und C₂₋₆ Alkynyl, wobei C₁₋₆ Alkyl; C_{Z}_₆ Alkenyl; und C_{Z}_₆ Alkynyl wahlweise substituiert sind mit einem oder mehreren R¹⁶, die identisch oder unterschiedlich sind;
R¹⁵, R¹⁶ sind unabhängig ausgewählt aus der Gruppe bestehend aus Halogen; CN; C(O)OR¹⁷; OR¹⁷; C(O)R¹⁷; C(O)N(R¹⁷R^{17a}); S(O)₂N(R¹⁷R^{17a}); S(O)N(R¹⁷R^{17a}); S(O)₂R¹⁷; S(O)R¹⁷; N(R¹⁷)S(O)₂N(R^{17a}R^{17b}); N(R¹⁷)S(O)N(R^{17a}R^{17b}); SR¹⁷; N(R¹⁷R^{17a}); NO₂; OC(O)R¹⁷; N(R¹⁷)C(O)R^{17a}; N(R¹⁷)S(O)₂R^{17a}; N(R¹⁷)S(O)R^{17a}; N(R¹⁷)C(O)N(R^{17a}R^{17b}); N(R¹⁷)C(O)OR^{17a}; OC(O)N(R¹⁷R^{17a}); C₁₋₆ Alkyl; C₂₋₆ Alkenyl; und C₂₋₆ Alkynyl, wobei C₁₋₆ Alkyl; C₂₋₆ Alkenyl; und C₂₋₆ Alkynyl wahlweise substituiert sind mit einem oder mehreren Halogen(en), die identisch oder unterschiedlich sind;
R¹⁷, R^{17a}, R^{17b} sind unabhängig ausgewählt aus der Gruppe bestehend aus H; C₁₋₆ Alkyl; C₂₋₆ Alkenyl; und C₂₋₆ Alkynyl, wobei C₁₋₆ Alkyl; C₂₋₆ Alkenyl; und C₂₋₆ Alkynyl wahlweise substituiert sind mit einem oder mehreren Halogen(en), die identisch oder unterschiedlich sind;
X¹ ist N(R^{18a})S(O)₂R¹⁸; oder N(R^{18a})S(O)₂N(R^{18b}R¹⁸);
R⁹, R^{18a} R^{18b} sind unabhängig ausgewählt aus der Gruppe bestehend aus H; C₁₋₄ Alkyl; C₃₋₅ Cycloalkyl; und C₃₋₅ Cycloalkylmethyl, wobei C₁₋₄ Alkyl; C₃₋₅ Cycloalkyl und C₃₋₅ Cycloalkylmethyl wahlweise substituiert sind mit einem oder mehreren Halogen(en), die identisch oder unterschiedlich sind;
R¹⁸ ist T; C₁₋₆ Alkyl; C_{Z}_₆ Alkenyl; oder C₂₋₆ Alkynyl, wobei C₁₋₆ Alkyl; C₂₋₆ Alkenyl; und C_{Z}_₆ Alkynyl wahlweise substituiert sind mit einem oder mehreren R¹⁹, die identisch oder unterschiedlich sind;
R¹⁹ ist T; Halogen; CN; C(O)OR²⁰; OR²⁰; C(O)R²⁰; C(O)N(R²⁰R^{20a})_{;} S(O)₂N(R²⁰R^{20a}); S(O)N(R²⁰R^{20a}); S(O)₂R²⁰; S(O)R²⁰; N(R²⁰)S(O)₂N(R^{20a}R^{20b}); N(R²⁰)S(O)N(R^{20a}R^{20b}); SR²⁰; N(R²⁰R^{20a}); NO₂; OC(O)R²⁰; N(R²⁰)C(O)R^{20a}_{;} N(R²⁰)S(O)₂R^{20a}; N(R²⁰)S(O)R^{20a}; N(R²⁰)C(O)N(R^{20a}R^{20b}); N(R²⁰)C(O)OR^{20a}; OC(O)N(R²⁰R^{20a}); C₁₋₆ Alkyl; C₂₋₆ Alkenyl; oder C₂₋₆ Alkynyl, wobei C₁₋₆ Alkyl; C₂₋₆ Alkenyl; und C_{Z}_₆ Alkynyl wahlweise substituiert sind mit einem oder mehreren Halogen(en), die identisch oder unterschiedlich sind;
R²⁰, R^{20a}, R^{20b} sind unabhängig ausgewählt aus der Gruppe bestehend aus H; C₁₋₆ Alkyl; C₂₋₆ Alkenyl; und C₂₋₆ Alkynyl, wobei C₁₋₆ Alkyl; C_{Z}_₆ Alkenyl; und C₂₋₆ Alkynyl wahlweise substituiert sind mit einem oder mehreren Halogen(en), die identisch oder unterschiedlich sind;
T ist Phenyl; C₃₋₇ Cycloalkyl; oder ein 4 bis 7-gliedriges Heterocyclyl, wobei T wahlweise substituiert ist mit einem oder mehreren R²¹, die identisch oder unterschiedlich sind;
R²¹, ist Halogen; CN; C(O)OR²²; OR²²; Oxo (=O), wobei der Ring mindestens partiell gesättigt ist; C(O)R²²; C(O)N(R²²R^{22a}); S(O)₂N(R²²R^{22a})_{;} S(O)N(R²²R^{22a}); S(O)₂R²²; S(O)R²²_{;} N(R²²)S(O)₂N(R^{22a}R^{22b}); N(R²²)S(O)N(R^{22a}R^{22b}); SR²²; N(R²²R^{22a}); NO₂; OC(O)R²²; N(R²²)C(O)R^{22a}; N(R²²)S(O)₂R^{22a}; N(R²²)S(O)R^{22a}; N(R²²)C(O)N(R^{22a}R^{22b}); N(R²²)C(O)OR^{22a}; OC(O)N(R²²R^{22a}); C₁₋₆ Alkyl; C₂₋₆ Alkenyl; oder C₂₋₆ Alkynyl, wobei C₁₋₆ Alkyl; C₂₋₆ Alkenyl; und C₂₋₆ Alkynyl wahlweise substituiert sind mit einem oder mehreren Halogen(en), die identisch oder unterschiedlich sind;
R²², R^{22a}, R^{22b} sind unabhängig ausgewählt aus der Gruppe bestehend aus H; C₁₋₆ Alkyl; C₂₋₆ Alkenyl; und C₂₋₆ Alkynyl, wobei C₁₋₆ Alkyl; C₂₋₆ Alkenyl; und C₂₋₆ Alkynyl wahlweise substituiert sind mit einem oder mehreren Halogen(en), die identisch oder unterschiedlich sind;
R⁸ ist H; F; Cl; Br; CN; C₁₋₄ Alkyl; CH₂F; CHF₂; CF₃; OH; OCH₃; NO₂; NH₂; NHCH₃; N(CH₃)₂; oder NO₂.

2. Immobilisierungsverbindung nach Anspruch 1, ausgewählt aus der Gruppe bestehend aus 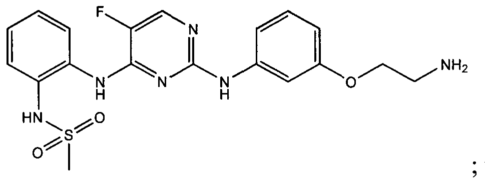 und

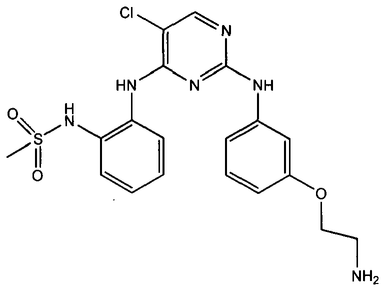

3. Verfahren für die Herstellung eines Immobilisierungsproduktes, wobei mindestens eine Immobilisierungsverbindung nach Anspruch 1 oder 2 auf einem festen Träger immobilisiert ist, insbesondere wobei der feste Träger ausgewählt ist aus der Gruppe bestehend aus Agarose, modifizierter Agarose, Sepharose-Beads (z.B. NHS-aktivierte Sepharose), Latex, Zellulose, und ferro- oder ferrimagnetischen Partikeln.

4. Verfahren nach Anspruch 3, wobei das Immobilisierungsprodukt aus einer direkten kovalenten oder Linker-vermittelten Bindung einer Immobilisierungsverbindung an einen festen Träger resultiert, insbesondere wobei der Linker eine C₁₋₁₀-Alkylengruppe ist, die wahlweise unterbrochen oder durch ein oder mehrere Atome oder funktionelle Gruppen ausgewählt aus der Gruppe bestehend aus S, O, NH, C(O)O, C(O) und C(O)NH abgeschlossen ist, und wobei der Linker wahlweise substituiert ist mit einem oder mehreren Substituenten unabhängig ausgewählt aus der Gruppe bestehend aus Halogen, OH, NH₂, C(O)H, C(O)NH₂, SO₃H, NO₂ und CN, insbesondere wobei die Immobilisierung durch eine primäre Aminogruppe im Rest X-(CH₂)ₙ-NH₂ für einen aus R¹, R², R³ in Formel (I) nach Anspruch 1 erfolgt.

5. Immobilisierungsprodukt, erhältlich durch das Verfahren nach einem der Ansprüche 3 oder 4.

6. Immobilisierungsprodukt, umfassend die Immobilisierungsverbindung nach einem der Ansprüche 1 oder 2 immobilisiert auf einem festen Träger, insbesondere wobei der feste Träger ausgewählt ist aus der Gruppe bestehend aus Agarose, modifizierter Agarose, Sepharose-Beads (z.B. NHS-aktivierte Sepharose), Latex, Zellulose, und ferro- oder ferrimagnetischen Partikeln.

7. Verfahren zur Identifizierung einer Kinase-interagierenden Verbindung, umfassend die Schritte
a) Bereitstellen einer Proteinzubereitung umfassend eine Vielzahl an Kinasen,
b) Inkontaktbringen der Proteinzubereitung mit einem Immobilisierungsprodukt nach einem der Ansprüche 5 oder 6 und mit einer gegebenen Verbindung unter Bedingungen, die die Bildung eines oder mehrerer unterschiedlicher Komplexe zwischen einer der Kinasen und dem Immobilisierungsprodukt erlauben, und
c) Bestimmen des in Schritt b) gebildeten Komplexes oder der Komplexe.

8. Verfahren zur Identifizierung einer Kinase-interagierenden Verbindung umfassend die Schritte
a) Bereitstellen zweier Aliquote einer Proteinzubereitung enthaltend eine Vielzahl an Kinasen,
b) Inkontaktbringen eines Aliquots mit dem Immobilisierungsprodukt nach einem der Ansprüche 5 oder 6 unter Bedingungen, die die Bildung eines oder mehrerer Komplexe zwischen einer der Kinasen und dem Immobilisierungsprodukt erlauben,
c) Inkontaktbringen des anderen Aliquots mit dem Immobilisierungsprodukt nach einem beliebigen der Ansprüche 5 oder 6 und mit einer gegebenen Verbindung unter Bedingungen, die die Bildung eines oder mehrerer unterschiedlicher Komplexe zwischen einer der Kinasen und dem Immobilisierungsprodukt erlauben, und
d) Bestimmen der Menge des Komplexes oder der Komplexe, die in den Schritten b) und c) gebildet wurde.

9. Verfahren zur Identifizierung einer Kinase-interagierenden Verbindung, umfassend die Schritte
a) Bereitstellen zweier Aliquote umfassend jeweils mindestens eine Zelle, die eine Vielzahl an Kinasen enthält,
b) Inkubieren eines Aliquots mit einer gegebenen Verbindung,
c) Ernten der Zellen eines jeden Aliquots,
d) Lysieren der Zellen um Proteinzubereitungen zu erhalten,
e) Inkontaktbringen der Proteinzubereitung mit dem Immobilisierungsprodukt nach einem der Ansprüche 5 oder 6 unter Bedingungen, die die Bildung eines oder mehrerer Komplexe zwischen einer der Kinasen und dem Immobilisierungsprodukt erlauben, und
f) Bestimmen der Menge des Komplexes oder der Komplexe, die in jedem der Aliquote in Schritt e) gebildet wurde.

10. Verfahren nach einem beliebigen der Ansprüche 7 bis 9, wobei die Vielzahl der Kinasen eine oder mehrere Mitglieder der IRAK-Familie, vorzugsweise IRAK 1 oder IRAK 4 einschließt.

11. Verfahren nach einem beliebigen der Ansprüche 7 bis 9, wobei die Vielzahl der Kinasen NIK einschließt.

12. Verfahren nach einem beliebigen der Ansprüche 8 bis 11, wobei eine geringere Menge des Komplexes, die sich in dem Aliquot, welches mit der Verbindung inkubiert wurde, im Vergleich zu dem Aliquot, welches nicht mit der Verbindung inkubiert wurde, gebildet hat, anzeigt, dass diese Kinase ein Ziel der Verbindung ist.

13. Verfahren nach einem beliebigen der Ansprüche 8 bis 11, wobei die Menge des Komplexes durch Abtrennen der Kinase von dem Immobilisierungsprodukt bestimmt wird und durch nachfolgende Detektion der abgetrennten Kinase oder nachfolgender Bestimmung der Menge der abgetrennten Kinase, vorzugsweise wobei die Kinase oder die Menge der Kinase durch Massenspektrometrie oder Immundetektionsverfahren bestimmt wird, vorzugsweise mit einem Antikörper gerichtet gegen die Kinase.

14. Verfahren nach einem beliebigen der Ansprüche 7 bis 13, wobei die gegebene Verbindung ausgewählt ist aus der Gruppe bestehend aus synthetischen Verbindungen oder organisch synthetischen Wirkstoffen, vorzugsweise niedermolekularen organischen Wirkstoffen und natürlich niedermolekularen Verbindungen.

15. Verfahren nach einem beliebigen der Ansprüche 7 bis 14, wobei die gegebene Verbindung ein Kinaseinhibitor ist.

16. Verfahren nach einem beliebigen der Ansprüche 7 bis 15, wobei die Bereitstellung der Proteinzubereitung die Schritte des Emtens mindestens einer Zelle, die Kinasen enthält, und Lysieren der Zelle umfasst.

17. Verfahren nach einem beliebigen der Ansprüche 7 bis 19, wobei die Schritte der Bildung des Komplexes unter im Wesentlichen physiologischen Bedingungen durchgeführt werden.

18. Verfahren zur Bestimmung der Anwesenheit einer oder mehrerer Kinasen in einer Probe, umfassend die Schritte:
a) Bereitstellen einer Proteinzubereitung, von der ausgegangen wird, dass sie eine oder mehrere Kinasen enthält,
b) Inkontaktbringen der Proteinzubereitung mit einem Immobilisierungsprodukt nach einem der Ansprüche 5 oder 6 unter Bedingungen, die die Bildung eines Komplexes zwischen einer der Kinasen und dem Immobilisierungsprodukt erlauben, und
c) Bestimmen, ob eine oder mehrere Kinasen einen Komplex mit dem Immobilisierungsprodukt gebildet haben.

19. Verwendung der Immobilisierungsverbindung gemäß einem der Ansprüche 1 oder 2 oder des Immobilisierungsprodukts nach einem der Ansprüche 5 oder 6 für die Identifizierung von Kinase-interagierenden Verbindungen oder für die Aufreinigung von Kinasen.

## Revendications

1. Composé d'immobilisation de formule (I) 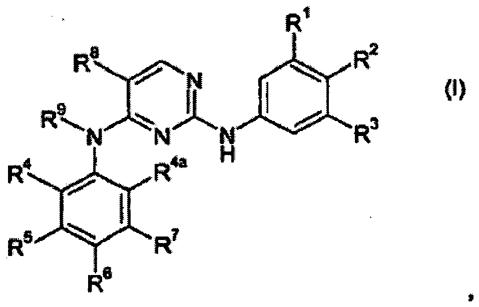 ou un sel de celui-ci, où
l'un de R¹, R², R³ représente X-(CH₂)ₙ-NH₂ et les deux autres sont choisis indépendamment dans le groupe constitué par H ; un atome d'halogène ; les groupes CN; C(O)OR¹⁰; OR¹⁰; C(O)R¹⁰; C(O)N(R¹⁰R^{10a}); S(O)₂N(R¹⁰R^{10a}); S(O)N(R¹⁰R^{10a}); S(O)₂R¹⁰; S(O)R¹⁰; N(R¹⁰)S(O)₂N(R^{10a}R^{10b}); SR¹⁰; N(R¹⁰R^{10a}); NO₂; OC(O)R¹⁰; N(R¹⁰)C(O)R^{10a}; N(R¹⁰)S(O)₂R^{10a}; N(R¹⁰)S(O)R^{10a}; N(R¹⁰)C(O)N(R^{10a}R^{10b}); N(R¹⁰)C(O)OR^{10a}; OC(O)N(R¹⁰R^{10a}); alkyle en C₁ à C₆; alcényle en C₂ à C₆ ; et alcynyle en C₂ à C₆, où les groupes alkyle en C₁ à C₆; alcényle en C₂ à C₆ ; et alcynyle en C₂ à C₆ sont éventuellement substitués par un ou plusieurs R¹¹, qui sont identiques ou différents ;
X représente une liaison chimique covalente simple ; O; S ; NH ; NHC(O) ; ou C(O)NH;
n vaut 0 ; 1 ; 2 ; 3 ; 4 ; 5 ; ou 6, lorsque X représente une liaison chimique covalente simple ou NHC(O) ; et n vaut 2 ; 3 ; 4 ; 5 ; ou 6, lorsque X représente O ; S ; ou C(O)NH ;
R¹⁰, R^{10a}, R^{10b} sont choisis indépendamment dans le groupe constitué par H ; les groupes alkyle en C₁ à C₆; alcényle en C₂ à C₆ ; et alcynyle en C₂ à C₆, où les groupes alkyle en C₁ à C₆ ; alcényle en C₂ à C₆ ; et alcynyle en C₂ à C₆ sont éventuellement substitués par un ou plusieurs R¹², qui sont identiques ou différents ;
R¹¹, R¹² sont choisis indépendamment dans le groupe constitué par un atome d'halogène; les groupes CN; C(O)OR¹³; OR¹³; C(O)R¹³; C(O)N(R¹³R^{13a}); S(O)₂N(R¹³R^{13a}); S(O)N(R¹³R^{13a}) ; S(O)₂R¹³; S(O)R¹³; N(R¹³)S(O)₂N(R^{13a}R^{13b}); N(R¹³)S(O)N(R^{13a}R^{13b}); SR¹³; N(R¹³R^{13a}); NO₂; OC(O)R¹³; N(R¹³)C(O)R^{13a}; N(R¹³)S(O)₂R^{13a}; N(R¹³)S(O)R^{13a}; N(R¹³)C(O)N(R^{13a}R^{13b}); N(R¹³)C(O)OR^{13a}; et OC(O)N(R¹³R^{13a});
R¹³, R^{13a}, R^{13b} sont choisis indépendamment dans le groupe constitué par H; les groupes alkyle en C₁ à C₆; alcényle en C₂ à C₆ ; et alcynyle en C₂ à C₆, où les groupes alkyle en C₁ à C₆; alcényle en C₂ à C₆ ; et alcynyle en C₂ à C₆ sont éventuellement substitués par un ou plusieurs atomes d'halogène, qui sont identiques ou différents ;
R⁴, R⁵, R⁶, R⁷, R^{4a} sont choisis indépendamment dans le groupe constitué par H ; X¹ ; un atome d'halogène; les groupes CN; C(O)OR¹⁴; OR¹⁴; C(O)R¹⁴; C(O)N(R¹⁴R^{14a}); S(O)₂N(R¹⁴R^{14a}); S(O)N(R¹⁴R^{14a}); S(O)₂R¹⁴; S(O)R¹⁴; SR¹⁴; N(R¹⁴R^{14a}); NO₂; OC(O)R¹⁴; N(R¹⁴)C(O)R^{14a}; N(R¹⁴)S(O)₂R^{14a}; N(R¹⁴)S(O)R^{14a}; N(R¹⁴)C(O)N(R^{14a}R^{14b}); N(R¹⁴)C(O)OR^{14a}; OC(O)N(R¹⁴R^{14a}); alkyle en C₁ à C₆ ; alcényle en C₂ à C₆ ; et alcynyle en C₂ à C₆, où les groupes alkyle en C₁ à C₆ ; alcényle en C₂ à C₆ ; et alcynyle en C₂ à C₆ sont éventuellement substitués par un ou plusieurs R¹⁵, qui sont identiques ou différents,
à condition que l'un de R⁴, R⁵, R⁶, R⁷, R^{4a} représente X¹;
R¹⁴, R^{14a}, R^{14b} sont choisis indépendamment dans le groupe constitué par H ; les groupes alkyle en C₁ à C₆ ; alcényle en C₂ à C₆ ; et alcynyle en C₂ à C₆, où les groupes alkyle en C₁ à C₆; alcényle en C₂ à C₆ ; et alcynyle en C₂ à C₆ sont éventuellement substitués par un ou plusieurs R¹⁶, qui sont identiques ou différents ;
R¹⁵, R¹⁶ sont choisis indépendamment dans le groupe constitué par un atome d'halogène; les groupes CN; C(O)OR¹⁷; OR¹⁷; C(O)R¹⁷; C(O)N(R¹⁷R^{17a}); S(O)₂N(R¹⁷R^{17a}); S(O)N(R¹⁷R^{17a}); S(O)₂R¹⁷; S(O)R¹⁷; N(R¹⁷)S(O)₂N(R^{17a}R^{17b}); N(R¹⁷)S(O)N(R^{17a}R^{17b}); SR¹⁷; N(R¹⁷R^{17a}) ; NO₂; OC(O)R¹⁷; N(R¹⁷)C(O)R^{17a}; N(R¹⁷)S(O)₂R^{17a}; , N(R¹⁷)S(O)R^{17a}; , N(R¹⁷)C(O)N(R^{17a}R^{17b}); , N(R¹⁷)C(O)OR^{17a}; OC(O)N(R¹⁷R^{17a}); alkyle en C₁ à C₆ ; alcényle en C₂ à C₆ ; et alcynyle en C₂ à C₆, où les groupes alkyle en C₁ à C₆ ; alcényle en C₂ à C₆ ; et alcynyle en C₂ à C₆ sont éventuellement substitués par un ou plusieurs atomes d'halogène, qui sont identiques ou différents ;
R¹⁷ , R^{17a}, R^{17b} sont choisis indépendamment dans le groupe constitué par H ; les groupes alkyle en C₁ à C₆; alcényle en C₂ à C₆ ; et alcynyle en C₂ à C₆, où les groupes alkyle en C₁ à C₆; alcényle en C₂ à C₆ ; et alcynyle en C₂ à C₆ sont éventuellement substitués par un ou plusieurs atomes d'halogène, qui sont identiques ou différents ;
X¹ représente N(R^{18a})S(O)₂R¹⁸; ou N(R^{18a})S(O)₂N(R^{18b}R¹⁸);
R⁹, R^{18a}, R^{18b} sont choisis indépendamment dans le groupe constitué par H ; les groupes alkyle en C₁ à C₄ ; cycloalkyle en C₃ à C₅; et cycloalkylméthyle en C₃ à C₅, où les groupes alkyle en C₁ à C₄; cycloalkyle en C₃ à C₅; et cycloalkylméthyle en C₃ à C₅, sont éventuellement substitués par un ou plusieurs atomes d'halogène, qui sont identiques ou différents ;
R¹⁸ représente T ; les groupes alkyle en C₁ à C₆; alcényle en C₂ à C₆ ; ou alcynyle en C₂ à C₆, où les groupes alkyle en C₁ à C₆ ; alcényle en C₂ à C₆ ; et alcynyle en C₂ à C₆ sont éventuellement substitués par un ou plusieurs R¹⁹, qui sont identiques ou différents ;
R¹⁹ représente T ; un atome d'halogène ; un groupe CN ; C(O)OR²⁰; OR²⁰; C(O)R²⁰; C(O)N(R²⁰R^{20a}); S(O)₂N(R²⁰R^{20a}); S(O)N(R²⁰R^{20a}); S(O)₂R²⁰; S(O)R²⁰; N(R²⁰)S(O)₂N(R^{20a}R^{20b}); , N(R²⁰)S(O)N(R^{20a}R^{20b}); , SR²⁰; , N(R²⁰R^{20a}); , NO₂; OC(O)R²⁰; N(R²⁰)C(O)R^{20a}; N(R²⁰)S(O)₂R^{20a}; N(R²⁰)S(O)R^{20a}; N(R²⁰)C(O)N(R^{20a}R^{20b}); N(R²⁰)C(O)OR^{20a}; OC(O)N(R²⁰)R^{20a}); alkyle en C₁ à C₆ ; alcényle en C₂ à C₆ ; ou alcynyle en C₂ à C₆, où les groupes alkyle en C₁ à C₆ ; alcényle en C₂ à C₆ ; et alcynyle en C₂ à C₆ sont éventuellement substitués par un ou plusieurs atomes d'halogène, qui sont identiques ou différents ;
R²⁰, R^{20a}, R^{20b} sont choisis indépendamment dans le groupe constitué par H ; les groupes alkyle en C₁ à C₆ ; alcényle en C₂ à C₆ ; et alcynyle en C₂ à C₆, où les groupes alkyle en C₁ à C₆ ; alcényle en C₂ à C₆ ; et alcynyle en C₂ à C₆ sont éventuellement substitués par un ou plusieurs atomes d'halogène, qui sont identiques ou différents ;
T représente un groupe phényle ; cycloalkyle en C₃ à C₇; ou hétérocyclyle de 4 à 7 chaînons, où T est éventuellement substitué par un ou plusieurs R²¹, qui sont identiques ou différents;
R²¹ représente un atome d'halogène, un groupe CN ; C(O)OR²²; OR²²; oxo (=O), où le cycle est au moins partiellement saturé; C(O)R²²; C(O)N(R²²R^{22a}); S(O)₂N(R²²R^{22a}); S(O)N(R²²R^{22a}); S(O)₂R²²; S(O)R²²; N(R²²)S(O)₂N(R^{22a}R^{22b}); N(R²²)S(O)N(R^{22a}R^{22b}); SR²²; N(R²²R^{22a}); NO₂; OC(O)R²²; N(R²²)C(O)R^{22a}; N(R²²)S(O)₂R^{22a}; , N(R²²)S(O)R^{22a}; , N(R²²)C(O)N(R^{22a}R^{22b}); , N(R²²)C(O)OR^{22a}; OC(O)N(R²²R^{22a}); alkyle en C₁ à C₆ ; alcényle en C₂ à C₆ ; ou alcynyle en C₂ à C₆, où les groupes alkyle en C₁ à C₆ ; alcényle en C₂ à C₆ ; et alcynyle en C₂ à C₆ sont éventuellement substitués par un ou plusieurs atomes d'halogène, qui sont identiques ou différents ;
R²², R^{22a}, R^{22b} sont choisis indépendamment dans le groupe constitué par H ; les groupes alkyle en C₁ à C₆ ; alcényle en C₂ à C₆ ; et alcynyle en C₂ à C₆, où les groupes alkyle en C₁ à C₆ ; alcényle en C₂ à C₆ ; et alcynyle en C₂ à C₆ sont éventuellement substitués par un ou plusieurs atomes d'halogène, qui sont identiques ou différents ;
R⁸ représente H ; F ; Cl; Br ; CN ; alkyle en C₁ à C₄ ; CH₂F; CHF₂; CF₃; OH ; OCH₃; NO₂; NH₂; NHCH₃; N(CH₃)₂; ou NO₂.

2. Composé d'immobilisation selon la revendication 1, choisi dans le groupe constitué par : 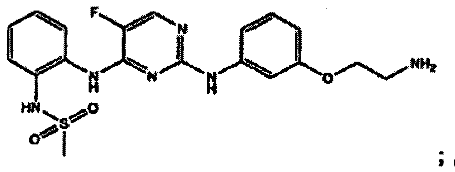 et

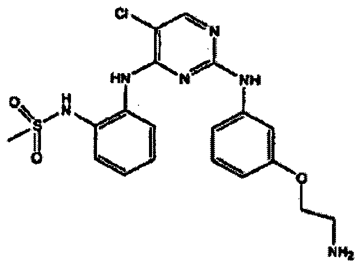

3. Procédé de préparation d'un produit d'immobilisation, dans lequel au moins un composé d'immobilisation selon la revendication 1 ou 2 est immobilisé sur un support solide, en particulier où le support solide est choisi dans le groupe constitué d'agarose, d'agarose modifié, de billes de sépharose (par exemple, sépharose activé par NHS), de latex, de cellulose, et de particules ferro- ou ferrimagnétiques.

4. Procédé selon la revendication 3, dans lequel le produit d'immobilisations résulte d'une fixation covalente directe ou médiée par un lieur du composé d'immobilisation au support solide, en particulier où le lieur est un groupe alkylène en C₁ à C₁₀, qui est éventuellement interrompu ou terminé par un ou plusieurs atomes ou groupes fonctionnels choisis dans le groupe constitué par S, O, NH, C(O)O, C(O), et C(O)NH et où le lieur est éventuellement substitué par un ou plusieurs substituants choisis indépendamment dans le groupe constitué par un atome d'halogène, les groupes OH, NH₂, C(O)H, C(O)NH₂, SO₃H, NO₂, et CN, en particulier où ladite immobilisation se produit par l'intermédiaire du groupe amino primaire dans le résidu X-(CH₂)ₙ-NH₂ pour l'un de R¹, R², R³ dans la formule (I) selon la revendication 1.

5. Produit d'immobilisation, pouvant être obtenu par le procédé selon l'une quelconque des revendications 3 ou 4.

6. Produit d'immobilisation, comprenant le composé d'immobilisation selon l'une quelconque des revendications 1 ou 2 immobilisé sur un support solide, en particulier où le support solide est choisi dans le groupe constitué d'agarose, d'agarose modifié, de billes de sépharose (par exemple, sépharose activé par NHS), de latex, de cellulose, et de particules ferro- ou ferrimagnétiques.

7. Procédé d'identification d'un composé interagissant avec une kinase, comprenant les étapes de
a) fourniture d'une préparation de protéines contenant toute une diversité de kinases,
b) mise en contact de la préparation de protéines avec le produit d'immobilisation selon l'une quelconque des revendications 5 ou 6 et avec un composé donné dans des conditions permettant la formation d'un ou de plusieurs complexes différents entre l'une des kinases et le produit d'immobilisation, et
c) détection du complexe ou des complexes formés dans l'étape b).

8. Procédé d'identification d'un composé interagissant avec une kinase, comprenant les étapes de :
a) fourniture de deux aliquots d'une préparation de protéines contenant toute une diversité de kinases,
b) mise en contact d'un aliquot avec le produit d'immobilisation selon l'une quelconque des revendications 5 ou 6 dans des conditions permettant la formation d'un ou de plusieurs complexes différents entre l'une des kinases et le produit d'immobilisation,
c) mise en contact de l'autre aliquot avec le produit d'immobilisation selon l'une quelconque des revendications 5 ou 6 et avec un composé donné dans des conditions permettant la formation d'un ou de plusieurs complexes différents entre l'une des kinases et le produit d'immobilisation, et
d) détermination de la quantité du complexe ou des complexes formés dans les étapes b) et c).

9. Procédé d'identification d'un composé interagissant avec une kinase, comprenant les étapes de :
a) fourniture de deux aliquots comprenant chacun au moins une cellule contenant toute une diversité de kinases,
b) incubation d'un aliquot avec un composé donné,
c) récolte des cellules de chaque aliquot,
d) lyse des cellules afin d'obtenir des préparations de protéines,
e) mise en contact des préparations de protéines avec le produit d'immobilisation selon l'une quelconque des revendications 5 ou 6 dans des conditions permettant la formation d'un ou de plusieurs complexes différents entre l'une des kinases et le produit d'immobilisation, et
f) détermination de la quantité du complexe ou des complexes formés dans chaque aliquot dans l'étape e).

10. Procédé selon l'une quelconque des revendications 7 à 9, dans lequel la diversité de kinases comprend un ou plusieurs membres de la famille des IRAK, de préférence IRAK 1 ou IRAK 4.

11. Procédé selon l'une quelconque des revendications 7 à 9, dans lequel la diversité de kinases comprend NIK.

12. Procédé selon l'une quelconque des revendications 8 à 11, dans lequel une quantité réduite du complexe formé dans l'aliquot incubé avec le composé comparativement à l'aliquot non incubé avec le composé indique que ladite kinase est une cible du composé.

13. Procédé selon l'une quelconque des revendications 8 à 11, dans lequel la quantité du complexe est déterminée par la séparation de la kinase du produit d'immobilisation et la détection subséquente de la kinase séparée ou la détermination subséquente de la quantité de la kinase séparée, en particulier où la kinase est détectée ou la quantité de la kinase est déterminée par des procédés de spectrométrie de masse ou d'immunodétection, de préférence avec un anticorps dirigé contre la kinase.

14. Procédé selon l'une quelconque des revendications 7 à 13, dans lequel ledit composé donné est choisi dans le groupe constitué de composés synthétiques, ou de médicaments organiques synthétiques, de préférence des petites molécules organiques médicamenteuses, et des petites molécules de composés naturels.

15. Procédé selon l'une quelconque des revendications 7 à 14, dans lequel le composé donné est un inhibiteur de kinase.

16. Procédé selon l'une quelconque des revendications 7 à 15, dans lequel la fourniture d'une préparation de protéines comprend les étapes de récolte d'au moins une cellule contenant des kinases et de lyse de la cellule.

17. Procédé selon l'une quelconque des revendications 7 à 16, dans lequel les étapes de la formation du complexe sont réalisées dans des conditions essentiellement physiologiques.

18. Procédé de détermination de la présence d'une ou de plusieurs kinases dans un échantillon, comprenant les étapes de :
a) fourniture d'une préparation de protéines qui est supposée contenir lesdites une ou plusieurs kinases,
b) mise en contact de la préparation de protéines avec le produit d'immobilisation selon l'une quelconque des revendications 5 ou 6 dans des conditions permettant la formation d'un complexe entre l'une des kinases et le produit d'immobilisation, et
c) détection si une ou plusieurs kinases ont formé un complexe avec le produit d'immobilisation.

19. Utilisation du composé d'immobilisation selon l'une quelconque des revendications 1 ou 2 ou du produit d'immobilisation selon l'une quelconque des revendications 5 ou 6 pour l'identification de composés interagissant avec des kinases ou pour la purification de kinases.
